# EUROPÄISCHE PATENTANMELDUNG

(11) **EP 1 498 422 A1**
(43) Veröffentlichungstag der Anmeldung: **19.01.2005**
(21) Anmeldenummer: 03016233.3
(22) Anmeldetag: 17.07.2003
(51) Int. Cl.: C07K 7/06, C07K 14/47, C07K 14/705, C07K 7/50

(54) **C5a-Rezeptor-Antagonisten**

(71) Anmelder: Jerini AG, 10115 Berlin (DE)
(72) Erfinder: Hummel, Gerd, Dr., 13357 Berlin (DE); Knolle, Jochen, Dr., 10115 Berlin (DE); Locardi, Elsa, Dr., 10551 Berlin (DE); Polakowski, Thomas, Dr., 10439 Berlin (DE); Scharn, Dirk, Dr., 13507 Berlin (DE); Schnatbaum, Karsten, Dr., 10245 Berlin (DE)
(74) Vertreter: Bohmann, Armin K., Dr.

(57) **Zusammenfassung**

Die vorliegende Erfindung betrifft eine Verbindung, bevorzugterweise ein C5a-Rezeptor-Antagonist, mit der folgenden Struktur: 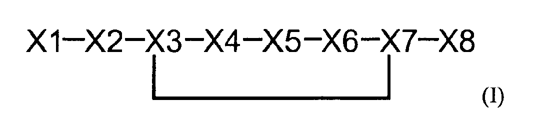 , wobei
X1 ein Radikal mit einer Masse von etwa 1-300 ist und wobei X1 bevorzugterweise ausgewählt ist aus der Gruppe, die R5-, R5-CO-, R5-N(R6)-CO-, R5-O-CO-, R5-SO₂-, R5-N(R6)-SO₂-, R5-N(R6)-, R5-N(R6)-CS-, R5-N(R6)-C(NH)-, R5-CS-, R5-P(O)OH-, R5-B(OH)-, R5-CH=N-O-CH₂-CO- umfasst, wobei R5 und R6 einzeln und unabhängig voneinander ausgewählt sind aus der Gruppe, die H, F, Hydroxy, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, Acyl, substituiertes Acyl, Alkoxy, Alkoxyalkyl, substituiertes Alkoxyalkyl, Aryloxyalkyl und substituiertes Aryloxyalkyl umfasst,
X2 ein Radikal ist, das die biologischen Bindungseigenschaften einer Phenylalanin-Einheit mimikt,
X3 und X4 einzeln und unabhängig voneinander ein Spacer ist, wobei der Spacer bevorzugterweise aus der Gruppe ausgewählt ist, die Aminosäuren, Aminosäure-Analoga und Aminosäure-Derivate umfasst,
X5 ein Radikal ist, das die biologischen Bindungseigenschaften einer Cyclohexylalanin-Einheit mimikt,
X6 ein Radikal ist, das die biologischen Bindungseigenschaften einer Tryptophan-Einheit mimikt,
X7 ein Radikal ist, das die biologischen Bindungseigenschaften einer Norleucin- oder Phenylalanin-Einheit mimikt,
X8 ein Radikal ist, wobei das Radikal optional in Struktur I enthalten ist und wenn es enthalten ist, ausgewählt ist aus der Gruppe, die H, NH₂, OH, NH-OH, Amino, substituiertes Amino, Alkoxy, substituiertes Alkoxy, Hydrazino, substituiertes Hydrazino, Aminooxy, substituiertes Aminooxy, Alkyl, substituiertes Alkyl, Cycloalkyl, substituiertes Cycloalkyl, Heterocyclyl, substituiertes Heterocyclyl, Heteroaryl, substituiertes Heteroaryl, Arylalkyl, substituiertes Arylalkyl, Aryl, substituiertes Aryl, Aminosäure, Aminosäurederivat und Aminosäureanalogon umfasst,
eine chemische Bindung zwischen X3 und X7 ausgebildet ist, und
die Verbindungslinien - in Formel (I) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, DisulfidBindungen, Ether-Bindungen, Thioether-Bindungen, Oxim-Bindungen und Aminotriazin-Bindungen umfasst.

## Beschreibung

Die vorliegende Erfindung betrifft Antagonisten des C5a-Rezeptors sowie Verwendungen derselben.

### Stand der Technik

Neben dem adaptiven Immunsystem gibt es ein stammesgeschichtlich deutlich älteres System zur Abwehr von Infektionen. Dieses System wird Komplementsystem genannt und besteht aus mehr als 30 löslichen und membrangebundenen Proteinen. Das Komplementsystem kann zusammen mit der adaptiven Immunantwort oder eigenständig aktiv werden, um z.B. pathogene Bakterien zu töten. Eine überschiessende Aktivierung bzw. nicht hinreichende Regulierung des Komplementsystems wird mit einer großen Zahl von entzündlichen Erkrankungen in Verbindung gebracht wie z.B. septischer Schock, Reperfusionsschaden, rheumatoide Arthritis, Transplantatabstossung, Akutes Lungenversagen (adult respiratory distress syndrome, ARDS), Systemischer Lupus erythematodes (SLE) und Glomerulonephritis. Übersichten über die Beziehungen zwischen dem Komplementsystem und Erkrankungen sind in großer Zahl publiziert worden (z.B.: Kirschfink 1997 Immunopharmacology 38: 51-62; Markides 1998 Pharmacological Reviews 50: 59-87, Walport 2001 The New England Journal of Medicine 344: 1140-1144, Walport 2001 The New England Journal of Medicine 344: 1058-66).

Die Aktivierung des Komplementsystems kann über drei unterschiedliche Wege stattfinden. Diese werden klassischer, alternativer und Mannose-binding Lectin (MBL)-Weg genannt. Alle Wege verlaufen über die sequentielle Prozessierung - und damit Aktivierung - von inaktiven Proformen von Proteasen. Da die jeweils aktivierte Protease die nächste Proform aktivieren kann, erhält man eine Verstärkung der auslösenden Reaktion vergleichbar mit der Gerinnungskaskade. Ein Überblick über das Komplementsystem ist bei Sim und Laich (2000 Biochemical Society Transactions 28: 545-550) zu finden.

Zu den wichtigsten Proteinen, die während der Komplementaktivierung entstehen, zählen C3a, C3b, C5a und C5b, auf die im Folgenden näher eingegangen wird.

C3b ist entscheidender Bestandteil einer zentralen Protease der Komplementkaskade, der C5-Convertase. C3b ist Bestandteil der C5-Convertase sowohl des klassischen als auch des alternativen Weges. Der MLB-Weg führt ebenfalls über die Konvertasen des klassischen Weges. Die C5-Konvertase ist verantwortlich für den Fortgang der Komplementkaskade und katalysiert die Spaltung von C5. Zudem wird C3b kovalent an die Oberfläche von z.B. Bakterien gebunden, die dadurch bevorzugt von Makrophagen aufgenommen werden können. Ähnliches gilt auch für die Klärung von Immunkomplexen.

C3a ist das kleine Fragment, das neben C3b durch die Spaltung von C3 entsteht. C3a ist ein verhältnismäßig schwaches Chemokin und zählt zu den Anaphylatoxinen.

C5b entsteht durch Spaltung von C5. Das Spaltprodukt ist der Ausgangspunkt für die Bildung des Membrane-Attack-Complex (MAC). Der MAC bildet eine Pore, durch die die Plasmamembran von Bakterien aber auch körpereigenen Zellen perforiert werden kann. Dadurch kann es zur Lyse der perforierten Zellen kommen.

C5a ist das 74 Aminosäuren große, N-terminale Spaltprodukt der α-Kette des Plasmaproteins C5 und wird durch die Aktivität der C5-Konvertase freigesetzt. C5a wird von seinem Rezeptor, der als C5aR oder auch CD88 bezeichnet wird, mit hoher Affinität gebunden und löst eine Vielzahl proinflammatorischer Effekte aus. Es ist eines der stärksten Chemokine und gehört wie C3a zu den Anaphylatoxinen. Der C5aR ist auf einer Vielzahl von Zellen zu finden. Insbesondere auf Neutrophilen, Makrophagen, Zellen der glatten Muskulatur und Endothelzellen findet man den Rezeptor.

Die Freisetzung von C5a wird direkt oder indirekt für eine Vielzahl von Erkrankungen verantwortlich gemacht. Beispiele hierfür sind Sepsis (Huber-Lang et al. 2001 Faseb Journal 15: 568-570), Multiple Sklerose (Mullerladner et al. 1996 Journal of Neurological Science 144: 135-141), Reperfusionsschaden (Riley et al. 2000 Journal of Thoriacic and Cardiovascular Surgery 120: 350-358), Psoriasis (Bergh et al. 1993 Archives of Dermatological Research 285: 131-134), rheumatoide Arthritis (Woodruff et al. 2002 Arthritis and Rheumattism 46: 2476-85) und Immunkomplex assoziierte Erkrankungen (Heller et al. 1999 Journal of Immunology 163: 985-994). Einen Überblick über inflammatorische Erkrankungen, an denen C5a beteiligt ist, bietet Köhl (2001 Molecular Immunology 38: 51-62).

Obwohl C5a offensichtlich für viele Symptome bei entzündlichen Erkrankungen verantwortlich ist, gibt es bis zum heutigen Tag kein zugelassenes Medikament, das direkt an der Wechselwirkung zwischen Rezeptor und Ligand angreift. Der C5aR stellt einen besonders interessanten Angriffspunkt dar. Dies liegt insbesondere an der Tatsache, dass Mäuse, denen der Rezeptor fehlt, keinen auffälligen Phänotyp besitzen (Hopken et al. 1996 Nature 383: 86-89). Das bedeutet, dass die Komplementkaskade mit ihren nützlichen Funktionen zur Abwehr von Krankheitserregern (MAC-Bildung) und dem Abbau von Immunkomplexen auch bei vollständiger Inaktivierung des Rezeptors ungehindert ablaufen kann.

Zur Entwicklung eines spezifischen C5a-Rezeptor-Antagonisten, hierin auch als C5aR-Antagonist bezeichnet, gab es in der Vergangenheit eine Reihe von Versuchen. Unter anderem wurde nach kleinen Molekülen gesucht. Als Beispiele für derartige Moleküle sind unter anderem L-156602 (Merck), RPR120033 (Rhone-Poulenc), W-54011 (Mitsubishi Pharma) und NGD 2000-1 (Neurogen) bekannt geworden. Alle bisher bekannten Inhibitoren mit einem Molekulargewicht von < 500 g/mol haben zumindest einen der folgenden Nachteile: eine geringe Spezifität, agonistische Wirkung, zu niedrige Affinität, schlechte Löslichkeit, unzureichende metabolische Stabilität oder Inhibierung von P450 Enzymen.

Ein anderer Weg zur Entwicklung von C5aR-Antagonisten wurde durch die Verwendung von rekombinanten Proteinen beschritten. Beispiele für derartige Antagonisten auf Protein-Basis sind CGS 32359 (Ciba-Geigy, Pellas et al. 1998 Journal of Immunology 160: 5616-5621), ΔpIII-A8 (Heller et al. 1999 Journal of Immunology 163: 985-994) bzw. Antikörper, die rekombinant oder nicht-rekombinanten Ursprungs sein können (Huber-Lang et al. 2001 Faseb Journal 15: 568-570). Diese C5aR-Antagonisten sind Proteine und entsprechend kostspielig herzustellen. Sie zeichnen sich durch eine verhältnismäßig hohe Affinität und Spezifität aus, haben aber den Nachteil einer hohen Immunogenität. Zudem sind Proteine nur durch aufwendige Verfahren wie z.B. Injektionen effizient zu verabreichen.

Die Sequenzinformation aus dem C-terminalen Bereich von C5a wurde zur Entwicklung von peptidischen Antagonisten verwendet. Peptide als therapeutisch nutzbare Antagonisten des C5aRs haben den Vorteil der geringeren Produktionskosten, der geringeren Immunogenität sowie hoher Plasmastabilität im Vergleich zu Proteintherapeutika und sind zudem spezifischer als die meisten der bisher bekannten kleinen Moleküle. Peptidische Antagonisten sind in großer Zahl beschrieben. Gemeinsames Merkmal fast aller peptidischer C5aR-Antagonisten ist ihr Ursprung im C-Terminus von C5a. Beispiele für peptidische C5aR-Antagonisten bzw. partielle Agonisten sind unter anderem in folgenden Patentanmeldungen bzw. Patenten beschrieben: US 4,692,511, US 5,663,148, WO 90/09162, WO 92/11858, WO 92/12168, WO 92/21361, WO 94/07518, WO 94/07815, WO 95/25957, WO 96/06629, WO 99/00406 und WO 99/13899, WO 03/033528.

Fast alle bisher beschriebenen Peptide, die an den C5aR binden, tragen C-terminal die positiv geladene Aminosäure Arginin. Sequenzen dieser Peptide wurden sowohl in der wissenschaftlichen Literatur (Finch et al. 1999 Journal of Medicinical Chemistry 42: 1965-1974; Wong et al. 1999 IDrugs 2: 686-693; Psczkowski et al. 1999 Pharmacology 128: 1461-1466) als auch in den oben zitierten Patenten und Patentanmeldungen offenbart. In WO 90/09162 werden 38 peptidische Inhibitoren mit ihren IC₅₀-Werten vorgestellt. Davon haben 37 Peptide ein C-terminales Arginin und ein Peptid trägt ein C-terminales Tyrosin. Die Substitution des C-terminalen Arginins führt in diesem Beispiel zu einem deutlichen Verlust an Affinität. Der niedrigste IC₅₀-Wert der Verbindungen aus dieser Patentanmeldung beträgt 0,011 µM. Im Gegensatz dazu führt ein Tyrosin am C-Terminus zu einem IC₅₀-Wert von 0,17 µM. In dem in dieser Anmeldung verwendeten funktionellen Assaysystem zeigt diese Verbindung einen IC₅₀-Wert von 1,3 µM.

In WO 92/12168 werden 20 Peptide mit ihren IC₅₀-Werten (Bindung an C5aR) angegeben. Davon haben 19 ein endständiges Arginin, das sowohl in der D- als auch der L-Form vorliegen kann. Ein Peptid trägt C-terminal einen Phenylbutanoyl-Rest. Dieses Peptid wird mit einem IC₅₀-Wert von lediglich 2,6 µM gegenüber dem besten Peptid mit 0,014 µM angegeben und ist damit nicht als Medikament geeignet. Von den 22 Peptiden aus WO 94/07518, zu denen IC₅₀-Werte angegeben wurden, tragen alle Peptide ein C-terminales Arginin.

Die in den genannten Anmeldungen angegebenen IC₅₀-Werte sind durch Messungen mit isolierten Membranen polymorphkerniger neutrophiler Granulozyten (PMN-Membranen) entstanden, da zum Zeitpunkt der Durchführung der Experimente keine C5aR überexprimierenden Zellen hergestellt werden konnten. Die so entstandenen Werte spiegeln nicht die Affinität der Verbindung an ganzen Zellen wieder. In der Regel sind die Verbindungen zu Rezeptoren auf ganzen Zellen deutlich weniger affin (Kawai et al. 1991 Journal of Medicinical Chemistry 34: 2068-71; Rollins et al. 1988 Journal of Biological Chemistry 263: 520-526). Es ist aussagekräftiger, anstelle der Bindung der Antagonisten an die Rezeptoren deren biologische Aktivität zu messen. Häufig werden derartige funktionelle Assays für G Protein gekoppelte Rezeptoren verwendet.

Auch die in den internationalen Patentanmeldungen WO 95/25957 und WO 96/06629 gegebenen Beispiele, zu denen IC₅₀-Werte bekannt sind, sind ausnahmslos Peptide, die C-terminal ein Arginin tragen. WO 99/00406 beschreibt eine Reihe zyklischer und linearer peptidischer Inhibitoren, deren gemeinsames Merkmal das C-terminale Arginin ist. Innerhalb eines in WO 99/00406 dargelegten Modells des Pharmakophors wird explizit auf die notwendige positive Ladung, die beispielsweise durch Arginin realisiert wird, eingegangen. Dies spiegelt sich auch in der Sequenz von C5a wider, das ebenfalls C-terminal ein Arginin trägt. Wird dieses Arginin durch Carboxypeptidasen abgespalten (C5a-desArg), erhält man einen 10-100fach geringer affinen Agonisten.

In WO 03/033528 wird von Einzelsubstitutionen verschiedener Aminosäuren im Molekül Ac-Phe[Om-Pro-cha-Trp-Arg] berichtet. Es wird gezeigt, das z.B. der Austausch von Arginin gegen Citrullin, Lysin oder Homoarginin zu einer Verschlechterung der Affinität zum C5a-Rezeptor führt. Die IC₅₀-Werte (Bindung) betragen 6 µM, 24 µM bzw. 1,4 µM für die drei beschriebenen Argininsubstitutionen (das Ausgangsmolekül trägt C-terminal Arginin und hat einen IC₅₀-Wert von 0,3 µM wie in WO 99/00406 dargelegt). Dies unterstreicht die Bedeutung des C-terminalen Arginins.

In einem Übersichtsartikel von Morikis und Lambris (2002 Biochemical Society Transactions 30: 1026-1036) wird ebenfalls die Bedeutung des Arginins für die Affinität von Antagonisten und Agonisten zum C5a Rezeptor betont.

Es wird deutlich, dass gemäß der technischen Lehre des Standes der Technik für peptidische und peptidomimetische C5a-Liganden nennenswerter inhibitorischer Aktivität (IC₅₀ < 200 nM) eine C-terminal lokalisierte positive Ladung verlangt wird. Diese Ladung wird in der Regel durch Arginin realisiert.

Der vorliegenden Erfindung liegt die Aufgabe zugrunde, Antagonisten des C5a-Rezeptors bereitzustellen. Eine weitere der vorliegenden Erfindung zugrundeliegende Aufgabe besteht darin, dass Medikamente bereitgestellt werden, die bei der Behandlung von Krankheitsbildern verwendet werden können, an denen kausal der C5a-Rezeptor beteiligt ist.

In einem ersten Aspekt wird die Aufgabe erfindungsgemäß gelöst durch eine Verbindung, bevorzugterweise einen C5a-Rezeptor-Antagonist, mit der folgenden Struktur: 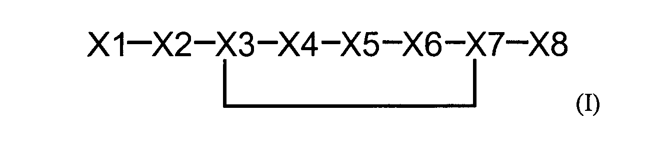 , wobei
X1 ein Radikal mit einer Masse von etwa 1-300 ist und wobei X1 bevorzugterweise ausgewählt ist aus der Gruppe, die R5-, R5-CO-, R5-N(R6)-CO-, R5-O-CO-, R5-SO₂-,- R5-N(R6)-SO₂-, R5-N(R6)-, R5-N(R6)-CS-, R5-N(R6)-C(NH)-, R5-CS-, R5-P(O)OH-, R5-B(OH)-, R5-CH=N-O-CH₂-CO- umfasst, wobei R5 und R6 einzeln und unabhängig voneinander ausgewählt sind aus der Gruppe, die H, F, Hydroxy, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, Acyl, substituiertes Acyl, Alkoxy, Alkoxyalkyl, substituiertes Alkoxyalkyl, Aryloxyalkyl und substituiertes Aryloxyalkyl umfasst,
X2 ein Radikal ist, das die biologischen Bindungseigenschaften einer Phenylalanin-Einheit mimikt,
X3 und X4 einzeln und unabhängig voneinander ein Spacer ist, wobei der Spacer bevorzugterweise aus der Gruppe ausgewählt ist, die Aminosäuren, Aminosäure-Analoga und Aminosäure-Derivate umfasst,
X5 ein Radikal ist, das die biologischen Bindungseigenschaften einer Cyclohexylalanin-Einheit mimikt,
X6 ein Radikal ist, das die biologischen Bindungseigenschaften einer Tryptophan-Einheit mimikt,
X7 ein Radikal ist, das die biologischen Bindungseigenschaften einer Norleucin- oder Phenylalanin-Einheit mimikt,
X8 ein Radikal ist, wobei das Radikal optional in Struktur I enthalten ist und wenn es enthalten ist, ausgewählt ist aus der Gruppe, die H, NH₂, OH, NH-OH, Amino, substituiertes Amino, Alkoxy, substituiertes Alkoxy, Hydrazino, substituiertes Hydrazino, Aminooxy, substituiertes Aminooxy, Alkyl, substituiertes Alkyl, Cycloalkyl, substituiertes Cycloalkyl, Heterocyclyl, substituiertes Heterocyclyl, Heteroaryl, substituiertes Heteroaryl, Arylalkyl, substituiertes Arylalkyl, Aryl, substituiertes Aryl, Aminosäure, Aminosäurederivat und Aminosäureanalogon umfasst,
eine chemische Bindung zwischen X3 und X7 ausgebildet ist, und
die Verbindungslinien - in Formel (I) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

In einer Ausführungsform ist vorgesehen, dass X3 und X7 jeweils eine Aminosäure, ein Aminosäurederivat oder ein Aminosäureanalogon ist, wobei die chemische Bindung zwischen X3 und X7 unter Beteiligung von Molekülteilen von X3 und X7 ausgebildet ist, und die Molekülteile für X3 und X7 einzeln und unabhängig voneinander ausgewählt sind aus der Gruppe, die den C-Terminus, den N-Terminus und die jeweilige Seitenkette der Aminosäure umfasst.

In einer weiteren Ausführungsform ist vorgesehen, dass
X1 ein Radikal mit einer Masse von etwa 1-300 ist, wobei das Radikal bevorzugterweise ausgewählt ist aus der Gruppe, die R5, R5-CO-, R5-N(R6)-CO-, R5-O-CO-, R5-SO₂-, R5-N(R6)-C(NH)-, umfasst, wobei bevorzugtererweise R5 und R6 einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl und substituiertes Aryl enthält;
X2 und X6 jeweils und unabhängig voneinander eine aromatische Aminosäure, ein Derivat oder ein Analogon davon ist;
X5 und X7 einzeln und unabhängig voneinander eine hydrophobe Aminosäure, ein Derivat oder ein Analogon davon sind.

In einer speziellen Ausführungsform ist vorgesehen, dass X1 ausgewählt ist aus der Gruppe, die H, Acetyl, Propanoyl, Butanoyl, Benzoyl, Fluormethylcarbonyl, Difluormethylcarbonyl, Phenyl, Oxycarbonyl, Methyl-oxycarbonyl, Phenyl-aminocarbonyl, Methyl-aminocarbonyl, Phenyl-sulfonyl und Methyl-sulfonyl umfasst.

In einer Ausführungsform ist vorgesehen, dass X2, X5, X6 und X7 einzeln und unabhängig voneinander die folgende Struktur aufweisen: 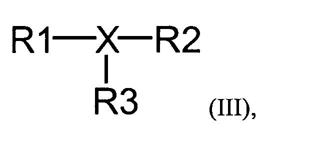 worin
X C(R4) oder N ist,
R1 optional vorhanden ist und wenn R1 vorhanden ist, R1 ein Radikal ist, das ausgewählt ist aus der Gruppe, die >N-R1B, >C(R1B)(R1C) und >O umfasst, wobei R1B und R1C einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, S=O, C=NH, C=N-CN, PO(OH), B(OH), CH₂, CH₂CO, CHF und CF₂ umfasst;
R4 ein Radikal ist, wobei das Radikal ausgewählt ist aus der Gruppe, die H, F, CH₃, CF₃, Alkyl und substituiertes Alkyl umfasst;
und die Bindung von Struktur (III) an die Molekülbestandteile X1 und X3, X4 und X6, X5 und X7, und X6 und X8 bevorzugt über R1 und R2 erfolgt;
für X2 und für X6 einzeln und unabhängig voneinander R3 ein Radikal ist, wobei das Radikal eine aromatische Gruppe enthält und ausgewählt ist aus der Gruppe, die Aryl, substituiertes Aryl, Heteroaryl, substituiertes Heteroaryl, Arylalkyl, substituiertes Arylalkyl, Heteroarylalkyl, substituiertes Heteroarylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst; und
für X5 und für X7 einzeln und unabhängig voneinander R3 ein Radikal ist, wobei das Radikal eine aliphatische oder aromatische Gruppe enthält und bevorzugterweise ausgewählt ist aus der Gruppe, die Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Cycloalkylalkyl, substituiertes Cycloalkylalkyl, Heterocyclylalkyl, substituiertes Heterocyclylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst.

In einer speziellen Ausführungsform ist vorgesehen, dass unter Beteiligung von R3 und R4 ein Ring ausgebildet wird.

In einer weiteren Ausführungsform ist vorgesehen, dass für X2 und für X6 einzeln und unabhängig voneinander R3 aus der Gruppe ausgewählt ist, die Phenyl, substituiertes Phenyl, Benzyl, substituiertes Benzyl, 1,1-Diphenylmethyl, substituiertes 1,1-Diphenylmethyl, Naphthylmethyl, substituiertes Naphthylmethyl, Thienylmethyl, substituiertes Thienylmethyl, Benzothienylmethyl, substituiertes Benzothienylmethyl, Imidazolylmethyl, substituiertes Imidazolylmethyl, Indolylmethyl und substituiertes Indolylmethyl umfasst.

In einer noch weiteren Ausführungsform ist vorgesehen, dass für X5 und für X7 einzeln und unabhängig voneinander R3 aus der Gruppe ausgewählt ist, die C3-C5-Alkyl, substituiertes C3-C5-Alkyl, C5-C7-Cycloalkyl, substituiertes C5-C7-Cycloalkyl, C5-C7-Cycloalkylmethyl, substituiertes CS-C7-Cycloalkylmethyl, Cycloalkylethyl, substituiertes Cycloalkylethyl, Benzyl, substituiertes Benzyl, Phenylethyl, Naphthylmethyl, Thienylmethyl, Propenyl, Propinyl, Methylthioethyl, Imidazolylmethyl, substituiertes Imidazolylmethyl, Indolylmethyl und substituiertes Indolylmethyl umfasst.

Wie hierin verwendet bezeichnet C3-C5-Alkyl einen Rest bestehend aus 3, 4 oder 5 C-Atomen und substituiertes C3-C5-Alkyl einen Rest bestehend aus 3, 4 oder 5 C-Atomen, wobei ein jedes der Atome einen oder mehrere Substituenten tragen kann. Besonders bevorzugte C3-C5-Alkylreste sind dabei *n*-Propyl, *n*-Butyl, *n*-Pentyl, *i*-Propyl, *i*-Butyl, und *i*-Pentyl. Dabei ist es im Rahmen der vorliegenden Erfindung, dass diese besonders bevorzugten C3-C5-Alkylreste eine oder mehrere Substitutionen tragen.

Wie hierin verwendet bezeichnet C5-C7-Cycloalkyl einen zyklischen Rest bestehend aus 5, 6 oder 7 C-Atomen und substituiertes C5-C7-Cycloalkyl einen zyklischen Rest bestehend aus 5, 6 oder 7 C-Atomen, wobei ein jedes der Atome einen oder mehrere Substituenten aufweisen kann. Besonders bevorzugte C5-C7-Cycloalkyl-Reste sind dabei Cycloheptyl, Cyclohexyl und Cyclopentyl, wobei es im Rahmen der vorliegenden Erfindung ist, dass diese besonders bevorzugten C5-C7-Cycloalkyl-Reste eine oder mehrere Substitutionen tragen.

Wie hierin verwendet, bezeichnet C5-C7-Cycloalkylmethyl einen Cycloalkyl-Rest, der über einen Methylenrest an den Rest des Moleküls gebunden ist.

Wie hierin verwendet bezeichnet Cycloalkylethyl einen Cycloalkyl-Rest, der über einen Ethylenrest an den Rest des Moleküls gebunden ist.

In einer Ausführungsform der erfindungsgemäßen Verbindung ist vorgesehen, dass
X2 ein Aminosäurederivat einer Aminosäure ist, das ausgewählt ist aus der Gruppe, die Phenylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine, 1-Naphtylalanin, 2-Thienylalanin, 3-Thienylalanin, 3,3-Diphenylalanin, Tyrosin, Tryptophan, Histidin und jeweilige Derivate davon umfasst;
oder X2 und X1 sind zusammengenommen PhCH₂CH₂CO- oder PhCH₂-;
X6 ein Aminosäurederivat einer Aminosäure ist, die ausgewählt ist aus der Gruppe, die Tryptophan, Phenylalanin, Tyrosin, Histidin, 1-Naphtylalanin, Benzothienylalanin, 2-Aminoindan-2-carbonsäure, 2-Thienylalanin, 3-Thienylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine und jeweilige Derivate davon umfasst;
X5 ein Aminosäurederivat einer Aminosäure ist, die ausgewählt ist aus der Gruppe, die D-Cyclohexylalanin, D-Cyclohexylglycin, D-Homo-Cyclohexylalanin, Octahydroindol-2-carbonsäure, 2-Methyl-D-Phenylalanin und jeweilige Derivate davon umfasst; und
X7 ein Aminosäurederivat einer Aminosäure ist, die ausgewählt ist aus der Gruppe, die Norvalin, Norleucin, Homo-Leucin, Leucin, Isoleucin, Valin, Cystein, Cystein(Me), Cystein(Et), Cystein(Pr), Methionin, Allylglycin, Propargylglycin, Cyclohexylglycin, Cyclohexylalanin, Phenylalanin, Tyrosin, Tryptophan, Histidin, 1-Naphtylalanin, 2-Thienylalanin, 3-Thienylalanin und jeweilige Derivate davon umfasst.

In einer weiteren Ausführungsform ist vorgesehen, dass X1 und/oder X4 eine oder mehrere die Wasserlöslichkeit verbessernde Gruppen aufweisen, wobei die die Wasserlöslichkeit verbessernde Gruppe ausgewählt ist aus der Gruppe, die Hydroxy, Keto, Carboxamido, Ether, Harnstoff, Carbamat, Amino, substituiertes Amino, Guanidino, Pyridyl und Carboxyl umfasst.

In einem zweiten Aspekt wird die Aufgabe erfindungsgemäß gelöst durch eine Verbindung, bevorzugterweise einen C5a-Rezeptor-Antagonist, mit der Struktur 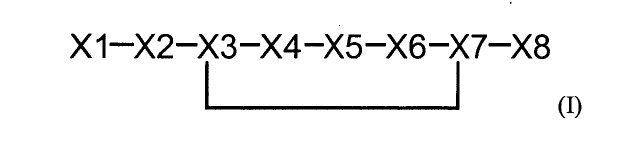 , wobei X1-X3 und X5-X8 definiert sind, wie im Zusammenhang mit der Verbindung gemäß dem ersten Aspekt der vorliegenden Erfindung definiert, wobei
X4 eine zyklische oder eine nichtzyklische Aminosäure ist, wobei die zyklische Aminosäure ausgewählt ist aus der Gruppe, die Prolin, Pipecolinsäure, Azetidin-2-carbonsäure, Tetrahydroisochinolin-3-carbonsäure, Tetrahydroisochinolin-1-carbonsäure, Octahydroindol-2-carbonsäure, 1-Aza-bicyclo-[3.3.0]-octan-2-carbonsäure, 4-Phenyl-pyrrolidin-2-carbonsäure, cis-Hyp und trans-Hyp umfasst, und die nichtzyklische Aminosäure ausgewählt aus der Gruppe, die Ser, Gln, Asn, Cys(O₂CH₂CH₂CONH₂), Arg, Hyp(COCH₂OCH₂CH₂OCH₂CH₂OCH₃), Hyp(CONH-CH₂CH(OH)-CH₂OH) und jeweilige Derivate davon und jeweilige Analoga davon umfasst; und
die Verbindungslinien - in Formel (I) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

In einer Ausführungsform ist dabei vorgesehen, dass die Derivate und/oder Analoga so ausgebildet sind, dass einzelne oder mehrere Wasserstoffatome der genannten Aminosäuren substituiert sein können.

In einer Ausführungsform ist vorgesehen, dass die Aminosäuren bevorzugt ausgewählt sind aus der Gruppe, die Prolin, Pipecolinsäure, Azetidin-2-carbonsäure, Tetrahydroisochinolin-3-carbonsäure, Tetrahydroisochinolin-1-carbonsäure, Octahydroindol-2-carbonsäure, 1-Azabicyclo-[3.3.0]-octan-2-carbonsäure, 4-Phenyl-pyrrolidin-2-carbonsäure, Hyp, Ser, Gln, Asn, Cys(O₂CH₂CH₂CONH₂) und Arg umfasst. Die durch die vorstehenden Abkürzungen bezeichneten Verbindungen sind hierin in Tabelle 3 nochmals definiert.

In einem dritten Aspekt wird die Aufgabe erfindungsgemäß gelöst durch eine Verbindung, bevorzugterweise einen C5a-Rezeptor-Antagonist, mit der Struktur 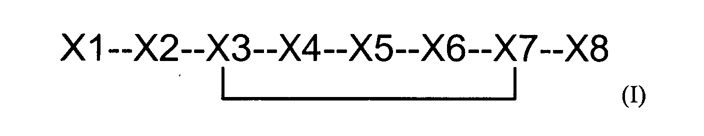 , wobei X1-X2 und X4-X8 definiert sind, wie im Zusammenhang mit der Verbindung gemäß dem ersten und zweiten Aspekt der vorliegenden Erfindung definiert, wobei
X3 folgende Struktur aufweist 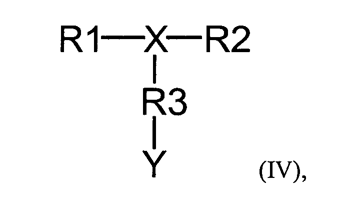 worin
X C(R4) oder N ist,
R1 optional vorhanden ist und wenn R1 vorhanden ist, Rlein Radikal ist, das ausgewählt ist aus der Gruppe, die >N-R1B, >C(R1B)(R1C) und >O umfasst, wobei R1B und R1C einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, PO(OH), B(OH), CH₂, CH₂CO, CHF und CF₂ umfasst;
R4 ein Radikal ist, wobei das Radikal ausgewählt ist aus der Gruppe, die H, F, CF₃, Alkyl und substituiertes Alkyl umfasst; die Bindung von Struktur (IV) an die Molekülbestandteile X2 und X4 bevorzugt über R1 und R2 erfolgt;
R3 ein Radikal ist, das ausgewählt ist aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Cycloalkylalkyl, substituiertes Cycloalkylalkyl, Heterocyclylalkyl, substituiertes Heterocyclylalkyl, Arylakyl, substituiertes Arylalkyl, Heteroarylalkyl und substituiertes Heteroarylalkyl umfasst.

Y optional vorhanden ist und wenn Y vorhanden ist, Y ein Radikal ist, das ausgewählt ist aus der Gruppe, die -N(YB)-, -O-, -S-, -S-S-, -CO-, -C=N-O-, -CO-N(YB)- und 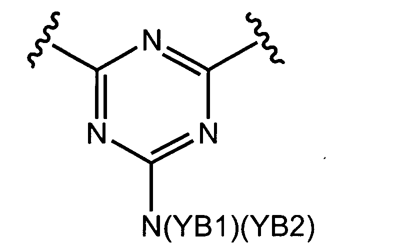 umfasst, wobei YB, YB1 und YB2 einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst.

In einer Ausführungsform ist vorgesehen, dass
R3 ein Radikal ist, das ausgewählt ist aus der Gruppe, die Methyl, Ethyl, Propyl, Butyl, Benzyl und 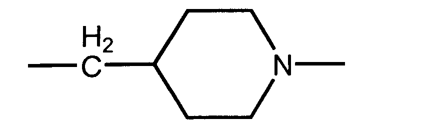 umfasst;
Y optional vorhanden ist und wenn Y vorhanden ist, Y ein Radikal ist, das ausgewählt ist aus der Gruppe, die -N(YB)-, -O-, -S- und -S-S- umfasst.

In einem vierten Aspekt wird die Aufgabe erfindungsgemäß gelöst durch eine Verbindung, bevorzugterweise einen C5a-Rezeptor-Antagonist, wobei die Verbindung
die folgende Struktur aufweist: 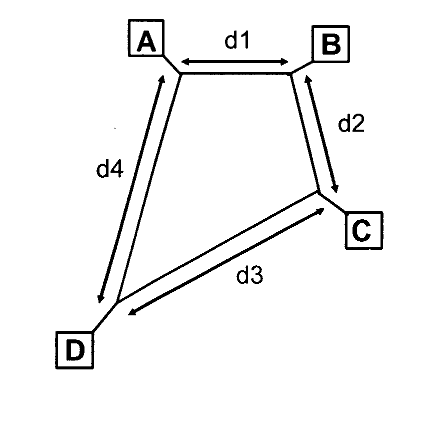 wobei d1, d2, d3 und d4 die Abstände von A, B, C und D in wenigstens einem energetisch zugänglichen Konformer der Verbindung bezeichnen und die folgenden Werte aufweisen:
d1 = 5.1 ± 1.0 Å
d2 = 11.5 ± 1.0 Å
d3 = 10.0 ± 1.5 Å
d4 = 6.9 ± 1.5 Å

A und C einzeln und unabhängig voneinander ein hydrophobes Radikal sind, wobei das hydrophobe Radikal ausgewählt ist aus der Gruppe, die Alkyl, Cycloalkyl, Heterocyclyl, Aryl und Heteroaryl umfasst;
B und D einzeln und unabhängig voneinander ein aromatisches oder heteroaromatisches Radikal sind, wobei bevorzugterweise das aromatische Radikal Aryl ist, und bevorzugterweise das heteroaromatische Radikal Heteroaryl ist.

In einer Ausführungsform ist vorgesehen, dass A und C einzeln und unabhängig voneinander ausgewählt sind aus der Gruppe, die C3-C6-Alkyl, C5-C7-Cycloalkyl, Methylthioethyl, Indolyl, Phenyl, Naphtyl, Thienyl, Propenyl, Propinyl, Hydroxyphenyl, Indolyl und Imidazolyl umfasst;
B ausgewählt ist aus der Gruppe, die Phenyl, Naphthyl, Thienyl, Benzothienyl, Hydroxyphenyl, Indolyl, und Imidazolyl umfasst; und
D ausgewählt ist aus der Gruppe, die Phenyl, Naphthyl, Thienyl, Thiazolyl, Furanyl, Hydroxyphenyl, Indolyl und Imidazolyl umfasst.

In einem fünften Aspekt wird die Aufgabe erfindungsgemäß gelöst durch eine Verbindung, bevorzugterweise einen C5a-Rezeptor-Antagonist,
mit folgender Struktur: 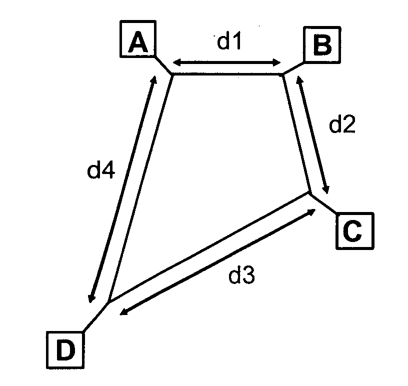 , wobei
A, B, C und D die C-alpha-Atome in Aminosäuren, Aminosäure-Analoga oder AminosäureDerivaten bezeichnen,
d1, d2, d3 und d4 die Abstände zwischen A, B, C und D in wenigstens einem energetisch zugänglichen Konformer der Verbindung bezeichnen und die folgenden Werte aufweisen:
d1 = 3,9 ± 0,5 Å
d2 = 3,9 ± 0,5 Å
d3 = 9,0 ± 1,5 Å
d4 = 9,0 ± 1,5 Å;

A und C einzeln und unabhängig voneinander eine hydrophobe Aminosäure-Seitenkette sind, die eine Alkyl-, Cycloalkyl, Heterocyclyl, Aryl- oder Heteroaryl-Gruppe umfasst.

B und D einzeln und unabhängig voneinander eine aromatische oder heteroaromatische Aminosäure-Seitenkette sind, die eine Aryl- oder Heteroaryl-Gruppe umfasst.

In einer Ausführungsform ist vorgesehen, dass
A ausgewählt ist aus der Gruppe, die C3-C6-Alkyl, Methylthioethyl, Propenyl, Propinyl, R5, Methyl-R5 und Ethyl-R5 umfasst, wobei R5 ein Radikal ist, das ausgewählt ist aus der Gruppe, die C5-C7-Cycloalkyl, Phenyl, substituiertes Phenyl, Hydroxyphenyl, Indolyl, Imidazolyl, Naphtyl und Thienyl umfasst;
B ausgewählt ist aus der Gruppe, die R5, Methyl-R5 und Ethyl-R5 umfasst, wobei R5 ausgewählt ist aus der Gruppe, die Phenyl, substituiertes Phenyl, Naphtyl, Thienyl, Benzothienyl, Hydroxyphenyl, Indolyl und Imidazolyl umfasst;
C ausgewählt ist aus der Gruppe, die C3-C6-Alkyl, R5, Methyl-R5 und Ethyl-R5 umfasst, wobei R5 ein Radikal ist, das aus der Gruppe ausgewählt ist, die C5-C7-Cycloalkyl, Phenyl, 1-Methyl-Phenyl, 2-Methyl-Phenyl und 3-Methyl-Phenyl umfasst; und
D ausgewählt ist aus der Gruppe, die R5, Methyl-R5 und Ethyl-R5 umfasst, wobei R5 ausgewählt ist aus der Gruppe, die Phenyl, Naphthyl, Thienyl, Thiazolyl, Furanyl, Hydroxyphenyl, Indolyl und Imidazolyl umfasst.

In einem sechsten Aspekt wird die Aufgabe erfindungsgemäß gelöst durch eine Verbindung, bevorzugterweise einen C5a-Rezeptor-Antagonist, mit der folgenden Struktur:

X1-X2-X3-X4-X5-X6-X7-X8 (II)

, wobei
X1 ein Radikal mit einer Masse von etwa 1-300 ist und wobei X1 bevorzugterweise ausgewählt ist aus der Gruppe, die R5-, R5-CO-, R5-N(R6)-CO-, R5-O-CO-, R5-SO₂-, R5-N(R6)-SO₂-, R5-N(R6)-, R5-N(R6)-CS-, R5-N(R6)-C(NH)-, R5-CS-, R5-P(O)OH-, R5-B(OH)-, R5-CH=N-O-CH₂-CO- umfasst, wobei R5 und R6 einzeln und unabhängig voneinander ausgewählt sind aus der Gruppe, die H, F, Hydroxy, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, Acyl, substituiertes Acyl, Alkoxy, Alkoxyalkyl, substituiertes Alkoxyalkyl, Aryloxyalkyl und substituiertes Aryloxyalkyl umfasst,
X2 ein Radikal ist, das die biologischen Bindungseigenschaften einer Phenylalanin-Einheit mimikt,
X3 und X4 einzeln und unabhängig voneinander ein Spacer ist, wobei der Spacer bevorzugterweise aus der Gruppe ausgewählt ist, die Aminosäuren, Aminosäure-Analoga und Aminosäure-Derivate umfasst,
X5 ein Radikal ist, das die biologischen Bindungseigenschaften einer Cyclohexylalanin-Einheit mimikt,
X6 ein Radikal ist, das die biologischen Bindungseigenschaften einer Tryptophan-Einheit mimikt,
X7 ein Radikal ist, das die biologischen Bindungseigenschaften einer Norleucin- oder Phenylalanin-Einheit mimikt,
X8 ein Radikal ist, wobei das Radikal optional in Struktur I enthalten ist und wenn es enthalten ist, ausgewählt ist aus der Gruppe, die H, NH₂, OH, NH-OH, Amino, substituiertes Amino, Alkoxy, substituiertes Alkoxy, Hydrazino, substituiertes Hydrazino, Aminooxy, substituiertes Aminooxy, Alkyl, substituiertes Alkyl, Cycloalkyl, substituiertes Cycloalkyl, Heterocyclyl, substituiertes Heterocyclyl, Heteroaryl, substituiertes Heteroaryl, Arylalkyl, substituiertes Arylalkyl, Aryl, substituiertes Aryl, Aminosäure, Aminosäurederivat und Aminosäureanalogon umfasst;
die Verbindungslinien - in Formel (II) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

In einer Ausführungsform ist vorgesehen, dass
X1 ein Radikal mit einer Masse von etwa 1-300 ist, wobei das Radikal bevorzugterweise ausgewählt ist aus der Gruppe, die R5, R5-CO-, R5-N(R6)-CO-, R5-O-CO-, R5-SO₂-, R5-N(R6)-C(NH)-, umfasst, wobei bevorzugtererweise R5 und R6 einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl und substituiertes Aryl enthält;
X2 und X6 jeweils und unabhängig voneinander eine aromatische Aminosäure, ein Derivat oder ein Analogon davon ist;
X5 und X7 einzeln und unabhängig voneinander eine hydrophobe Aminosäure, ein Derivat oder ein Analogon davon sind.

In einer bevorzugten Ausführungsform ist vorgesehen, dass X1 ausgewählt ist aus der Gruppe, die H, Acetyl, Propanoyl, Butanoyl, Benzoyl, Fluormethylcarbonyl, Difluormethylcarbonyl, Phenyl, Oxycarbonyl, Methyl-oxycarbonyl, Phenyl-aminocarbonyl, Methyl-aminocarbonyl, Phenyl-sulfonyl und Methyl-sulfonyl umfasst.

In einer Ausführungsform ist vorgesehen, dass X2, X5, X6 und X7 einzeln und unabhängig voneinander die folgende Struktur aufweisen: 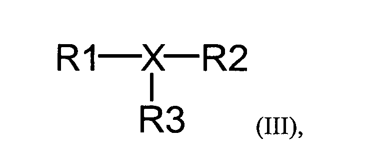 worin
X C(R4) oder N ist,
R1 ein Radikal ist, das ausgewählt ist aus der Gruppe, die >N-R1B, >C(R1B)(R1C) und >O umfasst, wobei R1B und R1C einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, S=O, C=NH, C=N-CN, PO(OH), B(OH), CH₂, CH₂CO, CHF und CF₂ umfasst;
R4 ein Radikal ist, wobei das Radikal ausgewählt ist aus der Gruppe, die H, F, CH₃, CF₃, Alkyl und substituiertes Alkyl umfasst;
und die Bindung von Struktur (III) an die Molekülbestandteile X1 und X3, X4 und X6, X5 und X7, und X6 und X8 bevorzugt über R1 und R2 erfolgt;
für X2 und für X6 einzeln und unabhängig voneinander R3 ein Radikal ist, wobei das Radikal eine aromatische Gruppe enthält und ausgewählt ist aus der Gruppe, die Aryl, substituiertes Aryl, Heteroaryl, substituiertes Heteroaryl, Arylalkyl, substituiertes Arylalkyl, Heteroarylalkyl, substituiertes Heteroarylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst; und
für X5 und für X7 einzeln und unabhängig voneinander R3 ein Radikal ist, wobei das Radikal eine aliphatische oder aromatische Gruppe enthält und bevorzugterweise ausgewählt ist aus der Gruppe, die Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Cycloalkylalkyl, substituiertes Cycloalkylalkyl, Heterocyclylalkyl, substituiertes Heterocyclylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst.

In einer bevorzugten Ausführungsform ist vorgesehen, dass unter Beteiligung von R3 und R4 ein Ring ausgebildet wird.

In einer weiteren bevorzugten Ausführungsform ist vorgesehen, dass für X2 und für X6 einzeln und unabhängig voneinander R3 aus der Gruppe ausgewählt ist, die Phenyl, substituiertes Phenyl, Benzyl, substituiertes Benzyl, 1,1-Diphenylmethyl, substituiertes 1,1-Diphenylmethyl, Naphthylmethyl, substituiertes Naphthylmethyl, Thienylmethyl, substituiertes Thienylmethyl, Benzothienylmethyl, substituiertes Benzothienylmethyl, Imidazolylmethyl, substituiertes Imidazolylmethyl, Indolylmethyl und substituiertes Indolylmethyl umfasst.

In einer Ausführungsform ist vorgesehen, dass für X5 und für X7 einzeln und unabhängig voneinander R3 aus der Gruppe ausgewählt ist, die C3-C5-Alkyl, substituiertes C3-C5-Alkyl, C5-C7-Cycloalkyl, substituiertes C5-C7-Cycloalkyl, C5-C7-Cycloalkylmethyl, substituiertes C5-C7-Cycloalkylmethyl, Cycloalkylethyl, substituiertes Cycloalkylethyl, Benzyl, substituiertes Benzyl, Phenylethyl, Naphthylmethyl, Thienylmethyl, Propenyl, Propinyl, Methylthioethyl, Imidazolylmethyl, substituiertes Imidazolylmethyl, Indolylmethyl und substituiertes Indolylmethyl umfasst.

In einer weiteren Ausführungsform ist vorgesehen, dass
X2 ein Aminosäurederivat einer Aminosäure ist, das ausgewählt ist aus der Gruppe, die Phenylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine, 1-Naphtylalanin, 2-Thienylalanin, 3-Thienylalanin, 3,3-Diphenylalanin, Tyrosin, Tryptophan, Histidin und jeweilige Derivate davon umfasst;
oder X2 und X1 sind zusammengenommen PhCH₂CH₂CO- oder PhCH₂-;
X6 ein Aminosäurederivat einer Aminosäure ist, die ausgewählt ist aus der Gruppe, die Tryptophan, Phenylalanin, Tyrosin, Histidin, 1-Naphtylalanin, Benzothienylalanin, 2-Aminoindan-2-carbonsäure, 2-Thienylalanin, 3-Thienylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine und jeweilige Derivate davon umfasst;
X5 ein Aminosäurederivat einer Aminosäure ist, die ausgewählt ist aus der Gruppe, die D-Cyclohexylalanin, D-Cyclohexylglycin, D-Homo-Cyclohexylalanin, Octahydroindol-2-carbonsäure, 2-Methyl-D-Phenylalanin und jeweilige Derivate davon umfasst; und
X7 ein Aminosäurederivat einer Aminosäure ist, die ausgewählt ist aus der Gruppe, die Norvalin, Norleucin, Homo-Leucin, Leucin, Isoleucin, Valin, Cystein, Cystein(Me), Cystein(Et), Cystein(Pr), Methionin, Allylglycin, Propargylglycin, Cyclohexylglycin, Cyclohexylalanin, Phenylalanin, Tyrosin, Tryptophan, Histidin, 1-Naphtylalanin, 2-Thienylalanin, 3-Thienylalanin und jeweilige Derivate davon umfasst.

In einer noch weiteren Ausführungsform ist vorgesehen, dass X1 und/oder X4 eine oder mehrere die Wasserlöslichkeit verbessernde Gruppen aufweisen, wobei die die Wasserlöslichkeit verbessernde Gruppe ausgewählt ist aus der Gruppe, die Hydroxy, Keto, Carboxamido, Ether, Harnstoff, Carbamat, Amino, substituiertes Amino, Guanidino, Pyridyl und Carboxyl umfasst.

In einem siebten Aspekt wird die Aufgabe erfindungsgemäß gelöst durch eine Verbindung, bevorzugterweise einen C5a-Rezeptor-Antagonist, mit der Struktur

X1-X2-X3-X4-X5-X6-X7-X8 (II)

, wobei X1-X3 und X5-X8 definiert sind, wie in einem der Ansprüche 17 bis 26 und wobei
X4 eine zyklische oder eine nichtzyklische Aminosäure ist, wobei die zyklische Aminosäure ausgewählt ist aus der Gruppe, die Prolin, Pipecolinsäure, Azetidin-2-carbonsäure, Tetrahydroisochinolin-3-carbonsäure, Tetrahydroisochinolin-1-carbonsäure, Octahydroindol-2-carbonsäure, 1-Aza-bicyclo-[3.3.0]-octan-2-carbonsäure, 4-Phenyl-pyrrolidin-2-carbonsäure, cis-Hyp und trans-Hyp umfasst und die nichtzyklische Aminosäure ausgewählt aus der Gruppe, die Ser, Gln, Asn, Cys(O₂CH₂CH₂CONH₂), Arg, Hyp(COCH₂OCH₂CH₂OCH₂CH₂OCH₃), Hyp(CONH-CH₂CH(OH)-CH₂OH) und jeweilige Derivate davon und jeweilige Analoga davon umfasst; und
die Verbindungslinien - in Formel (I) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

In einer Ausführungsform ist dabei vorgesehen, dass die Derivate und/oder Analoga so ausgebildet sind, dass einzelne oder mehrere Wasserstoffatome der genannten Aminosäuren substituiert sein können.

In einer Ausführungsform ist vorgesehen, dass die Aminosäuren bevorzugt ausgewählt sind aus der Gruppe, die Prolin, Pipecolinsäure, Azetidin-2-carbonsäure, Tetrahydroisochinolin-3-carbonsäure, Tetrahydroisochinolin-1-carbonsäure, Octahydroindol-2-carbonsäure, 1-Azabicyclo-[3.3.0]-octan-2-carbonsäure, 4-Phenyl-pyrrolidin-2-carbonsäure, Hyp, Ser, Gln, Asn, Cys(O₂CH₂CH₂CONH₂) und Arg umfasst.

In einem achten Aspekt wird die Aufgabe erfindungsgemäß gelöst durch eine Verbindung, bevorzugterweise ein C5a-Rezeptor-Antagonist, mit der Struktur

X1--X2--X3--X4--X5--X6--X7--X8 (II)

, wobei X1-X2 und X4-X8 definiert sind, wie in einem der Ansprüche 19 bis 29 und wobei
X3 folgende Struktur aufweist: 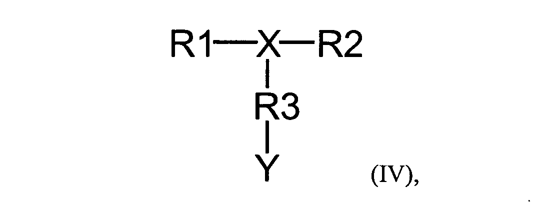 worin
X C(R4) oder N ist,
R1 optional vorhanden ist und wenn R1 vorhanden ist, R1 ein Radikal ist, das ausgewählt ist aus der Gruppe, die >N-R1B, >C(R1B)(R1C) und >O umfasst, wobei R1B und R1C unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, PO(OH), B(OH), CH₂, CH₂CO, CHF und CF₂ umfasst;
R4 ein Radikal ist, wobei das Radikal ausgewählt ist aus der Gruppe, die H, F, CH₃, CF₃, Alkyl und substituiertes Alkyl umfasst;
die Bindung von Struktur (IV) an die Molekülbestandteile X2 und X4 bevorzugt über R1 und R2 erfolgt;
R3 ein Radikal ist, das aus der Gruppe ausgewählt ist, die H, Alkyl, substituiertes Alkyl, Cycloalkyl, substituiertes Cycloalkyl, Cycloalkylalkyl, substituiertes Cycloalkylalkyl, Heterocyclyl, substituiertes Heterocyclyl, Heterocyclylalkyl, substituiertes Heterocyclylalkyl, Aryl, substituiertes Aryl, Arylalkyl, substituiertes Arylalkyl, Heteroaryl, substituiertes Heteroaryl, Heteroarylalkyl, substituiertes Heteroarylalkyl, Acyl, substituiertes Acyl, Alkoxyalkyl, substituiertes Alkoxyalkyl, Aryloxyalkyl, substituiertes Aryloxyalkyl, Sulfhydrylalkyl, substituiertes Sulfhydrylalkyl, Hydroxyalkyl, substituiertes Hydroxyalkyl, Carboxyalkyl, substituiertes Carboxyalkyl, Carboxamidoalkyl, substituiertes Carboxamidoalkyl, Carboxyhydrazinoalkyl, Ureidoalkyl Aminoalkyl, substituiertes Aminoalkyl, Guanidinoalkyl und substituiertes Guanidinoalkyl umfasst.
Y optional vorhanden ist und wenn Y vorhanden ist, Y ein Radikal ist, das ausgewählt ist aus der Gruppe, die H, -N(YB1)-CO-YB2, -N(YB1)-CO-N(YB2)(YB3), -N(YB1)-C(N-YB2)-N(YB3)(YB4), -N(YB1)(YB2), -N(YB1)-SO₂-YB2, O-YB1, S-YB1, -CO-YB1, -CO-N(YB1)(YB2) und -C=N-O-YB1 umfasst, wobei YB1, YB2, YB3 und YB4 einzeln und unabhängig voneinander ausgewählt sind aus der Gruppe, die H, CN, NO₂ Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst.

In einer Ausführungsform ist vorgesehen, dass
R3 ein Radikal ist mit der Struktur

-(CH₂)ₘ-Y (V)

oder

-(CH₂)ₘ-C₆H₄-Y (VI)

, wobei
m 1, 2, 3 oder 4 ist;
Y N(R3b)(R3c) oder -N(YB1)-C(N-YB2)-N(YB3)(YB4) ist, wobei R3b, R3c, YB1, YB2, YB3 und YB4 einzeln und unabhängig voneinander ausgewählt sind aus der Gruppe, die H, CN und Alkyl umfasst.

In einer Ausführungsform ist vorgesehen, dass ein Ring zwischen jeweils zwei Gruppierungen ausgebildet ist, wobei die Gruppierungen einzeln und unabhängig voneinander aus der Gruppe ausgewählt sind, die YB1, YB2, YB3 und YB4 umfasst.

In einer weiteren Ausführungsform ist vorgesehen, dass der Ring durch YB2 und YB3 ausgebildet wird.

In einer besonders bevorzugten Ausführungsform ist vorgesehen, dass Y-NH₂ oder

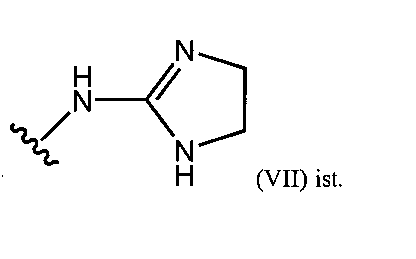

In einer Ausführungsform eines jeglichen Aspektes der vorliegenden Erfindung ist vorgesehen, dass die Verbindung eine der folgenden Verbindungen ist:

| **Nr.** | **Verbindung** |
|---|---|
| **1** | Ac-Phe-[Orn-Pro-cha-Trp-Phe] |
| **2** | Ac-Phe-[Orn-Hyp-cha-Trp-Phe] |
| **3** | HOCH₂(CHOH)₄-C=N-O-CH₂-CO-Phe-[Orn-Pro-cha-Trp-Nle] |
| **4** | X-Phe-[Orn-Pro-cha -Trp-Nle]; X = 2-Acetamido-1-Methyl-Glucuronyl |
| **5** | Ac-Phe-[Orn-Hyp(COCH₂OCH₂CH₂OCH₂CH₂OCH₃)-cha-Trp-Nle] |
| **6** | Ac-Phe-[Orn-Hyp(CONH-CH₂CH(OH)-CH₂OH)-cha-Trp-Nle] |
| **20** | Ac-Phe-[Orn-Pro-cha-Trp-Ecr] |
| **28** | Ac-Phe-[Orn-Pro-cha-Trp-Nle] |
| **29** | Ac-Phe-[Orn-Pro-cha-Trp-Met] |
| **31** | Ac-Phe-[Orn-Pro-cha-Trp-Nva] |
| **32** | Ac-Phe-[Orn-Pro-cha-Trp-Hle] |
| **33** | Ac-Phe-[Orn-Pro-cha-Trp-Eaf] |
| **34** | Ac-Phe-[Orn-Pro-cha-Trp-Ebd] |
| **35** | Ac-Phe-[Orn-Pro-cha-Trp-Eag] |
| **36** | Ac-Phe-[Orn-Pro-cha-Trp-Pmf] |
| **37** | Ac-Phe-[Orn-Pro-cha-Trp-2Ni] |
| **38** | Ac-Phe-[Orn-Pro-cha-Trp-Thi] |
| **41** | Ph-CH₂-CH₂-CO-[Orn-Pro-cha-Trp-Nle] |
| **42** | H-Phe-[Orn-Pro-cha-Trp-Nle] |
| **43** | Ac-Lys-Phe-[Orn-Pro-cha-Trp-Nle] |
| **44** | H-Phe-[Orn-Ser-cha-Trp-Nle] |
| **51** | Ac-Phe-Orn-Pro-cha-Trp-Phe-NH_{Z} |
| **52** | Ac-Phe-Orn-Aze-cha-Bta-Phe-NH_{Z} |
| **53** | Ac-Phe-Orn-Pro-cha-Bta-2Ni-NH_{Z} |
| **54** | Ac-Phe-Orn-Pro-cha-Bta-Cha-NH₂ |
| **55** | Ac-Phe-Orn-Pip-cha-Trp-Phe-NH_{Z} |
| **56** | Ph-CH₂-[Orn-Pro-cha-Trp-Nle] |
| **57** | Ph-CH₂-[Orn-Pro-cha-Trp-Phe] |
| **58** | Ac-Phe-[Orn-Pro-cha-Trp-1Ni] |
| **59** | Ph-CH(OH)-CH₂-CO-[Orn-Pro-cha-Trp-Nle] |
| **61** | Ac-Phe-Orn-Pro-cha-Trp-Phe-NH₂ |
| **62** | Ac-Phe-Orn-Pro-cha-Bta-Phe-NH₂ |
| **64** | Ac-Phe-Orn-Pro-cha-Trp-2Ni-NH₂ |
| **65** | Ac-Phe-Orn-Pro-cha-Trp-Cha-NH₂ |
| **66** | Ac-Thi-Orn-Aze-cha-Bta-Phe-NH₂ |
| **67** | Ac-Thi-Orn-Pip-cha-Bta-Phe-NH₂ |
| **68** | Ac-Phe-Orn-Pro-cha-Trp-Eap-NH₂ |
| **69** | Me₂-Phe-Om-Pro-cha-Trp-Phe-NH₂ |
| **70** | Ph₂-CH-CH₂-CO-Orn-Pro-cha-Trp-Phe-NH₂ |
| **71** | Ac-Ebw-Orn-Pro-cha-Trp-Phe-NH₂ |
| **72** | Ac-Phe-Orn-Pro-cha-Trp-NH-CH₂-CH₂-Ph |
| **73** | Ac-Phe-Orn-Aze-cha-Bta-NH-CH₂-CH₂-Ph |
| **74** | H-Phe-Orn-Pro-cha-Trp-Phe-NH₂ |
| **75** | H-Me-Phe-Orn-Pro-cha-Trp-Phe-NH₂ |
| **76** | Bu-NH-CO-Phe-Orn-Pro-cha-Trp-Phe-NH₂ |
| **77** | Ac-Thi-Orn-Pro-cha-Trp-Phe-NH₂ |
| **78** | Ac-Ebw-Orn-Pro-cha-Trp-Phe-NH₂ |
| **79** | Ac-Phe-Orn-Ala-cha-Trp-Phe-NH₂ |
| **80** | Ac-Phe-Orn-Pro-cha-Trp-Thi-NH₂ |
| **81** | Ac-Phe-Orn-Aze-cha-Pcf-Phe-NH₂ |
| **82** | Ac-Phe-Orn(Ac)-Pro-cha-Trp-Phe-NH₂ |
| **83** | Ac-Phe-Orn-Aze-cha-Trp-Phe-NH₂ |
| **84** | Ac-Phe-Trp-Pro-cha-Trp-Phe-NH₂ |
| **85** | Ph-NH-CO-Phe-Orn-Pro-cha-Trp-Phe-NH₂ |
| **86** | Bu-O-CO-Phe-Orn-Pro-cha-Trp-Phe-NH₂ |
| **87** | Ac-Phe-Lys-Pro-cha-Trp-Phe-NH₂ |
| **88** | Ac-Phe-Arg-Pro-cha-Trp-Phe-NH₂ |
| **89** | Ac-Phe-Gln-Pro-cha-Trp-Phe-NH₂ |
| **92** | Ac-Phe-Orn-Pip-cha-Trp-Phe-NH₂ |
| **93** | Ac-Phe-Orn-Hyp-cha-Trp-Phe-NH₂ |
| **94** | Ac-Phe-Orn-Pro-cha-Trp-1Ni-NH₂ |
| **95** | Ac-Phe-Orn-Aze-cha-Bta-Phe-NH-Me |
| **96** | CH₃-SO₂-Phe-Orn-Aze-cha-Bta-Phe-NH₂ |
| **99** | Ac-Phe-Orn-Aze-cha-Pff Phe-NH₂ |
| **100** | Ac-Phe-Orn-Aze-cha-Mcf-Phe-NH₂ |
| **101** | Ac-Phe-Orn(Ac)-Aze-cha-Bta-Phe-NH₂ |
| **102** | Ac-Ebw-Orn-Pro-cha-Trp-Phe-NH₂ |
| **103** | Ac-Phe-Trp-Pro-cha-Trp-Phe-NH₂ |
| **104** | Ac-Phe-Arg-Pro-cha-Trp-Phe-NH₂ |
| **105** | Ac-Phe-Orn-Pip-cha-Trp-Phe-NH₂ |
| **106** | 3PP-Orn-Aze-cha-Bta-Phe-NH₂ |
| **107** | Ac-Phe-Orn-Tic-cha-Trp-Phe-NH₂ |
| **108** | Ac-Phe-Orn-Ser-cha-Trp-Phe-NH₂ |
| **109** | Ac-Phe-Orn-Pro-chg-Trp-Phe-NH₂ |
| **110** | Ac-Phe-Orn-Pro-hch-Trp-Phe-NH₂ |
| **111** | Ac-Phe-Orn-Pro-cha-Trp-Phg-NH₂ |
| **112** | Ac-Phe-Bta-Aze-cha-Bta-Phe-NH₂ |
| **113** | Ac-Phe-Trp-Pro-cha-Bta-Phe-NH₂ |
| **115** | Ac-Phe-Orn-Pip-cha-Trp-Phe-OH |
| **116** | Ac-Phe-Orn-Tic-cha-Trp-Phe-OH |
| **117** | Ac-Phe-Orn-Ser-cha-Trp-Phe-OH |
| **118** | Ac-Phe-Orn-Pro-chg-Trp-Phe-OH |
| **119** | Ac-Phe-Eec-Pro-cha-Bta-Phe-NH₂ |
| **120** | Ac-Phe-Nle-Pro-cha-Bta-Phe-NH₂ |
| **121** | Ac-Phe-Har-Pro-cha-Bta-Phe-NH₂ |
| **122** | Ac-Phe-Arg-Pro-cha-Bta-Phe-NH₂ |
| **123** | Ac-Phe-Cys(Acm)-Pro-cha-Bta-Phe-NH₂ |
| **124** | Ac-Phe-Mpa-Pro-cha-Bta-Phe-NH₂ |
| **125** | Ac-Eby-Orn-Pro-cha-Bta-Phe-NH₂ |
| **126** | Ac-Phg-Orn-Pro-cha-Bta-Phe-NH₂ |
| **127** | Ac-Phe-Paf-Pro-cha-Bta-Phe-NH₂ |
| **128** | H₂N-CO-Phe-Orn-Pro-cha-Bta-Phe-NH₂ |
| **129** | Me-O-CO-Phe-Orn-Pro-cha-Bta-Phe-NH₂ |
| **130** | (-CO-CH₂-NH-CO-)-Phe-Orn-Pro-cha-Bta-Phe-NH₂ |
| **132** | Ac-Phe-Orn-Pro-hch-Trp-Phe-OH |
| **133** | (-CO-CH₂-CH₂-CO-)-Phe-Orn-Pro-cha-Bta-Phe-NH₂ |
| **134** | ^{t}Bu-CO-Phe-Orn-Pro-cha-Bta-Phe-NH₂ |
| **135** | Ac-Lys-Phe-Orn-Aze-cha-Bta-Phe-NH₂ |
| **136** | Ac-Gly-Phe-Orn-Aze-cha-Bta-Phe-NH₂ |
| **137** | Ac-Arg-Phe-Orn-Aze-cha-Bta-Phe-NH₂ |
| **138** | Ac-His-Phe-Orn-Aze-cha-Bta-Phe-NH₂ |
| **139** | Ac-Ser-Phe-Orn-Aze-cha-Bta-Phe-NH₂ |
| **140** | Ac-Guf-Phe-Orn-Aze-cha-Bta-Phe-NH₂ |
| **141** | Ac-Dab-Phe-Orn-Aze-cha-Bta-Phe-NH₂ |
| **142** | FH₂C-CO-Phe-Orn-Pro-cha-Bta-Phe-NH₂ |
| **143** | Ac-Phe-Orn(Et₂)-Pro-cha-Trp-Phe-NH₂ |
| **144** | Ac-Phe-[Orn-Hyp-cha-Trp-Nle] |
| **145** | 3PP-[Orn-Hyp-cha-Trp-Nle] |
| **146** | Ac-Phe-[Orn-Pro-cha-Trp-Tyr] |
| **147** | Ac-Phe-[Orn-Pro-omf Trp-Nle] |
| **149** | Ac-Phe-Orn-Pro-hle-Bta-Phe-NH₂ |
| **150** | Ac-Phe-Arg(CH₂-CH₂)-Pro-cha-Bta-Phe-NH₂ |

In einem neunten Aspekt wird die Aufgabe erfindungsgemäß gelöst durch eine pharmazeutische Formulierung umfassend mindestens eine Verbindung gemäß einem der Aspekte der vorliegenden Erfindung und zusätzlich ein pharmazeutisch akzeptables Trägermittel.

In einem zehnten Aspekt wird die Aufgabe erfindungsgemäß gelöst durch die Verwendung mindestens einer Verbindung nach einem der Aspekte der vorliegenden Erfindung zur Herstellung eines Medikamentes.

In einer Ausführungsform ist vorgesehen, dass das Medikament für die Prävention und/oder Behandlung einer Erkrankung verwendet wird, bei der das Komplementsystem aktiviert ist und/oder bei der die Inhibierung des Komplementsystems eine Linderung der Symptome hervorruft.

In einer weiteren Ausführungsform ist vorgesehen, dass die Krankheit und/oder die zu behandelnden Symptome ausgewählt sind aus der Gruppe, die Rheumatoide Arthritis, systemischen Lupus erythematodes, Multiple Sklerose, Psoriasis, septischer Schock, Asthma, Vaskulitis, Dermatomyositis, entzündliche Darmerkrankungen (IBD: inflammatory bowel disease) Pemphigus, Myasthenia gravis, akute respiratorische Insuffizienz, Gehirnschlag, Herzinfarkt, Reperfusionsschaden, Verbrennungen und akute Verletzungen des zentralen Nervensystems umfasst.

In einem elften Aspekt wird die Aufgabe erfindungsgemäß gelöst durch die Verwendung mindestens einer Verbindung nach einem der Aspekte der vorliegenden Erfindung zur Prävention und/oder Unterstützung chirurgischer Eingriffe.

In einer Ausführungsform des zehnten Aspektes der vorliegenden Erfindung ist vorgesehen, dass das Medikament zur Prävention und/oder Unterstützung chirurgischer Eingriffe verwendet werden.

In einer weiteren Ausführungsform des zehnten Aspektes ist vorgesehen, dass das Medikament zur Unterstützung und/oder zur Prävention und/oder Nachsorge eines chirurgischen Eingriffs verwendet werden, wobei der chirurgische Eingriff ausgewählt ist aus der Gruppe, die CABG, PACT; PTA; MidCAB, OPCAB, , Thrombolyse und Gefäßverschluss (clamping) umfasst.

In einer noch weiteren Ausführungsform des zehnten Aspektes ist vorgesehen, dass das Medikament für die thrombolytische Behandlung verwendet wird.

In einer noch weiteren Ausführungsform des zehnten Aspektes ist vorgesehen, dass das Medikament im Rahmen einer Dialyse-Behandlung, gegebenenfalls vor, während oder danach, verwendet wird.

In einem weiteren Aspekt ist vorgesehen, dass die erfindungsgemäßen Verbindungen zur Herstellung eines Medikaments zur Unterstützung von chirurgischen Eingriffen und/oder zur Prävention von unerwünschten Nebenwirkungen bei chirurgischen Eingriffen und/oder Nachsorge einer Krankheit verwendet werden, wobei die Krankheit ausgewählt ist aus der Gruppe, die Herzinfarkt, CABG (Coronary Artery Bypass Surgery), PTCA (Percutaneous Transluminal Coronary Angioplasty); PTA (Percutaneous Transluminal Angioplasty); MidCAB (Minimally Invasive Direct Coronary Artery Bypass), OPCAB (Off Pump Coronary Artery Bypass), Gehimschlag, Thrombolyse, Gefäßverschluss und Verbrennungen umfasst. In einer weiteren Ausführungsform können die erfindungsgemäßen Verbindungen verwendet werden für die Herstellung eines Medikamentes zur Unterstützung einer thrombolytischen Behandlung. In einer weiteren Ausführungsform werden die erfindungsgemäßen Verbindungen bei der Dialyse verwendet bzw. zur Herstellung eines Medikamentes verwendet, das im Rahmen einer Dialyse-Behandlung, gegebenenfalls vor, während oder danach, gegeben wird. Dabei sollen die negativen Wirkungen bei der extrakorporalen Zirkulation vermieden oder verringert werden.

In einem noch weiteren Aspekt betrifft die vorliegende Erfindung ein Verfahren zur Behandlung von Patienten, wobei das Verfahren die Verabreichung einer oder mehrerer der erfindungsgemäßen Verbindungen umfasst. Die Behandlung kann dabei eine Behandlung im engeren Sinne sein, schließt jedoch auch eine präventive Behandlung und eine Nachfolgebehandlung ein. Bei dem zu behandelnden Patienten handelt es sich bevorzugterweise um ein Säugetier, bevorzugtererweise um landwirtschaftliche Nutztiere, Sport- und Haustiere, und bevorzugtesterweise um den Menschen. In einer bevorzugten Ausführungsform ist der Patient ein solcher, der einer Behandlung bedarf. In einer weiteren bevorzugten Ausführungsform leidet der Patient unter einer der vorstehenden Erkrankungen, für deren Behandlung und/oder Prävention die erfindungsgemäßen Verbindungen verwendet werden können.

Damit stellt die vorliegende Erfindung erstmals solche Antagonisten des C5a-Rezeptors bereit, die die inhärenten pharmakologischen Nachteile der mit positiver Ladung versehenen antagonistisch aktiven Peptide des Standes der Technik überwinden.

Der vorliegenden Erfindung liegt die überraschende Erkenntnis zugrunde, dass im Gegensatz zur Lehre des Standes der Technik, auch Antagonisten des C5a-Rezeptors erhalten werden können, die unter physiologischen Bedingungen, insbesondere bei einem pH von 7.4, keine positive Nettoladung tragen und/oder deren C-terminale Aminosäure unter physiologischen Bedingungen keine positive Ladung aufweist.

Die positive Ladung in Peptiden kann aus pharmakologischer Sicht sehr nachteilig sein. So können positive Ladungen z.B. zu Histamin-Freisetzung führen und geringere Membrangängigkeit verursachen (vgl. hierzu Beispiel 15). Es ist deshalb besonders wünschenswert, einen peptidischen Antagonisten, der keine positive Nettoladung besitzt (im folgenden auch als Verbindung bezeichnet), zu entwickeln.

Die in der vorliegenden Erfindung genannten Verbindungen wurden in einem primären Test auf ihre IC₅₀-Werte in einem funktionellen Testsystem geprüft. Bevorzugterweise gelten alle Verbindungen, Peptide und Peptidomimetika als im Sinne der vorliegenden Erfindung nennenswert inhibitorisch aktiv, die einen IC₅₀-Wert von weniger als 200 nM in einem funktionellen Assay, wie er in Beispiel 1 beschreiben ist, zeigen.

Insbesondere handelt es sich bei den erfindungsgemäßen Verbindungen um Antagonisten des C5a-Rezeptors. Noch bevorzugterweise sind diese als Peptide oder Peptidomimetika ausgebildet. Weiterhin liegt der vorliegenden Erfindung die überraschende Erkenntnis zugrunde, dass die erfindungsgemäß als Antagonisten des C5a-Rezeptors zu verwendenden Verbindungen eine ungeladene C-terminale Aminosäure, Aminosäurederivat oder Aminosäureanalogon tragen.

Im Rahmen der vorliegenden Erfindung wurde jedoch auch überraschenderweise gefunden, dass lineare, also strukturell flexible Peptide ebenso potente Inhibitoren sein können, wie strukturell fixierte zyklische Peptide. Bedingung dafür ist die Substitution des C-terminalen geladenen Arginins durch hydrophobe Aminosäuren, Aminosäure-Derivate oder Aminosäure-Analoga. Beispiele für derartige erfindungsgemäße peptidische Inhibitoren sind insbesondere die in der folgenden Tabelle aufgeführten Verbindungen:.

| | |
|---|---|
| **51** | Ac-Phe-Orn-Pro-cha-Trp-Phe-NH₂ |
| **52** | Ac-Phe-Orn-Aze-cha-Bta-Phe-NH₂ |
| **53** | Ac-Phe-Orn-Pro-cha-Bta-2Ni-NH₂ |
| **54** | Ac-Phe-Orn-Pro-cha-Bta-Cha-NH₂ |
| **55** | Ac-Phe-Orn-Pip-cha-Trp-Phe-NH₂ |
| **61** | Ac-Phe-Orn-Pro-cha-Trp-Phe-NH₂ |
| **62** | Ac-Phe-Orn-Pro-cha-Bta-Phe-NH₂ |
| **64** | Ac-Phe-Orn-Pro-cha-Trp-2Ni-NH₂ |
| **65** | Ac-Phe-Orn-Pro-cha-Trp-Cha-NH₂ |
| **66** | Ac-Thi-Orn-Aze-cha-Bta-Phe-NH₂ |
| **67** | Ac-Thi-Orn-Pip-cha-Bta-Phe-NH₂ |
| **68** | Ac-Phe-Orn-Pro-cha-Trp-Eap-NH₂ |
| **69** | Me₂-Phe-Orn-Pro-cha-Trp-Phe-NH₂ |
| **70** | Ph₂-CH-CH₂-CO-Orn-Pro-cha-Trp-Phe-NH₂ |
| **71** | Ac-Ebw-Orn-Pro-cha-Trp-Phe-NH₂ |
| **72** | Ac-Phe-Orn-Pro-cha-Trp-NH-CH₂-CH₂-Ph |
| **73** | Ac-Phe-Orn-Aze-cha-Bta-NH-CH₂-CH₂-Ph |
| **74** | H-Phe-Orn-Pro-cha-Trp-Phe-NH₂ |
| **75** | H-Me-Phe-Orn-Pro-cha-Trp-Phe-NH₂ |
| **76** | Bu-NH-CO-Phe-Orn-Pro-cha-Trp-Phe-NH₂ |
| **77** | Ac-Thi-Orn-Pro-cha-Trp-Phe-NH₂ |
| **78** | Ac-Ebw-Orn-Pro-cha-Trp-Phe-NH₂ |
| **79** | Ac-Phe-Orn-Ala-cha-Trp-Phe-NH₂ |
| **80** | Ac-Phe-Orn-Pro-cha-Trp-Thi-NH₂ |
| **81** | Ac-Phe-Orn-Aze-cha-Pcf Phe-NH₂ |
| **82** | Ac-Phe-Orn(Ac)-Pro-cha-Trp-Phe-NH₂ |
| **83** | Ac-Phe-Orn-Aze-cha-Trp-Phe-NH₂ |
| **84** | Ac-Phe-Trp-Pro-cha-Trp-Phe-NH₂ |
| **85** | Ph-NH-CO-Phe-Orn-Pro-cha-Trp-Phe-NH₂ |
| **86** | Bu-O-CO-Phe-Orn-Pro-cha-Trp-Phe-NH₂ |
| **87** | Ac-Phe-Lys-Pro-cha-Trp-Phe-NH₂ |
| **88** | Ac-Phe-Arg-Pro-cha-Trp-Phe-NH₂ |
| **89** | Ac-Phe-Gln-Pro-cha-Trp-Phe-NH₂ |
| **92** | Ac-Phe-Orn-Pip-cha-Trp-Phe-NH₂ |
| **93** | Ac-Phe-Orn-Hyp-cha-Trp-Phe-NH₂ |
| **94** | Ac-Phe-Orn-Pro-cha-Trp-1Ni-NH₂ |
| **95** | Ac-Phe-Orn-Aze-cha-Bta-Phe-NH-Me |
| **96** | CH₃-SO₂-Phe-Orn-Aze-cha-Bta-Phe-NH-Me |
| **99** | Ac-Phe-Orn-Aze-cha-Pff Phe-NH₂ |
| **100** | Ac-Phe-Orn-Aze-cha-Mcf Phe-NH₂ |
| **101** | Ac-Phe-Orn(Ac)-Aze-cha-Bta-Phe-NH₂ |
| **102** | Ac-Ebw-Orn-Pro-cha-Trp-Phe-NH₂ |
| **103** | Ac-Phe-Trp-Pro-cha-Trp-Phe-NH₂ |
| **104** | Ac-Phe-Arg-Pro-cha-Trp-Phe-NH₂ |
| **105** | Ac-Phe-Orn-Pip-cha-Trp-Phe-NH₂ |
| **106** | 3PP-Orn-Aze-cha-Bta-Phe-NH₂ |
| **107** | Ac-Phe-Orn-Tic-cha-Trp-Phe-NH₂ |
| **108** | Ac-Phe-Orn-Ser-cha-Trp-Phe-NH₂ |
| **109** | Ac-Phe-Orn-Pro-chg-Trp-Phe-NH₂ |
| **110** | Ac-Phe-Orn-Pro-hch-Trp-Phe-NH₂ |
| **111** | Ac-Phe-Orn-Pro-cha-Trp-Phg-NH₂ |
| **112** | Ac-Phe-Bta-Aze-cha-Bta-Phe-NH₂ |
| **113** | Ac-Phe-Trp-Pro-cha-Bta-Phe-NH₂ |
| **115** | Ac-Phe-Orn-Pip-cha-Trp-Phe-OH |
| **116** | Ac-Phe-Orn-Tic-cha-Trp-Phe-OH |
| **117** | Ac-Phe-Orn-Ser-cha-Trp-Phe-OH |
| **118** | Ac-Phe-Orn-Pro-chg-Trp-Phe-OH |
| **119** | Ac-Phe-Eec-Pro-cha-Bta-Phe-NH₂ |
| **120** | Ac-Phe-Nle-Pro-cha-Bta-Phe-NH₂ |
| **121** | Ac-Phe-Har-Pro-cha-Bta-Phe-NH₂ |
| **122** | Ac-Phe-Arg-Pro-cha-Bta-Phe-NH₂ |
| **123** | Ac-Phe-Cys(Acm)-Pro-cha-Bta-Phe-NH₂ |
| **124** | Ac-Phe-Mpa-Pro-cha-Bta-Phe-NH₂ |
| **125** | Ac-Eby-Orn-Pro-cha-Bta-Phe-NH₂ |
| **126** | Ac-Phg-Orn-Pro-cha-Bta-Phe-NH₂ |
| **127** | Ac-Phe-Paf-Pro-cha-Bta-Phe-NH₂ |
| **128** | H₂N-CO-Phe-Orn-Pro-cha-Bta-Phe-NH₂ |
| **129** | Me-O-CO-Phe-Orn-Pro-cha-Bta-Phe-NH₂ |
| **130** | (-CO-CH₂-NH-CO-)-Phe-Orn-Pro-cha-Bta-Phe-NH₂ |
| **132** | Ac-Phe-Orn-Pro-hch-Trp-Phe-OH |
| **133** | (-CO-CH₂-CH₂-CO-)-Phe-Orn-Pro-cha-Bta-Phe-NH₂ |
| **134** | ^{t}Bu-CO-Phe-Orn-Pro-cha-Bta-Phe-NH₂ |
| **135** | Ac-Lys-Phe-Orn-Aze-cha-Bta-Phe-NH₂ |
| **136** | Ac-Gly-Phe-Orn-Aze-cha-Bta-Phe-NH₂ |
| **137** | Ac-Arg-Phe-Orn-Aze-cha-Bta-Phe-NH₂ |
| **138** | Ac-His-Phe-Orn-Aze-cha-Bta-Phe-NH₂ |
| **139** | Ac-Ser-Phe-Orn-Aze-cha-Bta-Phe-NH₂ |
| **140** | Ac-Guf-Phe-Orn-Aze-cha-Bta-Phe-NH₂ |
| **141** | Ac-Dab-Phe-Orn-Aze-cha-Bta-Phe-NH₂ |
| **142** | FH₂C-CO-Phe-Orn-Pro-cha-Bta-Phe-NH₂ |
| **143** | Ac-Phe-Om(Et₂)-Pro-cha-Trp-Phe-NH₂ |
| **149** | Ac-Phe-Orn-Pro-hle-Bta-Phe-NH₂ |
| **150** | Ac-Phe-Arg(CH₂-CH₂)-Pro-cha-Bta-Phe-NH₂ |

Die aus dem Stand der Technik, beispielsweise Finch et al. 1999 Journal of Medicinical Chemistry 42: 1965-1974; Wong et al. 1999 IDrugs 2: 686-693, US 4,692,511, US 5,663,148, WO 90/09162, WO 92/11858, WO 92/12168, WO 92/21361, WO 94/07518, WO 94/07815, WO 95/25957, WO 96/06629, WO 99/00406 und WO 99/13899, bekannten linearen Peptide sind in der Regel deutlich schlechtere Antagonisten von C5a als zyklische Peptide, wie die in WO 99/00406 beschriebenen (z.B. Ac-Phe-[Lys-Pro-cha-Trp-arg], Ac-Phe-[Orn-Pro-cha-Trparg], Ac-Phe-[Orn-Pro-cha-Trp-Arg], Ac-Phe-[Lys-Pro-cha-Trp-Arg]). Das hinsichtlich seiner antagonistischen Eigenschaften aktivste in WO 99/00406 beschriebene lineare Peptid weist die Sequenz Me-Phe-Lys-Pro-cha-Trp-arg auf und zeigt einen IC₅₀-Wert von 0,085 µM (gemessen mit dem zellulären Myeloperoxidase-Freisetzungs Assay mit humanen PMNs). Dagegen zeigt das vergleichbare zyklische Peptid Ac-Phe-[Lys-Pro-cha-Trp-arg] (ebenfalls aus WO 99/00406) einen IC₅₀-Wert von 0,012 µM. In WO 99/00406 wird ausgeführt, dass eine geringere strukturelle Flexibilität des zyklischen Peptids zu einer Verringerung, d. h. Verbesserung, des IC₅₀-Wertes führt. Dies spiegelt sich letztendlich in der Entwicklung von zyklischen - also am wenigsten flexiblen - Inhibitoren wie Ac-Phe-[Lys-Pro-cha-Trp-arg] und Ac-Phe-[Orn-Pro-cha-Trp-Arg] wider. Hinsichtlich zumindest eines Aspektes der vorliegenden Erfindung haben sich die Erfinder somit bewusst von der im Stand der Technik vorherrschenden Auffassung gelöst und damit eine neue Klasse von Verbindungen, die als C5aR-Antagonisten verwendet werden können, bereitgestellt.

Die vorliegende Erfindung beschreibt somit erstmals peptidische bzw. peptidomimetische C5aR-Antagonisten mit inhibitorischen Aktivitäten mit einem IC₅₀ < 200 nM, die unter physiologischen pH-Werten (pH 7.4) keine positive Nettoladung tragen und/oder deren C-terminale Aminosäure keine positive Ladung trägt. Der IC-Wert wird dabei mit einem funktionellen Test bestimmt (Köhl 1997 The Anaphylatoxins. In: Dodds, A.W., Sim, R.B. (Eds.), Complement: A Practical Approach. Oxford, pp. 135-163). Die erfindungsgemäßen Verbindungen können somit als C5aR-Antagonisten, insbesondere auch unter physiologischen Bedingungen verwendet werden.

Durch die erfindungsgemäßen Verbindungen wird deutlich, dass eine geeignete hydrophobe Substitution sowohl von aliphatischer als auch von aromatischer oder heteroaromatischer Natur das C-terminale Arginin in C5aR bindenden Peptiden ersetzen kann.

Ein weiteres Kennzeichen der erfindungsgemäßen Verbindungen, insbesondere der erfindungsgemäßen Peptide und Peptidomimetika, ist das Fehlen einer agonistischen Aktivität in einem zellulären Assay bis zu einer Konzentration von mindestens 1430 nM. Beispiel 12 zeigt beispielhaft die Ergebnisse von Messungen einer Auswahl der erfindungsgemäßen Peptide mittels eines Verfahrens, mit dem der Agonismus bzgl. des C5aR bestimmt wird. Es ist offensichtlich, dass die erfindungsgemäßen Verbindungen bis zu der maximal eingesetzten Konzentration keine agonistische Aktivität zeigen. Im Rahmen der vorliegenden Erfindung stellen die nachfolgenden erfindungsgemäßen Verbindungen Beispiele für erfindungsgemäße Peptide dar, die reine Antagonisten sind: HOCH₂(CHOH)₄-C=N-O-CH₂-CO-Phe-[Orn-Procha-Trp-Nle], Ph-CH₂-CH₂-CO-[Orn-Pro-cha-Trp-Nle], Ac-Phe-[Orn-Hyp-cha-Trp-Phe], H-Phe-[Orn-Pro-cha-Trp-Phe], Ac-Phe-[Orn-Pro-cha-Trp-Phe], Ac-Lys-Phe-[Orn-Pro-cha-Trp-Nle], H-Phe-[Orn-Pro-cha-Trp-Nle], H-Phe-[Orn-Ser-cha-Trp-Nle], Ac-Phe-[Om-Pro-cha-Trp-Eaf], Ac-Phe-Orn-Pro-cha-Trp-Phe-NH₂, Ac-Phe-Orn-Pro-cha-Bta-Phe-NH₂, Ac-Ebw-Om-Pro-cha-Trp-Phe-NH₂, Ac-Phe-Orn-cha-cha-Bta-Phe-NH₂, Ac-Phe-Arg-Pro-cha-Trp-Phe-NH₂, Ac-Phe-Orn-Pip-cha-Trp-Phe-NH₂, Ac-Phe-Orn-Aze-cha-Trp-Phe-NH₂, Ac-Phe-Trp-Pro-cha-Trp-Phe-NH₂, Ac-Thi-Orn-Pip-cha-Bta-Phe-NH₂, Ac-Phe-Orn-Pro-hle-Bta-Phe-NH₂, Ac-Phe-Arg(CH₂-CH₂)-Pro-cha-Bta-Phe-NH₂.

Zur genaueren Analyse des C5aR-Antagonismus und der Entwicklung eines Pharmakophor-Modells der Verbindung Ac-Phe-[Orn-Pro-cha-Trp-Arg] wurden die Aminosäuren Phe, Trp und Arg dieses Peptids durch L-Alanin ausgetauscht, Pro durch NMe-Ala und die Aminosäure cha durch D-Alanin (Einzelsubstitutionen). Diese Peptide wurden anschließend in einem funktionellen Assay auf ihren C5aR-Antagonismus hin untersucht (Beispiel 11). In diesem Versuch wurde deutlich, dass die Substitutionen der Aminosäureseitenketten von Trp, cha und Phe durch Methylgruppen zu einem deutlichen Aktivitätsverlust führen (IC_{5O}-Werte >30 µM. Dagegen ist die Aktivität des Antagonisten Ac-Phe-[Orn-Pro-cha-Trp-Arg] vergleichbar mit der Aktivität des am Pro mit NMeAla substituierten Moleküls (IC₅₀ = 20 nM gegenüber 25 nM). Die Substitution von Arg mit Ala führt ebenfalls zu einem deutlichen Aktivitätsverlust (IC₅₀-Wert von 16 nM auf 5,6 µM), der jedoch geringer ausfällt als bei der Substitution von Trp und Phe.

Weitere Substitutionen am Peptid Ac-Phe-[Orn-Pro-cha-Trp-Arg) und ähnlicher Verbindungen an der Position des Arg führten zu einer Reihe von Peptiden bzw. peptidomimetischen Verbindungen, die überraschenderweise eine nennenswerte inhibitorische Aktivität aufweisen (Beispiel 11). Insbesondere folgende Peptide sind nennenswert inhibitorisch aktiv: Ac-Phe-[Orn-Pro-cha-Trp-Phe], Ac-Phe-[Om-Hyp-cha-Trp-Phe], Ac-Phe-[Orn-Pro-cha-Trp-Paf], Ac-Phe-[Orn-Pro-cha-Trp-Ecr], Ac-Phe-[Orn-Pro-cha-Trp-Ppa], Ac-Phe-[Orn-Pro-cha-Trp-Nle], Ac-Phe-[Orn-Pro-cha-Trp-Met], Ac-Phe-[Orn-Pro-cha-Trp-Nva], Ac-Phe-[Orn-Pro-cha-Trp-Hle], Ac-Phe-[Orn-Pro-cha-Trp-Eaf], Ac-Phe-[Orn-Pro-cha-Trp-Ebd], Ac-Phe-[Om-Pro-cha-Trp-Eag], Ac-Phe-[Orn-Pro-cha-Trp-Pmf], Ac-Phe-[Orn-Pro-cha-Trp-2Ni], Ac-Phe-[Om-Procha-Trp-Thi], Phe-[Orn-Pro-cha-Trp-Nle], Ac-Lys-Phe-[Orn-Pro-cha-Trp-Nle], Ac-Phe-[Om-Ser-cha-Trp-Phe], HOCH₂(CHOH)₄-C=N-O-CH₂-CO-Phe-[Orn-Pro-cha-Trp-Nle], Ac-Phe-[Orn-Hyp(COCH₂OCH₂CH₂OCH₂CH₂OCH₃)-cha-Trp-Phe], Ac-Phe-[Om-Hyp(CONHCH₂COH(OH)CH₂OH)-cha-Trp-Phe], Phenylpropionyl-[Orn-Pro-cha-Trp-Nle] , Ac-Phe-Om-Pro-hle-Bta-Phe-NH₂, Ac-Phe-Arg(CH₂-CH₂)-Pro-cha-Bta-Phe-NH₂,

Die orale Aufnahme von Peptiden wird durch verschiedene Faktoren wie Größe, Ladung und Hydrophobizität beeinflusst. Dennoch lässt sich die orale Verfügbarkeit eines Peptids nicht a priori vorhersagen. In der Regel gelten Peptide als schlecht oral verfügbar (Burton et al. 1996 Journal of Pharmaceutical Sciences 85: 1337-1340). Ein Modell zur Abschätzung der oralen Absorption stellt die Messung der AB-Permeabilität durch eine Monolayer-Schicht von Darmepithelzellen (z.B. CaCo2 oder TC-7) dar (Beispiel 15, Lennernäs 1997 Journal of Pharmacy and Pharmacology 49: 627-38). Die erfindungsgemäßen Verbindungen, die als C5aR Antagonisten verwendet werden können, weisen durch die hydrophobe Substitution des C-terminalen Arginins eine deutlich verbesserte AB-Permeabilität auf. Beispielsweise zeigt der Antagonist Ac-Phe-[Orn-Hyp-cha-Trp-Phe] eine überraschend gute Permeabilität von 14.3×10⁻⁶ cm/s gegenüber der schlechten Permeabilität von 0.52x10⁻⁶ cm/s des geladenen Antagonisten Ac-Phe-[Orn-Pro-cha-Trp-Arg]. Die hohe Permeabilität liegt zahlenmäßig in einem Bereich, der nahe an den für oral gut verfügbare Substanzen heranreicht. Ein Beispiel für eine oral sehr gut verfügbare Verbindung ist Propanolol, das in diesem Test von Lennernäs eine AB-Permeabilität von 31.1 10⁻⁶ cm/s zeigt.

Es ist ebenfalls im Rahmen der vorliegenden Erfindung, dass die erfindungsgemäßen Verbindungen solche sind, die so ausgestaltet sind, dass bei ihnen an X1 und/oder X4 zusätzlich die Wasserlöslichkeit verbessernde Gruppen eingefugt sind. Besonders günstig zur Verbesserung der Wasserlöslichkeit ist die Einführung von Gruppen, die starke Wechselwirkungen mit Wasser eingehen und stark solvatisiert werden. Häufig verwendete Beispiele sind: Hydroxy, Keto, Carboxamido, Ether, Harnstoff, Carbamat, Amino, substituiertes Amino, Guanidino, Pyridyl, Carboxyl. Die beschriebenen Gruppen können ausdrücklich an allen Positionen von X1 und/oder X4 eingeführt werden und es können sowohl eine als auch mehrere der die Wasserlöslichkeit verbessernden Gruppen eingefügt werden. Beispiele für die Einführung mehrerer Gruppen sind die Anknüpfung von Kohlenhydratresten oder Ethylenglykolen.

Die vorliegende Erfindung umfasst daher insbesondere auch peptische bzw. peptidomimetische C5aR Antagonisten, insbesondere gemäß der vorliegenden Erfindung, deren Löslichkeit durch zusätzliche Modifikationen verbessert wurde. Derartige Modifikationen sind dem Fachmann bekannt und umfassen beispielsweise die Einführung der vorstehend genannten, die Wasserlöslichkeit verbessernden Gruppen. Dass dies eine wirksame Maßnahme ist bzw. zu hochwirksamen Antagonisten führt, sei an den folgenden Beispielen demonstriert.

Verbindung **1** löst sich gemäß Beispiel 13 zu 8% in wässerigem HEPES-Puffer (pH 7.4). Dagegen ist die Verbindung **40** zu 94% in HEPES-Puffer löslich. Die Verbindung **2**, die im Vergleich zu Verbindung **1** eine zusätzliche OH-Gruppe trägt, ist zu 13 % löslich. Durch Anfügen komplexerer hydrophiler Gruppen, wie für Verbindung **4** gezeigt, wird die Löslichkeit von 22 % (Verbindung **28**) auf 84 % (Verbindung **4**) gesteigert. Dies ist der Fall obwohl Verbindung **4** nicht geladen ist. Damit ist gewährleistet, dass die erfindungsgemäßen Peptide und Peptidomimetika trotz ihres hydrophoben Charakters in eine gut wasserlösliche Form umgewandelt werden können.

Nachfolgend werden einige Begrifflichkeiten angegeben, deren Bedeutung für Ausführungsformen der vorliegenden Erfindung, insbesondere jene, wie sie detaillierter hierin angegeben sind, herangezogen werden sollen. Obwohl diese Begrifflichkeiten gelegentlich als Definitionen bezeichnet werden, ist der Begriffsinhalt der verschiedenen Begriffe nicht notwendigerweise darauf beschränkt.

Der Begriff "enthält" bedeutet in bevorzugten Ausführungsformen, daß das jeweilige Strukturelement enthalten ist, aber die Struktur nicht darauf beschränkt ist.

Der Begriff "substituiert" bedeutet in bevorzugten Ausführungsformen, dass ein oder mehrere Wasserstoffatome einer Gruppe oder Verbindung die substituiert ist, durch ein anderes Atom, eine Gruppe von Atomen, ein Molekül oder eine Molekülgruppe ersetzt ist. Ein solches Atom, Gruppe von Atomen, Moleküle, sowie Molekülgruppen wird/werden hierbei selbst als Substituenten oder Substitutionen bezeichnet. Bei der Substitution ist vorrausgesetzt, daß die normale Valenz des jeweiligen Atomes nicht überschritten wird und daß die Substitution in einer stabilen Verbindung resultiert. Durch die Substitution zweier Wasserstoff-Atome kann eine Carbonyl-Gruppe (C=O) entstehen. Carbonyl-Substituenten sind bevorzugterweise nicht innerhalb von aromatischen Einheiten enthalten.

Substituenten oder Substitutionen können bevorzugt einzeln oder in beliebiger Kombination aus der Gruppe ausgewählt sein die Hydroxyl, Alkoxyl, Mercapto, Alkyl, Alkenyl, Alkynyl, Alkoxy, Alkylthio, Alkylsulfinyl, Cycloalkyl, Heterocyclyl, Aryl, Arylalkyl, Arylalkoxy, Heteroaryl, Aryloxy, Halogen, Trifluormethyl, Difluormethyl, Cyano, Nitro, Azido, Amino, Aminoalkyl, Carboxamido, -C(O)H, Acyl, Oxyacyl, Carboxyl, Carbamat, Sulfonyl, Sulfonamid und Sulfuryl umfasst. Jeder Substituent kann wiederum selbst durch einen oder mehrere weitere Substituenten substituiert sein. Dies gilt speziell für Alkyl, Cycloalkyl, Heterocyclyl, Aryl, Heteroaryl und Aryloxy. Weiterhin gelten jegliche hierin angegebenen Definitionen auch für Substituenten.

Unter dem Begriff "Alkyl" versteht man in einer Ausführungsform der vorliegenden Erfindung ein gesättigtes aliphatisches Radikal bestehend aus 1-10 Kohlenstoffatomen oder ein einfach oder mehrfach ungesättigtes aliphatisches Kohlenwasserstoffradikal, welches zwischen 2 und 12 Kohlenstoffatome sowie mindestens eine Doppel- oder Dreifachbindung enthält. Der Begriff "Alkyl" schließt sowohl geradkettige als auch verzweigte Alkylreste ein. Bevorzugt sind geradkettige Alkylreste mit 1 bis 8 Kohlenstoffatomen. Besonders bevorzugt sind geradkettige Alkylreste mit 1 bis 6 Kohlenstoffatomen sowie verzweigte Alkylreste mit 3 bis 6 Kohlenstoffatomen. Weiterhin beinhaltet der Begriff "Alkyl" jegliche Analoga die sich aus Kombinationsbezeichnungen der Vorsilben "Alk" oder "Alkyl" zusammensetzen lassen.

Beispielsweise beschreibt der Begriff "Alkoxy" oder "Alkylthio" eine Alkylgruppe die über einen Sauerstoff bzw. einen Schwefel gebunden ist. Unter "Alkanoyl" versteht man eine Alkylgruppe, die über eine Carbonylgruppe (C=O) gebunden ist.

Unter dem Begriff "Cycloalkyl" versteht man in einer Ausführungsform der vorliegenden Erfindung zyklische Derivate einer Alkylgruppe nach obiger Definition, optional ungesättigt und/oder substituiert. Bevorzugt sind gesättigte Cycloalkylgruppen, insbesondere Cycloalkylgruppen mit 3 bis 8 Kohlenstoffatomen. Besonders bevorzugt sind Cycloalkylgruppen, die 3 bis 6 Kohlenstoffatome enthalten.

Der Begriff "Aryl" bezeichnet in einer Ausführungsform der vorliegenden Erfindung einen aromatischen Rest mit 6 bis 14 Kohlenstoffatomen wobei "substituiertes Aryl" für Arylreste steht, die einen oder mehrere Substituenten tragen.

Jede der obig definierten Gruppen "Alkyl", "Cycloalkyl", und "Aryl" schließt die jeweiligen halogenierten Derivate mit ein, die ein oder mehrere Halogenatome tragen können. Die halogenierten Derivate beinhalten jegliches Halogenradikal nach der folgenden Definition.

"Halogen" bezeichnet in einer Ausführungsform der vorliegenden Erfindung ein Halogenradikal aus der Gruppe Fluor, Chlor, Brom und Jod. Bevorzugt hierbei sind Fluor, Chlor und Brom.

Der Begriff "Heteroaryl" bezeichnet in einer Ausführungsform der vorliegenden Erfindung ein 5- bis 8-gliedriges, bevorzugt 5- bis 6-gliedriges, monozyklisches oder 8- bis 11-gliedriges bizyklisches, aromatisches, heterozyklisches Radikal, wobei jeder Heterozyklus sowohl aus Kohlenstoffatomen, sowie 1-4 Heteroatomen aus der Reihe N, O oder S bestehen kann. Der Heterozyklus kann durch jedes Atom des Zyclus' verbunden sein, so dass eine stabile Struktur resultiert. Bevorzugte Heteroarylradikale im Rahmen dieser Erfindung sind beispielsweise Furyl, Thienyl, Pyrrolyl, Oxazolyl, Thiazolyl, Imidazolyl, Pyrazolyl, Isoxazolyl, Oxadiazolyl, Triazolyl, Tetrazolyl, Thiadiazolyl, Pyridinyl, Pyridazinyl, Pyrimidinyl, Pyrazinyl, Indolizinyl, Indolyl, Isoindolyl, Benzofuranyl, Benzothienyl, Indazolyl, Benzimidazolyl, Benzthiazolyl, Benzoxazolyl, Purinyl, Quinolizinyl, Quinolinyl, Isoquinolinyl, Cinnolinyl, Phthalazinyl, Quinazolinyl, Quinoxalinyl, Naphthridinyl, Pteridinyl, Carbazolyl, Acridinyl, Phenazinyl, Phenothiazinyl und Phenoxazinyl.

Unter dem Begriff "Heterocyclyl" versteht man in einer Ausführungsform der vorliegenden Erfindung ein 5- bis 8-gliedriges, bevorzugt 5- bis 6-gliedriges, monozyklisches oder 8- bis 11-gliedriges bizyklisches, heterozyklisches Radikal, welches entweder gesättigt oder ungesättigt, jedoch nicht aromatisch ist. Jeder Heterocyclus besteht sowohl aus Kohlenstoffatomen, sowie 1-4 Heteroatomen aus der Reihe N, O oder S. Der Heterocyclus kann durch jedes Atom des Cyclus verbunden sein, so dass eine stabile Struktur resultiert. Bevorzugte Heteroarylradikale im Rahmen dieser Erfindung sind beispielsweise Pyrrolinyl, Pyrrolidinyl, Pyrazolinyl, Pyrazolidinyl, Piperidinyl, Morpholinyl, Thiomorpholinyl, Pyranyl, Thiopyranyl, Piperazinyl, Indolinyl, Azetidinyl, Tetrahydropyranyl, Tetrahydrothiopyranyl, Tetrahydrofuranyl, Hexahydropyrimidinyl, Hexahydropyridazinyl, 1,4,5,6-Tetrahydropyrimidin-2-ylamin, Dihydro-oxazolyl, 1,2-thiazinyl-1,1-dioxid, 1,2,6-Thiadiazinanyl-1,1-dioxid, Isothiazolidinyl-1,1-dioxid und Imidazolidinyl-2,4-dion.

Werden die Begriffe "Heterocyclyl", "Heteroaryl" und "Aryl" zusammen mit anderen Ausdrücken oder Begriffen benutzt gelten weiterhin obige Definitionen. Zum Beispiel beschreibt der Begriff "Aroyl" einen Phenyl- oder Naphthylrest, der an eine Carbonylgruppe (C=O) gebunden ist.

Jede Aryl- oder Heteroarylverbindung beinhaltet außerdem die teilweise oder vollständig hydrierten Derivate. Zum Beispiel kann Quinolyl auch Decahydroquinolinyl und Tetrahydroquinolinyl einschließen. Naphthyl kann auch die hydrierten Derivate wie beispielsweise Tetrahydronaphthyl mit einschließen.

Im Rahmen dieser Erfindung sind mit den Ausdrücken "Stickstoff" oder "N" und "Schwefel" oder "S" auch jegliche oxidierten Derivate des Stickstoffs wie Nitrone, N-Oxide oder des Schwefels wie Sulfoxide, Sulfone und quartemierte Formen basischen Stickstoffs wie HCloder TFA-Salze mit eingeschlossen.

Radikale können sowohl Mono-, als auch Di-, Tri- oder Tetraradikale sein. Dadurch ist es möglich, daß sich auch die Bedeutung verschiedener Begriffe leicht ändert. So bedeutet ein Diradikal, das als "Propyl" beschrieben wird, automatisch "Propyplen" (z.B. -(CH₂)₃).

Beschreibungen, die die Grenzen eines Bereichs wie beispielsweise "1 bis 5" spezifizieren, bedeuten jede ganze Zahl von 1 bis 5. Im einzelnen 1, 2, 3, 4 und 5. Mit anderen Worten beinhaltet jeder Bereich, der durch zwei ganze Zahlen beschrieben ist, sowohl die beiden ganzen Zahlen der Definitionsgrenzen, als auch alle ganzen Zahlen innerhalb dieses Bereichs.

Die vorliegende Erfindung beinhaltet alle Isotope von Atomen in den beschriebenen Verbindungen. Isotope sind Atome, die die gleiche Ordnungszahl aber verschiedene Massenzahl haben. Beispielsweise sind das Tritium und das Deuterium Isotope von Wasserstoff, Beispiele für Kohlenstoff-Isotope sind ¹¹C, ¹³C und ¹⁴C.

Mit dem Begriff "energetisch zugängliches Konformer" ist jegliches Konformer einer Verbindung gemeint, das innerhalb eines 20 kcal/mol-Fensters oberhalb der Konformation mit der niedrigsten Energie fällt. Hier kann z.B. eine Monte Carlo oder systematische Konformationssuche mittels MM2-, MM3- oder MMFF-Kraftfeldern, die in Molecular-Modeling-Programmen wie MacroModel® v 7.0, Schrödinger Inc. Portland, Oregon, USA (http://www.schrodinger.com) implementiert sind, verwendet werden.

Aminosäuren sind dem Fachmann bekannt und dadurch definiert, daß in einem Molekül sowohl eine Amino- als auch eine Carboxylgruppe vorhanden ist. Dabei können sowohl natürliche als auch unnatürliche Aminosäuren gemeint sein. Beispiele sind α-, β-, und - Aminosäuren, wobei bevorzugt α-Aminosäuren, besonders bevorzugt α-L-Aminosäuren eingesetzt werden können. Wenn eine Aminosäure nicht genauer spezifiziert ist (z.B. "Tryptophan"), dann ist sowohl die L- als auch die D-Form gemeint.

Eine natürliche Aminosäure ist eine L-Aminosäure ausgewählt aus der Gruppe Glycin, Leucin, Isoleucin, Valin, Alanin, Phenylalanin, Tyrosin, Tryptophan, Asparaginsäure, Asparagin, Glutaminsäure, Glutamin, Cystein, Methionin, Arginin, Lysin, Prolin, Serin, Threonin und Histidin.

Eine unnatürliche Aminosäure ist eine nicht-proteinogene Aminosäure, die beinhaltet, aber nicht beschränkt ist auf D-Aminosäuren, N-Alkyl-Aminosäuren, Homo-Aminosäuren, α,α-Disubstituierte Aminosäuren, Dehydro-Aminosäuren.

Aminosäure-Derivate sind Verbindungen, die aus Aminosäuren dadurch entstehen, daß diese am N- und/oder C-Terminus modifiziert werden. Nicht limitierende Beispiele sind Umsetzungen der Carboxylgruppe zu Salzen, Estern, Acylhydraziden, Hydroxamsäuren oder Amiden und der Amino-Gruppe zu Amiden, Harnstoffen, Thioharnstoffen, Thioamiden, Sulfonamiden, Phosphorsäureamiden, Borsäureamiden oder Alkylaminen. Teile von Verbindungen, die durch Modifizierungen von Aminosäuren am C- und/oder N-Terminus entstehen, können auch als Aminosäure-Einheiten bezeichnet werden.

Aminosäure-Analoga sind Verbindungen, die aus Aminosäuren dadurch entstehen, daß die Amino- und/oder Carboxylgruppe durch andere Gruppen, die diese mimiken können, ersetzt werden. Nicht limitierende Beispiele sind der Einbau von Thioamiden, Harnstoffen, Thioharnstoffen, Acylhydraziden, Estern, Alkylaminen, Sulfonamiden, Phosphorsäureamiden, Ketonen, Alkoholen, Boronsäureamiden, Benzodiazepinen und anderen aromatischen oder nichtaromatischen Heterocyclen (für eine Übersicht siehe M. A. Estiarte, D. H. Rich in Burgers Medicinal Chemistry, 6th Edition, Volume 1, Part 4, John Wiley & Sons, New York, 2002).

Aromatische Aminosäuren sind Aminosäuren, die Aryl- oder Heteroaryl-Gruppen enthalten. Nicht limitierende Beispiele sind Phenylalanin, Tyrosin, Histidin, Tryptophan, Homo-Phenylalanin, Homo-Tyrosin, Homo-Histidin, Homo-Tryptophan, 1-Naphtylalanin, 2-Naphtylalanin, 2-Thienylalanin, 3-Thienylalanin, Benzothienylalanin, Furylalanin, Thiazolylalanin, Pyridylalanin, Tetrahydroisochinolin-2-Carbonsäure, 2-Aminoindan-2-carbonsäure, Biphenylalanin, 3,3-Diphenylalanin und entsprechende D- und β-Aminosäuren.

Hydrophobe Aminosäuren sind Aminosäuren, die hydrophobe Alkyl-, Cycloalkyl-, Heterocyclyl, Aryl- oder Heteroaryl-Gruppen enthalten. Nicht limitierende Beispiele sind Leucin, Isoleucin, Valin, Phenylalanin, Cystein, Methionin, Prolin, Tryptophan, Norleucin, Norvalin, Cyclohexylalanin, Cyclopentylalanin, 1-Naphtylalanin, 2-Naphtylalanin, 2-Thienylalanin, 3-Thienylalanin, Benzothienylalanin, Allylglycin, Propargylglycin, 2-Methyl-Phenylalanin, 3-Methyl-Phenylalanin, 4-Methyl-Phenylalanin, Homo-Cyclohexylalanin, Cyclohexylglycin, N-Cyclohexylglycin, Octahydroindol-2-carbonsäure und entsprechende D-und β-Aminosäuren.

Die biologischen Bindungseigenschaften einer Aminosäureeinheit sind dabei jene Bindungseigenschaften, die die jeweilige Aminosäure bei der Wechselwirkung mit einem biologischen Molekül aufweist. Dabei sind biologische Moleküle insbesondere solche, die eine biologische Funktion ausüben. Beispiele für derartige biologische Moleküle, aber nicht darauf beschränkt, sind beispielsweise Protein- oder Peptid-basierte Rezeptoren.

Gruppen oder Einheiten, die die biologischen Bindungseigenschaften einer Aminosäure mimiken oder nachahmen, sind definiert als Gruppen, die mindestens eine gleiche oder ähnliche Wechselwirkung mit einem Rezeptor oder Wechselwirkungspartner, bevorzugterweise einem biologischen Rezeptor oder biologischen Wechselwirkungspartner eingehen können wie die Aminosäure selbst. Bei der Auswahl solcher Gruppen ist es bevorzugt, die verbreitetsten im Sinne von bevorzugtesten Wechselwirkungen der infragestehenden Aminosäure mit biologischen Rezeptoren zu betrachten. So ist das Sauerstoffatom der Carbonylgruppe einer Aminosäure befähigt, als Wasserstoffbrücken-Akzeptor zu fungieren, wohingegen das NH-Proton Wechselwirkungen als Wasserstoffbrücken-Donor eingehen kann. Zusätzlich können Aminosäuren Wechselwirkungen mit Rezeptoren über Ihre Seitenketten eingehen. Phenylalanin und Tryptophan können sowohl hydrophobe Wechselwirkungen über die Seitenketten-Methylengruppe oder die aromatischen Gruppen als auch π-π-Wechselwirkungen über die aromatischen Gruppen eingehen. Zusätzlich kann die Indolgruppe des Tryptophans über die NH-Gruppe als Wasserstoffbrücken-Donor fungieren. Cyclohexylalanin und Norleucin können grundsätzlich hydrophobe Wechselwirkungen mit biologischen Rezeptoren über ihre Alkyl- bzw. Cycloalkyl-Seitenketten eingehen. Bei allen Aminosäuren können nicht nur die gesamte Seitenkette, sondern auch Teile davon grundsätzlich wichtige Wechselwirkungen eingehen.

Wenn eine Gruppe oder eine Einheit, die die biologische Bindungseigenschaft einer Aminosäure mimiken oder nachahmen soll bzw. diese Eigenschaft aufweisen soll, mindestens eine der vorstehenden Wechselwirkungen der jeweiligen Aminosäure eingehen kann, so kann sie deren biologische Bindungseigenschaften mimiken.

Bei den hierin angegebenen Gruppendefinitionen bezeichnet der Begriff "und jeweilige Derivate davon", dass alle Derivate der in der Gruppe angeführten Einzelverbindungen, Gruppen von Verbindungen, Molekülteile, Radikale oder chemische Gruppen jeweils als Derivate vorliegen können.

Die Begrifflichkeit "einzeIn und unabhängig voneinander" bezeichnet hierin, dass die zwei oder mehr angeführten Substituenten so ausgebildet werden können, wie in der entsprechenden Passage beschrieben. Die Formulierung "einzeIn und unabhängig voneinander" soll dabei lediglich unnötige Wiederholungen vermeiden und offenbart, dass ein jeglicher der angesprochenen Substituenten die beschriebene Ausgestaltung aufweisen kann, wobei die Ausgestaltung für jeden einzelnen Substituenten für sich erfolgt oder vorliegt und nicht durch die Auswahl einer oder mehrerer der anderen Substituenten beeinflusst wird.

Es ist allgemein im Rahmen der vorliegenden Erfindung, dass die für die einzelnen erfindungsgemäßen Verbindungen, insbesondere die generischen Strukturen, angegebenen Substituenten für alle generische Formeln mit den entsprechenden Substituenten gelten, sofern nichts gegenteiliges ausgeführt wird.

Spacer wie sie hierin verwendet werden, sind in bevorzugten Ausführungsformen, sofern im Einzelfall nicht anders angegeben, organische Radikale mit einer Masse von etwa 1-300, welche eine kovalente Verknüpfung verschiedener chemischer Gruppen ermöglichen. Beispiele sind einfache Gruppen wie 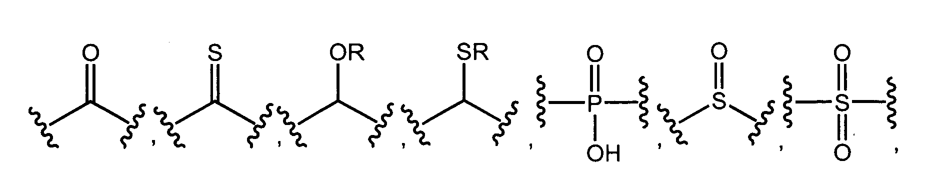 oder auch komplexere Einheiten wie

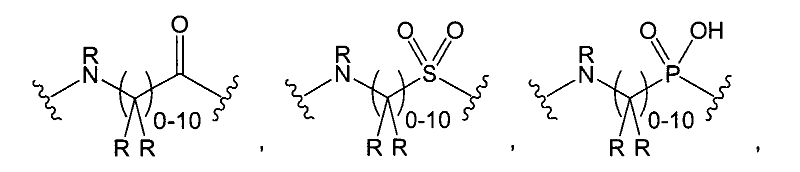

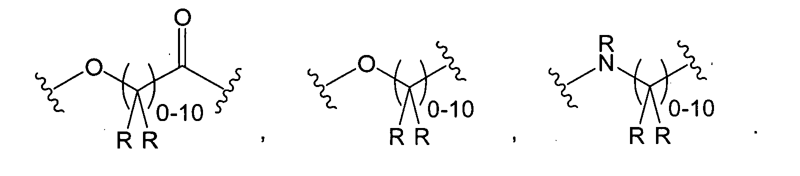

Dabei ist R, einzeln und unabhängig für eine jede Substitution, ein Rest mit einer Masse von etwa 1-300. Bevorzugterweise ist R ein Radikal, das aus der Gruppe ausgewählt ist, die H, Alkyl, substituiertes Alkyl, Cycloalkyl, substituiertes Cycloalkyl, Cycloalkylalkyl, substituiertes Cycloalkylalkyl, Heterocyclyl, substituiertes Heterocyclyl, Heterocyclylalkyl, substituiertes Heterocyclylalkyl, Aryl, substituiertes Aryl, Arylalkyl, substituiertes Arylalkyl, Heteroaryl, substituiertes Heteroaryl, Heteroarylalkyl, substituiertes Heteroarylalkyl, Acyl, substituiertes Acyl, Alkoxyalkyl, substituiertes Alkoxyalkyl, Aryloxyalkyl, substituiertes Aryloxyalkyl, Sulfhydrylalkyl, substituiertes Sulfhydrylalkyl, Hydroxyalkyl substituiertes Hydroxyalkyl, Carboxyalkyl, substituiertes Carboxyalkyl, Carboxamidoalkyl, substituiertes Carboxamidoalkyl, Carboxyhydrazinoalkyl, Ureidoalkyl Aminoalkyl, substituiertes Aminoalkyl, Guanidinoalkyl, substituiertes Guanidinoalkyl enthält.

Bevorzugterweise werden Spacer aus folgender Gruppe ausgewählt: 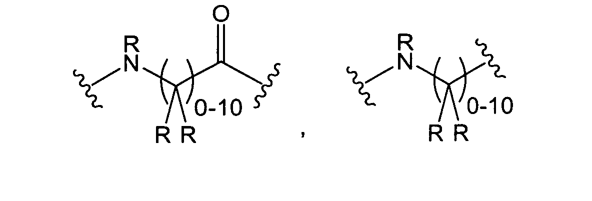 R ist bevorzugterweise ein Radikal, das aus der Gruppe ausgewählt ist, die H, Alkyl, substituiertes Alkyl, Arylalkyl, substituiertes Arylalkyl, Heteroarylalkyl, substituiertes Heteroarylalkyl enthält.

Peptide, die eine positive Nettoladung tragen, können eine Histaminfreisetzung verursachen (Jasani et al. 1979 Biochemical Journal 181: 623-632). Insbesondere eine subkutane Applikation bzw. das Setzen von subkutanen Depots ist mit derartigen Verbindungen nicht möglich. Bei oral verabreichten Medikamenten ist die Resorption der Pharmaka besonders wichtig. Geladene Moleküle werden bei sonst gleichen Randbedingungen in der Regel schlechter resorbiert als ungeladene (Veber et al. 2002 Journal of Medicinal Chemistry 45: 2615-2623). Aufgrund der fehlenden Nettoladung der erfindungsgemäßen Verbindungen eignen sich diese ganz besonders für die Verwendung als oral applizierbares Medikament. Die erfindungsgemäßen Erfindungen können für die Herstellung von Medikamenten, insbesondere für die Herstellung von Medikamenten für die Prävention und/oder Behandlung von immuno-inflammatorischen Erkrankungen herangezogen werden. Hierzu gehören insbesondere die folgenden Erkrankungen: Autoimmunkrankheiten, Rheumatoide Arthritis, systemischen Lupus erythematodes, Reperfusionsschäden, Herzinfarkt, Gehimschlag, Multiple Sklerose, Psoriasis, Dialyse, septischer Schock, Asthma, Vaskulitis, Dermatomyositis, Pemphigus, Myasthenia gravis, Verbrennungen, Organabstoßung, akute respiratorische Insuffiziens, intestinaler Reperfusionsschaden und akute Verletzungen des zentralen Nervensystems. Alle diese Erkrankungen bzw. Krankheitsbilder entstammen der Gruppe der immuno-inflammatorischen Erkrankungen, wobei die inflammatorischen Reaktionen bei diesen Erkrankungen entweder ursächlich oder als Folgereaktion auftreten.

Die vorliegende Erfindung betrifft auch Formulierungen, insbesondere pharmazeutische Formulierungen, die zumindest eine der erfindungsgemäßen Verbindungen enthalten. Häufig werden pharmazeutische Wirkstoffe mit anderen pharmazeutisch akzeptablen Bestandteilen gemischt, um eine verbesserte Wirkung wie beispielsweise verbesserten Transport, Haltbarkeit, zeitliches Verhalten bei der Freisetzung und dergleichen zu gewährleisten. Dem Fachmann ist eine Vielzahl entsprechender Formulierungen bekannt. Als Bestandteile derartiger Formulierungen sind unter anderem inerte Verdünnungsmittel, Calciumcarbonat, Natriumcarbonat, Lactose, Calciumphosphat, Natriumphosphat, Stärke, Alginate, Gelatine, Magnesiumstearat und Talk bekannt. Bestimmte Bestandteile können beigefügt werden, um eine zeitverzögerte Freisetzung der pharmazeutischen Wirkstoffe zu ermöglichen. Beispiele dafür sind Glycerolmonostearat und Glyceroldistearat. Zur oralen Applikation werden insbesondere Hartgelatinekapseln verwendet, wobei der pharmazeutisch aktive Bestandteil mit Calciumcarbonat, Calciumphosphat oder Kaolin gemischt wird. Bei Weichgelatinekapseln werden die pharmazeutisch aktiven Wirkstoffe z.B. mit Ölen gemischt (Erdnussöl, flüssiges Paraffin, Olivenöl). Für die Applikation in wässerigen Lösungen können die pharmazeutisch wirksamen Bestandteile insbesondere mit folgenden Bestandteilen gemischt werden: Carboxymethylcellulose, Methylcellulose, Hydropropylmethylcellulose, Natriumalgenat, Polyvinylpyrrolidon, Lecithin, Polymerisierungsprodukte von Alkylenoxiden und Fettsäuren wie beispielsweise Polyoxyethylenstearat, Heptadecaethylenoxycetanol, Polyoxyethylensorbitolmonooleat und Polyoxyethylensorbitanmonooleat. Zur Konservierung können verschiedene Zusatzstoffe zum Einsatz kommen. Beispiele dafür sind Ethyl- oder n-Propyl-p-hydroxybenzonat.

Bestimmte Formulierungen werden eingesetzt, um spezielle Applikationsformen zu ermöglichen. Beispiele für Applikationsformen von erfindungsgemäßen Verbindungen sind orale, subkutane, intravenöse, topische, intramuskulare, rektale und inhalative Applikationen. Die erfindungsgemäßen Verbindungen können dabei als pharmazeutisch akzeptable Salze vorliegen.

### Beispiele

### Beispiel 1: Material und Methoden

Die im Folgenden beschriebenen Materialien und Methoden sowie allgemeinen Arbeitsvorschriften wurden im Rahmen der hierin beschriebenen weiteren Beispiele durchgeführt.

### Lösungsmittel:

Alle verwendeten Lösungsmittel wurden in der angegebenen Qualität ohne weitere Reinigung eingesetzt:
Acetonitril (Gradient grade, J.T. Baker); Dichlormethan (zur Synthese, Merck Eurolab); Diethylether (zur Synthese, Merck Eurolab); *N*,*N*-Dimethylformamid (LAB, Merck Eurolab); Dioxan (zur Synthese, Aldrich); Methanol (zur Synthese, Merck Eurolab).

Wasser wurde unter Verwendung einer Vollentsalzungsanlage (Milli-Q Plus, Millipore) entmineralisiert.

### Reagenzien:

Die verwendeten Reagenzien wurden von den Firmen Advanced ChemTech (Bamberg, Deutschland), Sigma-Aldrich-Fluka (Deisenhofen, Deutschland), Bachem (Heidelberg, Deutschland), J.T. Baker (Phillipsburg, USA), Lancaster (Mühlheim/Main, Deutschland), Merck Eurolab (Darmstadt, Deutschland), Neosystem (Strassburg, Frankreich), Novabiochem (Bad Soden, Deutschland, ab 2003 Merck Biosciences, Darmstadt, Deutschland) und Acros (Geel, Belgien, Vertriebsgesellschaft Fisher Scientific GmbH, Schwerte, Deutschland), Peptech (Cambridge, MA, USA), Synthetech (Albany, OR, USA), Pharmacore (High Point, NC, USA), Anaspec (San Jose, CA, USA) bezogen und ohne weitere Aufreinigung verwendet. Nicht kommerziell erhältliche unnatürliche Aminosäuren oder Carbonsäuren zur N-terminalen Modifizierung wurden nach Standardvorschriften hergestellt. So erhielt man Fmoc-cis-Hyp-OH durch Umsetzung von H-cis-Hyp-OH mit Fmoc-OSu [Paquet et al. 1982 Canadian Journal of Chemistry 60: 976-980A]. Fmoc-Phe(4-STrt-Amidino)-OH synthetisierte man durch eine bekannte Vorschrift [Pearson et al. 1996 Journal of Medicinal Chemistry 39:1372-1382]. Seitenketten-mödifizierte Cystein-Derivate wurden durch Alkylierung von Fmoc-Cystein-OH mit Alkylhalogeniden dargestellt.

Bei den Konzentrationen der Reagenzien in Prozent handelt es sich, sofern nicht anders angegebenen, um Volumenprozent (v/v).

### RP-HPLC-MS-Analysen:

Analytische Chromatographie erfolgte unter Verwendung eines Hewlett Packard Serie 1100-Systems (Entgaser G1322A, quaternäre Pumpe G1311A, automatischer Probengeber G1313A, thermostatiertes Säulenfach G 1316A, Variabler UV-Detektor G1314A) und gekoppelter ESI-MS (Finnigan LCQ Ion-Trap-Massenspektrometer). Dazu wurde eine Steuersoftware der Firma Finnigan verwendet (Navigator Ver 1.1 spl). Als Stossgas in der Ionenfalle diente Helium. Die Trennung erfolgte an RP-18-Säulenmaterial (Vydac 218 TP5215, 2,1 x 150 mm, 5 µm, C18, 300 A mit Vorsäule (Merk)) bei 30°C und einem Fluss von 0,3 ml/min unter Anwendung eines linearen Gradienten für alle Chromatogramme (5-95 % B innerhalb von 25 min, wobei A: 0,05 % TFA in Wasser und B: 0,05 % TFA in CH₃CN). Die UV-Detektion erfolgte bei λ = 220 nm. Retentionszeiten (Rₜ) sind im Dezimalsystem angegeben (z.B. 1,9 min = 1 min 54 sec) und beziehen sich auf die Detektion im Massenspektrometer. Die Totzeit zwischen Injektion und UV-Detektion (HPLC) betrug 1,65 min, zwischen UV-Detektion und Massendetektion 0,21 min. Die Genauigkeit des Massenspektrometers beträgt ca. ± 0,2 amu.

Analytik mittels HPLC-MS, Injektion von 5 µl, Gradient linear von 95:5 zu 5:95 in 9.5 min (A; Wasser mit 0,05% TFA, B: Acetonitril mit 0,05% TFA), RP-Säule der Firma Phenomenex, Typ Luna (C-18), 3um, 50x2.00 mm, Fluss 0,3 ml, bei Raumtemperatur HPLC; ThermoFinnigan Surveyor mit PDA-Detektor (210-350 nm), MS; ThermoFinnigan Advantage bzw. LCQ-Classic (beide Iontrap), ESI-Ionisation, als Stossgas in der Ionenfalle diente Helium. Excalibur Vers. 1.3 bzw. 1.2. Retentionszeiten (Rt) werden im Dezimalsystem angegeben (z.B. 1,9 min = 1 min 54 sec).

### Präparative HPLC:

Präparative HPLC-Trennungen wurden auf Vydac R18-RP-Säulen mit Gradienten folgender Lösungsmittel durchgeführt: A: 0,05 % TFA in Wasser und B: 0,05 % TFA in CH₃CN).

**Tabelle 1:**

| Verwendete Abkürzungen: | |
|---|---|
| Abb. | Abbildung |
| AAV | Allgemeine Arbeitsvorschrift |
| Ac | Acetyl |
| Acm | Acetamidomethyl |
| Ac | Acetyl |
| d | Dublett |
| DCM | Dichlormethan |
| DIC | Diisopropylcarbodiimid |
| DIPEA | N,N-Diisopropylethylamin |
| DMF | *N*,*N*-Dimethylformamid |
| DMEM | Dulbecco's Modified Eagle Medium |
| DMSO | Dimethylsulfoxid |
| eq. | Äquivalent(e) |
| Fmoc | 9-Fluorenylmethyloxycarbonyl |
| h | Stunde(n) |
| HATU | O-(7-Azabenzotriazol-1-yl)-1,1,3,3-tetramethyluronium-Hexafluorophosphat |
| HBTU | *O*-(Benzotriazol-1-yl)-1,1,3,3-tetramethyluronium-Hexafluorophosphat |
| HEPES | N-2-2-Hydroxyethyl-1-piperazin-N'-2-ethanolsulfonsäure |
| HOBt | 1-Hydroxybenzotriazol |
| HPLC | high-pressure liquid chromatography |
| m | Multiplett |
| Me | Methyl |
| min | Minute(n) |
| ml | Mililiter |
| NMI | *N*-Methylimidazol |
| NMP | *N*-Methylpyrrolidon |
| NMR | nuclear magnetic resonance |
| s | Singulett |
| ^{t}_{Bu} | tert-Butyl |
| THF | Tetrahydrofuran |
| TFA | Trifluoressigsäure |

**Tabelle 2:**

| Für proteinogene Aminosäuren wurde der Drei-Buchstaben-Code verwendet: | | | |
|---|---|---|---|
| 3-Buchstaben-Code | Aminosäure | 3-Buchstaben-Code | Aminosäure |
| Ala | Alanin | Met | Methionin |
| Cys | Cystein | Asn | Asparagin |
| Asp | Asparaginsäure | Pro | Prolin |
| Glu | Glutaminsäure | Gln | Glutamin |
| Phe | Phenylalanin | Arg | Arginin |
| Gly | Glycin | Ser | Serin |
| His | Histidin | Thr | Threonin |
| Ile | Isoleucin | Val | Valin |
| Lys | Lysin | Trp | Tryptophan |
| Leu | Leucin | Tyr | Tyrosin |

**Tabelle 3:**

| Für nicht-proteinogene Aminosäuren wird ein Dreibuchstabencode verwendet, bei dem der erste Buchstabe die Stereochemie des C-alpha-Atoms beschreibt. Ein großer erster Buchstabe steht für die L-Form, ein kleiner erster Buchstabe für die D-Form der jeweiligen Aminosäure. | |
|---|---|
| 1Ni | 1-Naphthylalanin |
| 2Ni | 2-Naphthylalanin |
| 3PP | 3-Phenylpropionyl |
| Aoa | Aminooxyessigsäure |
| Aoc | 1-Aza-bicyclo-[3.3.0]-octan-2-carbonsäure |
| Aze | Azetidin-2-carbonsäure |
| Bta | Benzothienylalanin |
| Cha | Beta-Cyclohexylalanin |
| Chg | Cyclohexylglycin |
| Cit | Citrullin |
| Dab | α, γ-Diaminobuttersäure |
| Eaf | Allylglycin |
| Eag | 2-Propargylglycine |
| Eap | Phe(4-^{t}Bu) |
| Eay | 4-Phenyl-pyrrolidin-2-carbonsäure |
| Ebd | Cys(Et) |
| Ebo | Cys(4-picolyl) |
| Ebu | Cys(3-picolyl) |
| Ebw | 3,3-Diphenylalanin |
| Eby | (S)-3-Amino-3-phenylpropansäure |
| Ecf | Cys(O-3-picolyl) |
| Ecg | Cys(2-picolyl) |
| Ecp | His(tau-4-Methoxybenzyl) |
| Ecr | His(tau-methyl) |
| Edn | Cys(CH₂-CH₂-4-Pyridyl) |
| Eec | Cys(1-Methylen-1H-benzotriazol) |
| Eew | Arg(NO₂) |
| Guf | Phe(4-guanidin) |
| Har | Homo-Arginin |
| Hch | Homo-Cyclohexylalanin |
| Hci | Homo-Citrullin |
| Hle | Homo-Leucin |
| Hyp | Hydroxyprolin |
| Hyp | Hydroxyproline |
| Mcf | Phe(3-Cl) |
| Mpa | 3-Pyridyl-Alanin |
| Nle | Norleucin |
| Nva | Norvalin |
| Oic | Octahydroindol-2-carbonsäure |
| Omf | Phe(2-Me) |
| Orn | Ornithin |
| Paf | Phe(4-NH₂) |
| Pcf | Phe(4-Cl) |
| Pff | Phe(4-F) |
| Phg | Phenylglycin |
| Pip | Pipecolinsäure |
| Pmf | Phe(4-Me) |
| Ppa | 3-(4-Pyridyl)-alanine |
| Thi | 2-Thienylalanin |
| Tic | Tetrahydroisochinolin-3-carbonsäure |
| Tiq | Tetrahydroisochinolin-1-carbonsäure |
| XX1 | 2-Amino-3-(4-piperidinyl)propionsäure |
| XX2 | 4-Guanidyl-piperidinyl-Alanin |

Die Aktivität von Verbindungen wird durch folgende Konvention vereinfacht beschrieben:

| | |
|---|---|
| IC₅₀ < 5 nM | A |
| 5 nM < IC₅₀ ≤ 10 nM | B |
| 10 nM < IC₅₀ ≤ 20 nM | C |
| 20 nM < IC₅₀ ≤ 50 nM | D |
| 50 nM < IC₅₀ ≤ 200 nM | E |

200 nM < IC₅₀ ≤ 2000 nM: F
2000 nM < IC₅₀ G

### Allgemeine Arbeitsvorschrift (AAV) 1: Synthese linearer Peptide

Lineare Peptide wurden nach der Fmoc-'Bu-Strategie im Batch-Verfahren synthetisiert. Dabei führte man entweder eine manuelle Synthese in Polypropylen-Spritzen mit Fritten durch oder setzte automatische Synthesizer (Syro der Fa. Multisyntech, Witten oder Sophas der Fa. Zinsser, Frankfurt) ein.

Zur Herstellung von Peptiden mit C-terminaler Carbonsäure wurde die C-terminale Aminosäure entweder an Tritylchlorid-Harz angeknüpft (ca. 200 mg Harz; Beladung reaktiver Gruppen ca. 1,5 mmol/g; Anknüpfung durch Umsetzung mit 0,8 eq. Fmoc-Aminosäure und 3,0 eq. DIPEA in CH₂Cl₂ für 2 Stunden; erhaltene Beladung der Aminosäure ca. 0,2-0,4 mmol/g) oder es erfolgte eine Anknüpfung an Wang-Harz (200-500 mg Harz; Beladung reaktiver Gruppen ca. 0,6 mmol/g; Anknüpfung durch Umsetzung mit 4 eq. Fmoc-Aminosäure und 4 eq. DIC und 3 eq. NMI in DMF für 3 Stunden; erhaltene Beladung der Aminosäure ca. 0,2-0,6 mmol/g).

Bei der Herstellung von Peptiden mit C-terminalem Carboxamid wurde die erste Aminosäure durch Fmoc-Abspaltung von Fmoc-Rinkamid-Harz (ca. 200 mg Harz; Fmoc-Abspaltung mit 20 % Piperidin in DMF für 20 min) und anschließende Kupplung der Fmoc-Aminosäure (einmalige oder mehrmalige Umsetzung mit 5 eq. Fmoc-Aminosäure; 5 eq. HBTU und 15 eq. DIPEA in DMF für 30-60 min) angeknüpft.

Nach Anknüpfung der ersten Aminosäure wurde das gewünschte Peptid durch eine je nach Bedarf wiederholte Sequenz aus Fmoc-Abspaltung und Anknüpfung der jeweils benötigten Fmoc-Aminosäure oder Carbonsäure hergestellt. Zur Abspaltung der Fmoc-Schutzgruppe wurde dabei das Harz mit 20 % Piperidin in DMF für 20 min umgesetzt. Kupplungen erfolgten durch einmalige oder mehrmalige Umsetzung mit 5 eq. der Aminosäure, 5 eq. HBTU und 15 eq. DIPEA in DMF für 30-60 min). Zur Einführung N-terminaler Acetylgruppen setzte man das N-terminal freie harzgebundene Peptid mit 10 % Essigsäureanhydrid und 20 % DIPEA in DMF für 20 min um.

Zur Abspaltung des fertig synthetisierten Peptids vom Harz und zur Entfernung von Seitenschutzgruppen setzte man ein Gemisch aus 95 % TFA, 2,5 % Wasser, 2,5 %TIPS oder eine ähnliche saure Lösung ein. Anschließend wurde das TFA am Rotationsverdampfer entfernt oder das erhaltene Peptid mit Methyl-^{t}Butyl-Ether bei 0°C gefällt und nach Zentrifugation und Dekantieren der überstehenden Lösung isoliert. Zur Überführung eventuell erhaltener Trifluoracetat-Salze in die entsprechenden HCl-Salze wurde das Peptid mit einem Gemisch aus 2 N HCl und MeCN gelöst und lyophilisiert.

Peptide mit C-terminalem Carboxamid wurden direkt einer Reinigung per HPLC unterzogen, Peptide mit C-terminaler Carbonsäure wurden dagegen in der Regel als Rohprodukt nach AAV2 cyclisiert.

### Allgemeine Arbeitsvorschrift (AAV) 2: Cyclisierung von Peptiden mit C-terminaler Carbonsäure

Zur Cyclisierung wurden ca. 80 mg des nach AAV1 synthetisierten linearen Peptids in 5 ml DMF und 5 ml CH₂Cl₂ gelöst. Anschließend stellte man mit N-Ethylmorpholin einen pH-Wert von ca. 8 ein und setzte 1 eq. HOBt sowie 10 eq. DIC zu. Nach 2-16stündigem Rühren bei Raumtemperatur wurde das Lösungsmittel am Rotationsverdampfer entfernt und das erhaltene Rohprodukt per HPLC gereinigt.

### Allgemeine Arbeitsvorschrift (AAV) 3: Reduktive Alkylierung von N-terminal freien Harzgebundenen Peptiden

Das nach AAV synthetisierte lineare Peptid mit freiem N-Terminus wurde vor der Abspaltung vom Harz mit 10 eq. des entsprechenden Aldehyds in 5 %iger Essigsäure und 5 %igem Trimethylorthoformiat in THF umgesetzt. Nach ca. 4 Stunden wurde das erhaltene Imin mit 5 eq. Natriumcyanoborhydrid über Nacht reduziert.

Nach Abspaltung des fertig synthetisierten Peptids vom Harz gemäß AAV1 konnte das erhaltene Rohprodukt gemäß AAV2 cyclisiert werden. Neben der gewünschten Cyclisierung trat hier in der Regel auch eine unerwünschte Cyclisierung auf das N-terminale sekundäre Amin auf. Das hierdurch entstandene Nebenprodukt ließ sich aber problemlos per HPLC abtrennen.

### Beispiel 2: Synthese von Ac-Phe-[Orn-Pro-cha-Trp-Phel (1)

Nach linearer Peptidsynthese gemäß AAV 1, Cyclisierung gemäß AAV 2 und anschließender Reinigung per HPLC erhielt man 50,9 mg des gewünschten Produkts Ac-Phe-[Om-Pro-cha-Trp-Phe] als weißen Feststoff.
MS (ESI): m/z = 888,3 [(M+H)⁺].

### Beispiel 3: Synthese von Ac-Phe-[Orn-Hyp-cha-Trp-Phe] (2)

Das lineare Peptid Ac-Phe-Orn-Hyp-cha-Trp-Phe-OH wurde nach linearer Peptidsynthese gemäß AAV 1 hergestellt und nach AAV 2 cyclisiert. Aufgrund der höheren Nucleophilizität von Aminen gegenüber Alkoholen entstand dabei neben dem gewünschtem cyclisierten Produkt kein Produkt durch unerwünschte Veresterung der Hyp-OH-Gruppe mit der C-terminalen Carboxylgruppe. Reinigung des erhaltenen Rohproduktes durch HPLC führte zu 26,9 mg des gewünschten weißen Feststoffes Ac-Phe-[Orn-Hyp-cha-Trp-Phe] (**2**).
MS (ESI): m/z = 903,5 [(M+H)⁺].

### Beispiel 4 : Synthese von Ph-CH₂-[Orn-Pro-cha-Trp-Nle] (56)

Das harzgebundene Peptid H-Orn-Pro-cha-Trp-Nle-Trityl-Harz wurde nach linearer Peptidsynthese gemäß AAV 1 hergestellt und gemäß AAV 3 einer reduktiven Alkylierung mit Benzaldehyd unterzogen. Cyclisierung gemäß AAV 2 und anschließende Reinigung per HPLC führten zu 0,9 mg des gewünschten Produkts **56** als weißen Feststoff.
MS (ESI): m/z = 753,4 [(M+H)⁺].

### Beispiel 5: Synthese von HOCH₂(CHOH)₄-C=N-O-CH₂-CO-Phe-[Orn-Pro-cha-Trp-Nle] (3)

Das lineare Peptid H-Aoa-Phe-Orn-Pro-cha-Trp-Nle-OH wurde gemäß AAV 1 hergestellt, in 24 ml MeCN/Natriumacetatpuffer (0.2 M, pH = 4) 1:1 gelöst und mit 58 mg (10 eq.) D-Glucose versetzt. Nach 4-tägigem Rühren wurde zum quenchen von nicht umgesetztem Aminooxyessigsäure-Peptid mit 2,4 ml Aceton versetzt und nach 5 min das Lösungsmittel im Vakuum entfernt. Das erhaltene Rohprodukt wurde per HPLC gereinigt und anschließend nach AAV 2 cyclisiert. Reinigung des erhaltenen Rohproduktes durch HPLC führte zu 1,9 mg des gewünschten weißen Feststoffes **3**.
MS (ESI): m/z = 1046,5 [(M+H)⁺].

### Beispiel 6: Synthese von 2-Acetamido-1-Methyl-Glucuronyl-Phe-[Orn-Pro-cha-Trp-Nle] (4)

Das harzgebundene Peptid H-Phe-Orn-Pro-cha-Trp-Nle-Trityl-Harz wurde nach linearer Peptidsynthese gemäß AAV 1 hergestellt und mit 39,8 mg (2,0 eq.) 2-Acetamido-1-Methyl-Glucuronsäure (Schämann et al. 2003 European Journal of Organic Chemistry: 351-358), 60,8 mg (2,0 eq.) HATU und 105,7 *µ*l (10 eq.) 2,4,6-Collidin in 1,6 ml DMF umgesetzt. Nach 1,5-stündigem Rühren wurde das Harz mit DMF (5x), MeOH (5x) und CH₂Cl₂ (3x) gewaschen und das Peptid mit einem Gemisch aus 95 % TFA, 2,5 % Wasser und 2,5 %TIPS vom Harz abgespalten. Cyclisierung gemäß AAV 2 und anschließende Reinigung per HPLC führten zu 29,0 mg des gewünschten Produkts **4** als weißen Feststoff.
MS (ESI): m/z = 1043,0 [(M+H)⁺].

### Beispiel 7: Synthese von Ac-Phe-[Orn-Hyp(COCH₂OCH₂CH2OCH₂CH₂OCH₃)-Cha-Trp-Nle] (5)

Das lineare Peptid Ac-Phe-Orn-Hyp-cha-Trp-Nle-OH wurde gemäß AAV 1 hergestellt, nach AAV 2 cyclisiert und das entstandene zyklisierte Peptid Ac-Phe-[Orn-Hyp-cha- Trp-Nle] per HPLC gereinigt. 35,4 µl (40 eq.) 2-(2-(2-Methoxyethoxy)ethoxy)essigsäure wurden für 15 min bei 40 °C mit 50,3 *µ*l (120 eq.) Thionylchlorid umgesetzt. Nach Entfernung des Lösungsmittels im Vakuum setzte man 78,8 ml (80 eq.) DIPEA, 1 ml CH₂Cl₂ und 5,0 mg der Verbindung Ac-Phe-[Orn-Hyp-cha-Trp-Nle] zu. Man rührte für 3 Tage bei Raumtemperatur und reinigte per HPLC. Dieses führte zu 1,6 mg des gewünschten weißen Feststoffes 5.
MS (ESI): m/z = 1029,6 [(M+H)⁺].

### Beispiel 8: Synthese von Ac-Phe-[Orn-Hyp(CONH-CH₂CH(OH)-CH₂OH)-cha-Trp-Nle] (6)

Das lineare Peptid Ac-Phe-Orn-Hyp-cha-Trp-Nle-OH wurde gemäß AAV 1 hergestellt, nach AAV 2 cyclisiert und das entstandene zyklisierte Peptid Ac-Phe-[Orn-Hyp-cha-Trp-Nle] per HPLC gereinigt. Anschließend setzte man 5,0 mg des Peptids mit 26,1 mg 4-Isocyanatomethyl-2,2-dimethyl-[1,3]dioxolan und 1,88 µl (2,0 eq.) DIPEA in 0,3 ml MeCN um. Nach 3-tägigem Rühren bei 40 °C wurde das Lösungsmittel am Rotationsverdampfer entfernt und das erhaltene Rohprodukt per HPLC gereinigt. Man erhielt 0,22 mg des gewünschten weißen Feststoffes 6.
MS (ESI): m/z = 986,5 [(M+H)⁺].

### Beispiel 9: Synthese von Ac-Phe-[Orn-Pro-cha-Trp-Arg(CH₂CH₂)] (7)

Man stellte das lineare Peptid Ac-Phe-Orn-Pro-cha-Trp-Orn-OH gemäß AAV 1 her, cyclisierte nach AAV 2 und reinigte das entstandene zyklisierte Peptid Ac-Phe-[Orn-Pro-cha-Trp-Orn] per HPLC. Anschließend setzte man 2,6 mg des Peptids mit 22,6 mg (30 eq.) 2-(Methylmercapto)-2-imidazolin-Hydroiodid und 29,7 *µ*l (60 eq.) DIPEA in 260 *µ*l MeOH um. Nach 2-tägigem Rühren bei 50 °C wurde das Lösungsmittel am Rotationsverdampfer entfernt und das erhaltene Rohprodukt per HPLC gereinigt. Man erhielt 0,86 mg des gewünschten weißen Feststoffes 7.
MS (ESI): m/z = 922,8 [(M+H)⁺].

### Beispiel 10: Synthese von Ph-CH₂-CH₂-CO-[Orn-Pro-cha-Trp-Nle] (41)

Das Peptid Ph-CH₂-CH₂-CO-Orn-Pro-cha-Trp-Nle-OH wurde nach linearer Peptidsynthese gemäß AAV 1 hergestellt, wobei 3-Phenylproionsäure als N-terminale Carbonsäure eingesetzt wurde. Man cyclisierte gemäß AAV 2 und reinigte das erhaltene Rohprodukte per HPLC. Man erhielt 3,13 mg des gewünschten weißen Feststoffes **41**.
MS (ESI): m/z = 796,5 [(M+H)⁺].

### Beispiel 11: Bestimmung des IC₅₀ Wertes in einem Enzymfreisetzungsassay

Die Durchführung des Assays ist bei Köhl (Köhl 1997 The Anaphylatoxins. In: Dodds, A.W., Sim, R.B. (Eds.), Complement: A Practical Approach. Oxford, pp. 135-163) beschrieben. Basophile Leukämie-Zellen aus Ratten (RBL), die den humanen C5aR (CD88) exprimieren, werden in DMEM mit 10% fötalem Kälberserum, 100 U/ml Penicillin, 100µg/ml Streptomycin und 2 mM Glutamin (alle Medienbestandteile Biochrome, Berlin) bis zur Konfluenz bei 37°C und 10% CO₂ angezogen. Alle folgenden Angaben beziehen sich auf eine Kulturflasche mit 75 cm² Fläche. Verbrauchtes Medium wird abgegossen. Zellen werden mit 10 ml PBS (Dulbecco's PBS, Biochrome) gewaschen und anschließend mit 3 ml Cell Dissociation Solution (CDS, Sigma) überschichtet. Zellen werden bei RT 1 min inkubiert. Anschließend wird die CDS entfernt und die Zellen werden weitere 10-15 min bei 37°C zum Ablösen inkubiert. Im Assay werden 20 µl Lösung der zu testenden Verbindung verwendet. Die Assaylösung darf nicht mehr als 2,8 % DMSO enthalten. In Verdünnungsreihen wird in 1/3 oder 1/2 Schritten verdünnt. Zu den 20 µl Lösungen der Verbindungen werden 75 µl folgendermaßen behandelter RBL-Zellen gegeben: Nach der Ablösephase werden die Zellen heftig abgeklopft und in 10 ml auf 37°C temperierten HAG-CM aufgenommen (20 mM HEPES; 125 mM NaCl, 5mM KCl, 1 mM CaCl₂, 1mM MgCl₂, 0,5 mM Glucose, 0,25% BSA. HEPES herstellen: 2,3 g/l HEPES-Salz + 2,66 g/l HEPES Säure). Zellen werden gezählt und zentrifugiert (200g, 10 min). Das Zellpellet wird mit vorgewärmten HAG-CM (d.h. Hepesgepufferte NaCl-Glucose-Lösung mit Calcium und Magnesium) aufgenommen, und die Zelldichte auf 2x10⁶ Zellen/ml eingestellt. Die Zellen werden bei 37°C für 5 min inkubiert. Zu den Zellen kommen pro ml Zellsuspension 27 µl einer Cytochalasin B-Lösung (100µg/ml in DMSO, Sigma). Die Zellen werden weitere 3 min bei 37°C inkubiert. 75 µl Zellsuspension werden zu den 20 µl Lösung mit der zu testenden Verbindung gegeben. Damit ergibt sich ein Volumen von 95 µl pro Well. Die Zellen werden 10 min. bei 37°C inkubiert. Dann werden pro Well 10 µl hrC5a (10,5 nM in HAG-CM, Sigma) gegeben. Es folgt eine Inkubation für 5 min bei 37°C. Anschließend werden die Platten auf Eis gestellt und bei 1200xg und 4°C für 3 min zentrifugiert. 75 µl des Überstands werden zu 100 µl Substrat-Lösung (2,7 mg/ml p-Nitrophenyl-N-acetyl-b-D-Glucosaminide (Sigma) in 42,5 mM Na-Acetat pH 4.5) gegeben. Die Platte wird für 1 h bei 37°C inkubiert. Pro Well werden 75 µl 0,4 M Glycin pH 10.4 gegeben. Die Platte kann anschließend bei 405 nm gemessen werden. Der IC₅₀-Wert wird durch die Lösung der 4-Parametergleichung y=((A-D)/(1+(x/C)^{B}))+D bestimmt.

Die Ergebnisse des Tests zur Bestimmung der IC₅₀-Werte sind in Tabelle 4 dargestellt.

**Tabelle 4:**

| Daten zur Antagonistischen Aktivität repräsentativer erfindungsgemäßer Verbindungen | | | |
|---|---|---|---|
| **Nr.** | **Verbindung** | (M+H)⁺ im MS [amu] | **Aktivität (klassifiziert)** |
| **1** | Ac-Phe-[Orn-Pro-cha-Trp-Phe] | 888,3 | D |
| **2** | Ac-Phe-[Orn-Hyp-cha-Trp-Phe] | 903,5 | D |
| **3** | HOCH₂(CHOH)₄-C=N-O-CH₂-CO-Phe-[Om-Pro-cha-Trp-Nle] | 1046,5 | E |
| **4** | X-Phe-[Orn-Pro-cha -Trp-Nle]; X = 2-Acetamido-1-Methyl-Glucuronyl | 1043,0 | D |
| **5** | Ac-Phe-[Orn-Hyp(COCH₂OCH₂CH2OCH₂CH₂OCH₃)-cha-Trp-Nle] | 1029,6 | E |
| **6** | Ac-Phe-[Orn-Hyp(CONH-CH₂CH(OH)-CH₂OH)-cha-Trp-Nle] | 986,5 | E |
| **7** | Ac-Phe-[Orn-Pro-cha-Trp-Arg(CH₂CH₂)] | 922,8 | F |
| **8** | Ac-Phe-[Orn-Pro-cha-Trp-Har] | 910,7 | F |
| **9** | Ac-Phe-[Orn-Pro-cha-Trp-Guf] | 944,6 | F |
| **10** | Ac-Phe-[Orn-Pro-cha-Trp-Cit] | 897,5 | F |
| **11** | Ac-Phe-[Orn-Pro-cha-Trp-Eew] | 941,5 | F |
| **12** | Ac-Phe-[Orn-Pro-cha-Trp-arg] | 896,7 | F |
| **13** | Ac-Phe-[Orn-Pro-cha-Trp-Hci] | 911,6 | F |
| **14** | Ac-Phe-[Orn-Pro-cha-Trp-Paf] | 902,7 | D |
| **15** | Ac-Phe-[Orn-Pro-cha-Trp-Ebo] | 934,6 | F |
| **16** | Ac-Phe-[Orn-Pro-cha-Trp-Ecf] | 950,6 | F |
| **17** | Ac-Phe-[Orn-Pro-cha-Trp-Ebu] | 934,7 | F |
| **18** | Ac-Phe-[Orn-Pro-cha-Trp-Ecg] | 934,6 | F |
| **19** | Ac-Phe-[Orn-Pro-cha-Trp-Edn] | 948,6 | F |
| **20** | Ac-Phe-[Orn-Pro-cha-Trp-Ecr] | 891,7 | E |
| **21** | Ac-Phe-[Orn-Pro-cha-Trp-Phe(4-Amidin)] | 929,7 | F |
| **22** | Ac-Phe-[Orn-Pro-cha-Trp-Lys] | 868,6 | G |
| **23** | Ac-Phe-[Orn-Pro-cha-Trp-Ppa] | 888,6 | E |
| **24** | Ac-Phe-[Orn-Pro-cha-Trp-Arg(Me₂)] | 924,7 | E |
| **25** | Ac-Phe-[Orn-Pro-cha-Trp-Dab] | 840,4 | E |
| **26** | Ac-Phe-[Orn-Pro-cha-Trp-Ecp] | 997,7 | F |
| **27** | Ac-Phe-[Orn-Pro-cha-Trp-XX1] | 894,6 | G |
| **28** | Ac-Phe-[Orn-Pro-cha-Trp-Nle] | 852,6 | D |
| **29** | Ac-Phe-[Orn-Pro-cha-Trp-Met] | 871,6 | E |
| **30** | Ac-Phe-[Orn-Pro-cha-Trp-XX2] | 936,5 | G |
| **31** | Ac-Phe-[Orn-Pro-cha-Trp-Nva] | 839,5 | C |
| **32** | Ac-Phe-[Orn-Pro-cha-Trp-Hle] | 867,5 | D |
| **33** | Ac-Phe-[Orn-Pro-cha-Trp-Eaf] | 837,5 | B |
| **34** | Ac-Phe-[Orn-Pro-cha-Trp-Ebd] | 871,5 | D |
| **35** | Ac-Phe-[Orn-Pro-cha-Trp-Eag] | 835,5 | B |
| **36** | Ac-Phe-[Orn-Pro-cha-Trp-Pmf] | 901,6 | D |
| **37** | Ac-Phe-[Orn-Pro-cha-Trp-2Ni] | 937,5 | E |
| **38** | Ac-Phe-[Orn-Pro-cha-Trp-Thi] | 893,5 | D |
| 39 | Ac-Phe-[Orn-Pro-cha-Trp-Ala] | 811,7 | G |
| **40** | Ac-Phe-[Orn-Pro-cha-Trp-Arg] | 896,6 | C |
| **41** | Ph-CH₂-CH₂-CO-[Orn-Pro-cha-Trp-Nle] | 796,5 | C |
| **42** | H-Phe-[Orn-Pro-cha-Trp-Nle] | 811,5 | C |
| **43** | Ac-Lys-Phe-[Orn-Pro-cha-Trp-Nle] | 1015,7 | D |
| **44** | H-Phe-[Orn-Ser-cha-Trp-Nle] | 843,5 | D |
| **45** | Ac-Ala-[Orn-Pro-cha-Trp-Arg] | 820,6 | G |
| **46** | Ac-Phe-[Orn-NMeAla-cha-Trp-Arg] | 884,8 | D |
| 47 | Ac-Phe-[Orn-Pro-ala-Trp-Arg] | 814,8 | G |
| 48 | Ac-Phe-[Orn-Pro-cha-Ala-Arg] | 781,8 | G |
| **49** | Ac-Phe-[Orn-Pro-cha-Trp-Ala] | 811,7 | G |
| **50** | Ac-Phe-Orn-Pro-cha-Trp-Arg-NH₂ | 913,3 | E |
| **51** | Ac-Phe-Orn-Pro-cha-Trp-Phe-NH₂ | 904,5 | D |
| **52** | Ac-Phe-Orn-Aze-cha-Bta-Phe-NH₂ | 907,5 | C |
| **53** | Ac-Phe-Orn-Pro-cha-Bta-2Ni-NH₂ | 954,4 | D |
| **54** | Ac-Phe-Orn-Pro-cha-Bta-Cha-NH₂ | 910,5 | E |
| **55** | Ac-Phe-Orn-Pip-cha-Trp-Phe-NH₂ | 941,3 | D |
| **56** | Ph-CH₂-[Orn-Pro-cha-Trp-Nle] | 753,4 | D |
| **57** | Ph-CH₂-[Orn-Pro-cha-Trp-Phe] | 787,5 | D |
| **58** | Ac-Phe-[Orn-Pro-cha-Trp-1Ni] | 937,7 | D |
| **59** | Ph-CH(OH)-CH₂-CO-[Om-Pro-cha-Trp-Nle] | 812,4 | D |
| **60** | Ac-Phe-Lys-Ala-Cha-Ala-Leu-ala-Tyr-OH | 978,9 | G |
| **61** | Ac-Phe-Orn-Pro-cha-Trp-Phe-NH₂ | 904,9 | D |
| **62** | Ac-Phe-Orn-Pro-cha-Bta-Phe-NH₂ | 921,8 | D |
| **64** | Ac-Phe-Orn-Pro-cha-Trp-2Ni-NH₂ | 954,9 | D |
| **65** | Ac-Phe-Orn-Pro-cha-Trp-Cha-NH₂ | 911,1 | E |
| **66** | Ac-Thi-Orn-Aze-cha-Bta-Phe-NH₂ | 913,5 | C |
| **67** | Ac-Thi-Orn-Pip-cha-Bta-Phe-NH₂ | 941,3 | D |
| **68** | Ac-Phe-Orn-Pro-cha-Trp-Eap-NH₂ | 960,9 | F |
| **69** | Me₂-Phe-Orn-Pro-cha-Trp-Phe-NH₂ | 890,8 | E |
| **70** | Ph₂-CH-CH₂-CO-Orn-Pro-cha-Trp-Phe-NH₂ | 923,7 | F |
| **71** | Ac-Ebw-Orn-Pro-cha-Trp-Phe-NH₂ | 980,8 | F |
| **72** | Ac-Phe-Orn-Pro-cha-Trp-NH-CH₂-CH₂-Ph | 861,8 | F |
| **73** | Ac-Phe-Orn-Aze-cha-Bta-NH-CH₂-CH₂-Ph | 864,7 | F |
| **74** | H-Phe-Orn-Pro-cha-Trp-Phe-NH₂ | 862,7 | E |
| **75** | H-Me-Phe-Orn-Pro-cha-Trp-Phe-NH₂ | 876,7 | E |
| **76** | Bu-NH-CO-Phe-Orn-Pro-cha-Trp-Phe-NH₂ | 961,8 | F |
| **77** | Ac-Thi-Orn-Pro-cha-Trp-Phe-NH₂ | 910,7 | E |
| **78** | Ac-Ebw-Orn-Pro-cha-Trp-Phe-NH₂ | 980,8 | E |
| **79** | Ac-Phe-Orn-Ala-cha-Trp-Phe-NH₂ | 878,7 | E |
| **80** | Ac-Phe-Orn-Pro-cha-Trp-Thi-NH₂ | 910,7 | E |
| **81** | Ac-Phe-Orn-Aze-cha-Pcf Phe-NH₂ | 885,7 | F |
| **82** | Ac-Phe-Orn(Ac)-Pro-cha-Trp-Phe-NH₂ | 946,9 | E |
| **83** | Ac-Phe-Orn-Aze-cha-Trp-Phe-NH₂ | 890,9 | D |
| **84** | Ac-Phe-Trp-Pro-cha-Trp-Phe-NH₂ | 976,5 | E |
| **85** | Ph-NH-CO-Phe-Orn-Pro-cha-Trp-Phe-NH₂ | 981,7 | E |
| **86** | Bu-O-CO-Phe-Orn-Pro-cha-Trp-Phe-NH₂ | 963,2 | F |
| **87** | Ac-Phe-Lys-Pro-cha-Trp-Phe-NH₂ | 918,4 | E |
| **88** | Ac-Phe-Arg-Pro-cha-Trp-Phe-NH₂ | 946,4 | D |
| **89** | Ac-Phe-Gln-Pro-cha-Trp-Phe-NH₂ | 918,4 | F |
| **90** | Ac-Phe-Ser-Pro-cha-Trp-Phe-NH₂ | 877,3 | F |
| **91** | Ac-Phe-Glu-Pro-cha-Trp-Phe-NH₂ | 919,3 | F |
| **92** | Ac-Phe-Orn-Pip-cha-Trp-Phe-NH₂ | 919,8 | E |
| **93** | Ac-Phe-Orn-Hyp-cha-Trp-Phe-NH₂ | 920,3 | F |
| **94** | Ac-Phe-Orn-Pro-cha-Trp-1Ni-NH₂ | 934,5 | D |
| **95** | Ac-Phe-Orn-Aze-cha-Bta-Phe-NH-Me | 921,6 | F |
| **96** | CH₃-SO₂-Phe-Orn-Aze-cha-Bta-Phe-NH₂ | 943,9 | D |
| **99** | Ac-Phe-Orn-Aze-cha-Pff-Phe-NH₂ | 869,7 | E |
| **100** | Ac-Phe-Orn-Aze-cha-Mcf-Phe-NH₂ | 885,7 | E |
| **101** | Ac-Phe-Orn(Ac)-Aze-cha-Bta-Phe-NH₂ | 921,7 | D |
| **102** | Ac-Ebw-Orn-Pro-cha-Trp-Phe-NH₂ | 980,8 | E |
| **103** | Ac-Phe-Trp-Pro-cha-Trp-Phe-NH₂ | 876,5 | E |
| **104** | Ac-Phe-Arg-Pro-cha-Trp-Phe-NH₂ | 946,4 | E |
| **105** | Ac-Phe-Orn-Pip-cha-Trp-Phe-NH₂ | 919,8 | E |
| **106** | 3PP-Orn-Aze-cha-Bta-Phe-NH₂ | 850,8 | E |
| **107** | Ac-Phe-Orn-Tic-cha-Trp-Phe-NH₂ | 966,3 | E |
| **108** | Ac-Phe-Orn-Ser-cha-Trp-Phe-NH₂ | 894,5 | D |
| **109** | Ac-Phe-Orn-Pro-chg-Trp-Phe-NH₂ | 890,4 | E |
| **110** | Ac-Phe-Orn-Pro-hch-Trp-Phe-NH₂ | 918,5 | D |
| **111** | Ac-Phe-Orn-Pro-cha-Trp-Phg-NH₂ | 890,4 | F |
| **112** | Ac-Phe-Bta-Aze-cha-Bta-Phe-NH₂ | 996,6 | D |
| **113** | Ac-Phe-Trp-Pro-cha-Bta-Phe-NH₂ | 993,7 | E |
| **115** | Ac-Phe-Orn-Pip-cha-Trp-Phe-OH | 919,4 | F |
| 116 | Ac-Phe-Orn-Tic-cha-Trp-Phe-OH | 967,7 | F |
| 117 | Ac-Phe-Orn-Ser-cha-Trp-Phe-OH | 895,7 | F |
| **118** | Ac-Phe-Orn-Pro-chg-Trp-Phe-OH | 891,8 | F |
| **119** | Ac-Phe-Eec-Pro-cha-Bta-Phe-NH₂ | 1041,7 | E |
| **120** | Ac-Phe-Nle-Pro-cha-Bta-Phe-NH₂ | 920,5 | E |
| **121** | Ac-Phe-Har-Pro-cha-Bta-Phe-NH₂ | 978,0 | D |
| **122** | Ac-Phe-Arg-Pro-cha-Bta-Phe-NH₂ | 964,0 | D |
| **123** | Ac-Phe-Cys(Acm)-Pro-cha-Bta-Phe-NH₂ | 981,5 | F |
| **124** | Ac-Phe-Mpa-Pro-cha-Bta-Phe-NH₂ | 955,7 | E |
| **125** | Ac-Eby-Orn-Pro-cha-Bta-Phe-NH₂ | 921,7 | D |
| **126** | Ac-Phg-Orn-Pro-cha-Bta-Phe-NH₂ | 907,8 | E |
| **127** | Ac-Phe-Paf-Pro-cha-Bta-Phe-NH₂ | 969,6 | F |
| **128** | H₂N-CO-Phe-Orn-Pro-cha-Bta-Phe-NH₂ | 922,8 | D |
| **129** | Me-O-CO-Phe-Orn-Pro-cha-Bta-Phe-NH₂ | 937,8 | E |
| **130** | (-CO-CH₂-NH-CO-)-Phe-Orn-Pro-cha-Bta-Phe-NH₂ | 962,9 | E |
| **132** | Ac-Phe-Orn-Pro-hch-Trp-Phe-OH | 919,8 | E |
| **133** | (-CO-CH₂-CH₂-CO-)-Phe-Orn-Pro-cha-Bta-Phe-NH₂ | 961,9 | F |
| **134** | ^{t}Bu-CO-Phe-Orn-Pro-cha-Bta-Phe-NH₂ | 963,9 | E |
| **135** | Ac-Lys-Phe-Orn-Aze-cha-Bta-Phe-NH₂ | 1036,0 | C |
| **136** | Ac-Gly-Phe-Orn-Aze-cha-Bta-Phe-NH₂ | 965,0 | D |
| **137** | Ac-Arg-Phe-Orn-Aze-cha-Bta-Phe-NH₂ | 1064,1 | D |
| **138** | Ac-His-Phe-Orn-Aze-cha-Bta-Phe-NH₂ | 1045,0 | E |
| **139** | Ac-Ser-Phe-Orn-Aze-cha-Bta-Phe-NH₂ | 995,0 | E |
| **140** | Ac-Guf-Phe-Orn-Aze-cha-Bta-Phe-NH₂ | 1112,1 | E |
| **141** | Ac-Dab-Phe-Orn-Aze-cha-Bta-Phe-NH₂ | 1008,0 | E |
| **142** | FH₂C-CO-Phe-Orn-Pro-cha-Bta-Phe-NH₂ | 939,8 | D |
| **143** | Ac-Phe-Orn(Et₂)-Pro-cha-Trp-Phe-NH₂ | 960,9 | E |
| **144** | Ac-Phe-[Orn-Hyp-cha-Trp-Nle] | 868,6 | C |
| **145** | 3PP-[Orn-Hyp-cha-Trp-Nle] | 811,6 | D |
| **146** | Ac-Phe-[Orn-Pro-cha-Trp-Tyr] | 902,7 | D |
| **147** | Ac-Phe-[Orn-Pro-omf Trp-Nle] | 860,6 | C |
| **148** | Ac-Phe-N(ⁿBu)-CH₂-CO-Pro-cha-Trp-Phe-NH₂ | 920,8 | F |
| **149** | Ac-Phe-Orn-Pro-hle-Bta-Phe-NH₂ | 895,4 | C |
| **150** | Ac-Phe-Arg(CH₂-CH₂)-Pro-cha-Bta-Phe-NH₂ | 990,1 | B |

### Beispiel 12: Bestimmung des EC₅₀-Wertes in einem Enzymfreisetzungsassay

Die Bestimmung des EC₅₀-Wertes verläuft vergleichbar mit dem in Beispiel 11 beschriebenen Vorgehen. Einziger Unterschied ist, dass 30 µl der zu testenden Substanzen mit 75 µl der unter Beispiel 11 beschriebenen Zellsuspension gemischt werden. Es erfolgt keine Vorinkubation und keine Zugabe von C5a zur Stimulation der Enzymfreisetzung. Die Ergebnisse für die getesteten Verbindungen sind in Tabelle 5 wiedergegeben.

**Tabelle 5:**

| Daten zur agonistischen Aktivität repräsentativer erfindungsgemäßer Verbindungen | | |
|---|---|---|
| **Nr.** | **Verbindung** | **EC**_{**50**} **(nM)** |
| - | hrC5a | 2,4 |
| 3 | HOCH2(CHOH)4-C=N-O-CH2-CO-Phe[OP-dCha-W-Nle] | »1430 |
| 41 | Ph-CH₂-CH₂-CO-[Orn-Pro-cha-Trp-Nle] | »1430 |
| 2 | Ac-Phe-[Orn-Hyp-cha-Trp-Phe] | »1430 |
| 42 | H-Phe-[Orn-Pro-cha-Trp-Nle] | »1430 |
| 1 | Ac-Phe-[Orn-Pro-cha-Trp-Phe] | »1430 |
| 43 | Ac-Lys-Phe-[OP-dCha-W-Nle] | »1430 |
| **28** | H-Phe-[Orn-Pro-cha-Trp-Nle] | »1430 |
| **44** | H-Phe-[Orn-Ser-cha-Trp-Nle] | »1430 |
| **33** | Ac-Phe-[Orn-Pro-cha-Trp-Eaf] | »1430 |
| **61** | Ac-Phe-Orn-Pro-cha-Trp-Phe-NH₂ | > 100000 |
| **62** | Ac-Phe-Orn-Pro-cha-Bta-Phe-NH₂ | >100000 |
| **71** | Ac-Ebw-Orn-Pro-cha-Trp-Phe-NH₂ | >100000 |
| **88** | Ac-Phe-Arg-Pro-cha-Trp-Phe-NH₂ | > 100000 |
| **55** | Ac-Phe-Orn-Pip-cha-Trp-Phe-NH₂ | > 100000 |
| **83** | Ac-Phe-Orn-Aze-cha-Trp-Phe-NH₂ | > 100000 |
| **84** | Ac-Phe-Trp-Pro-cha-Trp-Phe-NH₂ | > 100000 |
| **67** | Ac-Thi-Orn-Pip-cha-Bta-Phe-NH₂ | >100000 |

### Beispiel 13: Bestimmung der Löslichkeit von ausgewählten C5aR-Antagonisten

Die Löslichkeit von Verbindungen wurde folgendermaßen bestimmt: 20 µl einer 10 mM Stammlösung (in DMSO) der Verbindung werden in 980 µl des zu untersuchenden Lösungsmittels verdünnt. Nach 24 h Inkubation bei RT unter Schütteln werden die Proben bei 11.000 rpm in einer Eppendorfzentrifuge zentrifugiert. Der Überstand wird photometrisch quantifiziert. Die optische Dichte der Probe und eines Kontrollwertes in 60% MeOH dient als Maß der Löslichkeit. Substanzen, die sich ähnlich gut in dem zu untersuchenden Lösungsmittel wie in der Kontrolle lösen, werden wie folgt auf ihre maximale Löslichkeit untersucht. Dazu werden 10 mg/ml Verbindung in den Lösungsmitteln der Wahl gelöst. Der nicht gelöste Anteil wird nach 24 h durch Zentrifugation (s.o.) abgetrennt. Der Überstand wird photometrisch gemessen und auf einen entsprechenden Kontrollwert (60% MeOH) bezogen. Die Löslichkeit für einige der erfindungsgemäßen Verbindungen sind in Tabelle 6 angegeben.

**Tabelle 6:**

| Löslichkeit repräsentativer Vertreter erfindungsgemäßer Verbindungen | | |
|---|---|---|
| **Nr.** | **Verbindung** | **Löslichkeit in 20 mM HEPES pH 7.4 (% von 200 µM)** |
| **1** | **Ac-Phe-[Orn-Pro-cha-Trp-Phe]** | 8 |
| **2** | **Ac-Phe-[Orn-Hyp-cha-Trp-Phe]** | 13 |
| **28** | **Ac-Phe-[Orn-Pro-cha-Trp-Nle]** | 22 |
| **42** | **H-Phe-[Orn-Pro-cha-Trp-Phe]** | 45 |
| **4** | **X-Phe-[Orn-Pro-cha -Trp-Ne]; X = 2- Acetamido-1-Methyl-Glucuronyl** | 84 |
| **40** | **Ac-Phe-[Orn-Pro-cha-Trp-Arg]** | 94 |
| **43** | **Ac-Lys-Phe-[Orn-Pro-cha-Trp-Nle]** | 93 |

### Beispiel 14: Entwicklung des den Antagonisten zugrundeliegenden Pharmakophor-Modelles

Ein Austausch des Arginins in der Verbindung 40 mit Alanin (39) unterstreicht die Bedeutung der Seitenkette an dieser Position für die Wirksamkeit des Peptids als Inhibitor. Ersetzt man Arginin durch die positiv geladene Aminosäure Lysin 22 so findet man überraschenderweise einen sehr deutlichen Anstieg des IC₅₀-Wertes (von 20 nM auf 8700 nM). Dies bedeutet, dass die positive Ladung für die Antagonistische Wirkung allein nicht ausreichend ist. Verwendet man dagegen C-terminal die Aminosäure 4-Aminophenylalanin (Paf) **14,** findet man einen IC₅₀-Wert von 30 nM. Die Aminogruppe von Paf hat einen ähnlichen Abstand vom Cα-Atom wie die des Lysins. Erfolgt nun ein Austausch der Aminosäure Arginin aus der Verbindung **40** gegen das ungeladene und sehr hydrophobe Phenylalanin, erhält man die Verbindung **1,** die überraschenderweise einem der Verbindung **40** vergleichbaren IC₅₀-Wert von 23 nM zeigt. Damit ist offensichtlich, dass überraschenderweise nicht die positiv geladene Seitenkette des Arginins bzw. von Paf die entscheidende Wechselwirkung mit dem C5aR eingeht, sondern der hydrophobe Bereich von Paf oder Phe bzw. der aliphatischen Seitenkette des Arginins hierfür verantwortlich ist. Es ist möglich, weitere hydrophobe Substitutionen des Arginins durchzuführen, ohne dabei eine deutliche Erhöhung des IC₅₀- Wertes gegenüber der Verbindung **40** in kauf nehmen zu müssen. Beispiele für derartige Substitutionen sind unter anderem die Verbindungen (**1, 28, 29, 31, 32, 33, 34, 35, 36, 37, 38**).

Der Austausch weiterer Aminosäuren in **40** durch Ala, N-Me-Ala oder d-Ala zeigte, daß die Seitenketten der folgenden Aminosäuren wichtig für die antagonistische Wirkung sind: Phe, cha, Trp.

Aufbauend auf den Struktur-Aktivitäts-Beziehungen dieser und weiterer Peptide wurde ein Pharmakophor-Modell entwickelt. Hierbei sagte man den Abstand der für die Aktivität kritischen Gruppen (2 hydrophobe und zwei aromatische Gruppen) nach folgender Methode vorher:

Das Pharmakophor-Modell wurde auf der Grundlage einer 2 ns langen Moleküldynamik-Simulation (Schrittweite 2 fs) der Verbindung **28** erstellt. Die Simulation erfolgte unter Verwendung des AMBER94-Kraftfeldes sowie eines expliziten Wassermodells (TIP3) und periodischen Randbedingungen. Die statistische Analyse der Snapshots aus der letzten Nanosekunde der Trajektorie (1000 Strukturen) führte auf die unten angegebenen Abstände zwischen den Massenschwerpunkten der pharmakophoren Gruppen.

Die Startstruktur für die Moleküldynamik-Simulation resultiert aus Ensemble-Dynamik-Berechnungen mit sieben sich gegenseitig strukturell einschränkenden, im unteren nanomolaren Bereich (IC₅₀) aktiven zyklischen Peptiden.

### Beispiel 15: Bestimmung der AB-Permeabilität in einem TC-7 basierten Assaysystem

Zu testende Verbindungen werden auf eine Konzentration von 50 µM in HBSS-MES (5 mM, pH 6.5) eingestellt (aus einer 10 mM Stammlösung in 100% DMSO). ¹⁴ C-Mannitol (ca. 4 µM) wird zu der Probe gemischt. Diese Lösung wird anschließend zentrifugiert und der Überstand wird zur apikalen Seite einer TC-7 Zellkultur gegeben (Passage 15, in 24 Well Transwell Platte) so dass sich eine DMSO-Konzentration von 1% einstellt. Auf der basolatheralen Seite befindet sich HBSS-HEPES (5mM, pH 7.4). Anschließend werden die Zellen 120 min bei 37°C inkubiert. Die Integrität der TC-7 Zellschicht wird über das zugegebene Mannitol geprüft (Pₐₚₚ <2.5 10⁻⁶ cm/s). Die Permeabilität Pₐₚₚ [cm/s] ist gleich (V_{R}xC_{R120})/(ΔtxAx(C_{D,mid}-C_{R,mid})) wobei V_{R} das Volumen der Aufnahmekammer (engl. receiver chamber), C_{R120} die Konzentration der Testverbindung in der Aufnahmekammer nach 120 min, Δt die Inkubationszeit, A die Fläche der TC-7 Zellschicht, C_{D,mid} die midpoint Konzentration der Testsubstanz in der donor chamber und C_{R,mid} die Konzentration der Testverbindung in der receiver chamber ist.

| **Verbindung** | **AB-Permebilität [cm/s]** |
|---|---|
| **Ac-Phe[Orn-Pro-cha-Trp-Arg]** | 0.52 |
| **Ac-Phe[Orn-Hyp-cha-Trp-Phe]** | 14.25 |

### Beispiel 16: Synthese von Ac-Phe-Orn-Pro-cha-Trp-Phe-NH₂ (51)

Nach linearer Peptidsynthese gemäß AAV 1 und anschließender Reinigung per HPLC erhielt man 10,0 mg des gewünschten Produkts **51** als weißen Feststoff.
MS (ESI): m/z = 904,5 [(M+H)⁺].

### Beispiel 17: Synthese von Ac-Phe-Orn-Aze-cha-Bta-Phe-NH₂ (52)

Das lineare Peptid **52** wurde nach linearer Peptidsynthese gemäß AAV 1 hergestellt und per HPLC gereinigt. Man erhielt 10,5 mg der Verbindung **52** als weißen Feststoff.
MS (ESI): m/z = 907,5 [(M+H)⁺].

### Beispiel 18: Synthese von Ac-Phe-Orn-Pro-cha-Trp-NH-CH₂-CH₂-Ph (72)

200 mg Brom-(4-methoxyphenyl)methylpolystyrolharz wird mit 5 ml einer 50%igen Lösung von Phenylethylamin in THF (v/v) bei Raumtemperatur für 18 h inkubiert. Anschliessend wird das Harz gewaschen (DMF; 3 x 5,0 ml, MeOH; 3 x 5,0 ml, DCM; 3 x 5,0 ml) und das Peptid nach AAV 1 synthetisert. Nach HPLC-Reinigung erhält man 4,1 der Verbindung 72 als weißen Feststoff.
MS (ESI): m/z = 861,8 [(M+H)⁺].

### Beispiel 19: Synthese von Ac-Phe-Orn-Aze-cha-Bta-Phe-NH-Me (95)

4,5 g 4-(4-Formyl-3-methoxy-phenoxy)-butansäure-Polystyrol-Harz wurde für 15 min in THF aufgeschwämmt. Man filtrierte und setzte das Harz um mit einem Gemisch aus 3,04 g (10 eq.) Methylamin-Hydrochlorid, 2,7 ml Essigsäure, 2,7 ml Trimethylorthoformiat und 90 ml THF. Nach einstündigem Rühren wurden 45 ml DMF und 2,83 g (10 eq.) Natriumcyanoborhydrid zugesetzt. Man rührte über Nacht bei Raumtemperatur, filtrierte und wusch das Harz mit DMF (5x), MeOH (5x) und CH₂Cl₂ (5x). Anschließend führte man eine Aminosäurekupplung mit 968 mg (5 eq.) Fmoc-Phe-OH, 950 mg (5 eq.) HATU und 3,75 ml DIPEA in 10 ml DMF für 2 Stunden durch. Man filtrierte und wusch mit DMF (5x), MeOH (5x) und CH₂Cl₂ (5x). Mit 200 mg des erhaltenen Harzes führte man eine lineare Peptidsynthese nach AAV 1 durch und nach anschließender Reinigung per HPLC erhielt man 10,0 mg des gewünschten Produktes 95 als weißen Feststoff.
MS (ESI): m/z = 921,6 [(M+H)⁺].

### Beispiel 20: Synthese von CH₃-SO₂-Phe-Orn-Aze-cha-Bta-Phe-NH₂ (96)

Nach linearer Peptidsynthese gemäß AAV 1, bei der CH₃-SO₂-Cl statt einer N-terminalen Aminosäure eingesetzt wurde, und anschließender Reinigung per HPLC erhielt man 5,5 mg des gewünschten Produktes 96 als weißen Feststoff.
MS (ESI): m/z = 943,9 [(M+H)⁺].

### Beispiel 21: Synthese von H₂N-CO-Phe-Orn-Pro-cha-Bta-Phe-NH₂ (128)

Das harzgebundene Peptid H-Phe-Orn-Pro-cha-Bta-Phe-Rink-Amid-Harz wurde gemäß AAV 1 hergestellt. Anschließend setzte man Diphenylmethylisocyanat (5 eq.) sowie DIPEA (10 eq.) in DMF zu und agitierte für 2 Stunden. Nach Abspaltung vom Harz mit einem Gemisch aus 95 % TFA, 2,5 % Wasser und 2,5 %TIPS wurde per HPLC gereinigt. Man erhielt 0,92 mg der Verbindung als weißen Feststoff.
MS (ESI): m/z = 922,8 [(M+H)⁺].

### Beispiel 22: Synthese von (-CO-CH₂-NH-CO-)-Phe-Orn-Pro-cha-Bta-Phe-NH₂ (130)

Das harzgebundene Peptid H-Gly-Phe-Orn-Pro-cha-Bta-Phe-Rink-Amid-Harz wurde gemäß AAV 1 hergestellt. Anschließend setzte man Disuccinimidylcarbonat (3 eq.) und DIPEA (3 eq.) in DMF zu und agitierte für 3 Stunden. Anschließend wurden weitere 3 eq. DIPEA zugegeben und man agitierte zusätzliche 5 Stunden bei Raumtemperatur. Nach Abspaltung vom Harz mit einem Gemisch aus 95 % TFA, 2,5 % Wasser und 2,5 %TIPS wurde per HPLC gereinigt. Man erhielt 3,8 mg der Verbindung als weißen Feststoff.
MS (ESI): m/z = 962,9 [(M+H)⁺].

### Beispiel 23 : Synthese von (-CO-CH₂-CH₂-CO-)-Phe-Orn-Pro-cha-Bta-Phe-NH₂ (133)

Das harzgebundene Peptid H-Phe-Orn-Pro-cha-Bta-Phe-Rink-Amid-Harz wurde gemäß AAV 1 hergestellt. Anschließend setzte man Succinanhydrid (5 eq.) sowie DIPEA (10 eq.) in DMF zu und agitierte für 2 Stunden. Man filtrierte und wusch mit DMF (5x), MeOH (5x) und CH₂Cl₂ (5x). Anschließend wurde das Harz mit HBTU (5 eq.) und DIPEA (10 eq.) in DMF für 1 Tag umgesetzt. Man spaltete das Peptid mit einem Gemisch aus 95 % TFA, 2,5 % Wasser und 2,5 %TIPS vom Harz ab und reinigte per HPLC, wodurch man 0,47 mg der Verbindung als weißen Feststoff erhielt.
MS (ESI): m/z = 961,9 [(M+H)⁺].

### Beispiel 24: Synthese von FH₂C-CO-Phe-Orn-Pro-cha-Bta-Phe-NH₂ (142)

Nach linearer Peptidsynthese gemäß AAV 1, bei der Fluoressigsäure statt einer N-terminalen Aminosäure eingesetzt wurde, und anschließender Reinigung per HPLC erhielt man 0,90 mg des gewünschten Produktes 142 als weißen Feststoff.
MS (ESI): m/z = 939,8 [(M+H)⁺].

### Beispiel 25: Synthese von Ac-Phe-Orn(Et₂)-Pro-cha-Trp-Phe-NH₂ (143)

Nach linearer Peptidsynthese gemäß AAV 1 und anschließender Reinigung per HPLC erhielt man 10,0 mg der Verbindung **51**. 5,0 mg dieser Verbindung wurden in 5 ml THF gelöst und mit 1 ml Acetaldehyd versetzt. Nach Zugabe von 100 mg (Polystyrolmethyl)trimethylammoniumcyanoborhydrd (3 mmol/g) wurde 12 h bei Raumtemperatur langsam gerührt, anschliessend das Harz abfiltriert und zur Trockene eingeengt. Nach HPLC-Reinigung erhielt man 1,2 mg des gewünschten Produktes **143.**
MS (ESI): m/z = 960,9 [(M+H)⁺].

### Beispiel 26: Synthese von Ac-Phe-N(ⁿBu)-CH₂-CO-Pro-cha-Trp-Phe-NH₂ (144)

Die Synthese des Peptids H-Pro-cha-Trp-Phe-Rink-Amid-Harz erfolgte nach AAV 1. Die freie Aminogruppe wurde mit 4 ml einer 0,4 M Lösung von Bromessigsäureanhydrid in DCM acyliert (2x 15 min). Das Harz wurde gewaschen (DMF; 3 x 5,0 ml, MeOH; 3 x 5,0 ml, DCM; 3 x 5,0 ml) und für 2 x 30 min mit 4 ml einer 5 M Lösung von ⁿButylamin versetzt. Nach Waschen des Harzes (DMF; 3 x 5,0 ml, MeOH; 3 x 5,0 ml, DCM; 3 x 5,0 ml) erfolgte die restliche Synthese des Peptomers nach AAV1.

### Beispiel 27: Synthese von Ac-Phe-Arg(CH₂CH₂)-Pro-cha-Bta-Phe-NH₂ (150)

Nach linearer Peptidsynthese gemäß AAV 1 erhielt man 700 mg der Verbindung Ac-Phe-Om-Pro-cha-Bta-Phe-NH₂ (**62**) als Rohprodukt. 15 mg dieses Rohproduktes (0,016 mmol) wurde mit 39,7 mg (10 eq.) 2-Methylthio-2-imidazolin-Hydroiodid und 55,4 *µ*l (20 eq.) DIPEA in 1 ml MeCN versetzt und für einen Tag bei 40 °C gerührt. Nach Entfernen des Lösungsmittels am Rotationsverdampfer reinigte man per HPLC, lyophilisierte nach Zusatz von 1 ml 0,1 N HCl und 0,5 ml MeCN und erhielt 0,7 mg der Verbindung 150 als weißen Feststoff.
MS (ESI): m/z = 960,9 [(M+H)⁺].

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. Verbindung, bevorzugterweise ein C5a-Rezeptor-Antagonist, mit der folgenden Struktur: 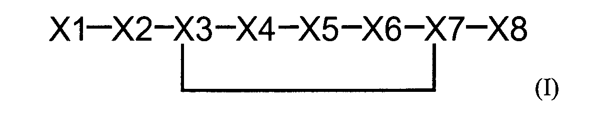 , wobei
X1 ein Radikal mit einer Masse von etwa 1-300 ist und wobei X1 bevorzugterweise ausgewählt ist aus der Gruppe, die R5-, R5-CO-, R5-N(R6)-CO-, R5-O-CO-, R5-SO₂-, R5-N(R6)-SO₂-, R5-N(R6)-, R5-N(R6)-CS-, R5-N(R6)-C(NH)-, R5-CS-, R5-P(O)OH-, R5-B(OH)-, R5-CH=N-O-CH₂-CO- umfasst, wobei R5 und R6 einzeln und unabhängig voneinander ausgewählt sind aus der Gruppe, die H, F, Hydroxy, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, Acyl, substituiertes Acyl, Alkoxy, Alkoxyalkyl, substituiertes Alkoxyalkyl, Aryloxyalkyl und substituiertes Aryloxyalkyl umfasst,
X2 ein Radikal ist, das die biologischen Bindungseigenschaften einer Phenylalanin-Einheit mimikt,
X3 und X4 einzeln und unabhängig voneinander ein Spacer ist, wobei der Spacer bevorzugterweise aus der Gruppe ausgewählt ist, die Aminosäuren, Aminosäure-Analoga und Aminosäure-Derivate umfasst,
X5 ein Radikal ist, das die biologischen Bindungseigenschaften einer Cyclohexylalanin-Einheit mimikt,
X6 ein Radikal ist, das die biologischen Bindungseigenschaften einer Tryptophan-Einheit mimikt,
X7 ein Radikal ist, das die biologischen Bindungseigenschaften einer Norleucin- oder Phenylalanin-Einheit mimikt,
X8 ein Radikal ist, wobei das Radikal optional in Struktur I enthalten ist und wenn es enthalten ist, ausgewählt ist aus der Gruppe, die H, NH₂, OH, NH-OH, Amino, substituiertes Amino, Alkoxy, substituiertes Alkoxy, Hydrazino, substituiertes Hydrazino, Aminooxy, substituiertes Aminooxy, Alkyl, substituiertes Alkyl, Cycloalkyl, substituiertes Cycloalkyl, Heterocyclyl, substituiertes Heterocyclyl, Heteroaryl, substituiertes Heteroaryl, Arylalkyl, substituiertes Arylalkyl, Aryl, substituiertes Aryl, Aminosäure, Aminosäurederivat und Aminosäureanalogon umfasst,
eine chemische Bindung zwischen X3 und X7 ausgebildet ist, und
die Verbindungslinien - in Formel (I) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

2. Verbindung nach Anspruch 1, **dadurch gekennzeichnet, dass** X3 und X7 jeweils eine Aminosäure, ein Aminosäurederivat oder ein Aminosäureanalogon ist, wobei die chemische Bindung zwischen X3 und X7 unter Beteiligung von Molekülteilen von X3 und X7 ausgebildet ist, und die Molekülteile für X3 und X7 einzeln und unabhängig voneinander ausgewählt sind aus der Gruppe, die den C-Terminus, den N-Terminus und die jeweilige Seitenkette der Aminosäure umfasst.

3. Verbindung nach Anspruch 1 oder 2, wobei
X1 ein Radikal mit einer Masse von etwa 1-300 ist, wobei das Radikal bevorzugterweise ausgewählt ist aus der Gruppe, die R5, R5-CO-, R5-N(R6)-CO-, R5-O-CO-, R5-SO₂-, R5-N(R6)-C(NH)-, umfasst, wobei bevorzugtererweise R5 und R6 einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl und substituiertes Aryl enthält;
X2 und X6 jeweils und unabhängig voneinander eine aromatische Aminosäure, ein Derivat oder ein Analogon davon ist;
X5 und X7 einzeln und unabhängig voneinander eine hydrophobe Aminosäure, ein Derivat oder ein Analogon davon sind.

4. Verbindung nach Anspruch 3, **dadurch gekennzeichnet, dass** X1 ausgewählt ist aus der Gruppe, die H, Acetyl, Propanoyl, Butanoyl, Benzoyl, Fluormethylcarbonyl, Difluormethylcarbonyl, Phenyl, Oxycarbonyl, Methyl-oxycarbonyl, Phenyl-aminocarbonyl, Methyl-aminocarbonyl, Phenyl-sulfonyl und Methyl-sulfonyl umfasst.

5. Verbindung nach einem der Ansprüche 1 bis 4, wobei X2, X5, X6 und X7 einzeln und unabhängig voneinander die folgende Struktur aufweisen: 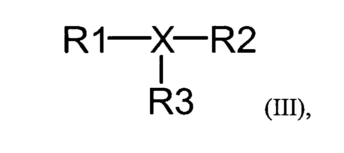 worin
X C(R4) oder N ist,
R1 optional vorhanden ist und wenn R1 vorhanden ist, R1 ein Radikal ist, das ausgewählt ist aus der Gruppe, die >N-R1B, >C(R1B)(R1C) und >O umfasst, wobei R1B und R1C einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, S=O, C=NH, C=N-CN, PO(OH), B(OH), CH₂, CH₂CO, CHF und CF₂ umfasst;
R4 ein Radikal ist, wobei das Radikal ausgewählt ist aus der Gruppe, die H, F, CH₃, CF₃, Alkyl und substituiertes Alkyl umfasst;
und die Bindung von Struktur (III) an die Molekülbestandteile X1 und X3, X4 und X6, X5 und X7, und X6 und X8 bevorzugt über R1 und R2 erfolgt;
für X2 und für X6 einzeln und unabhängig voneinander R3 ein Radikal ist, wobei das Radikal eine aromatische Gruppe enthält und ausgewählt ist aus der Gruppe, die Aryl, substituiertes Aryl, Heteroaryl, substituiertes Heteroaryl, Arylalkyl, substituiertes Arylalkyl, Heteroarylalkyl, substituiertes Heteroarylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst; und
für X5 und für X7 einzeln und unabhängig voneinander R3 ein Radikal ist, wobei das Radikal eine aliphatische oder aromatische Gruppe enthält und bevorzugterweise ausgewählt ist aus der Gruppe, die Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Cycloalkylalkyl, substituiertes Cycloalkylalkyl, Heterocyclylalkyl, substituiertes Heterocyclylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst.

6. Verbindung nach Anspruch 5, **dadurch gekennzeichnet, dass** unter Beteiligung von R3 und R4 ein Ring ausgebildet wird.

7. Verbindung nach Anspruch 5 oder 6, **dadurch gekennzeichnet, dass** für X2 und für X6 einzeln und unabhängig voneinander R3 aus der Gruppe ausgewählt ist, die Phenyl, substituiertes Phenyl, Benzyl, substituiertes Benzyl, 1,1-Diphenylmethyl, substituiertes 1,1-Diphenylmethyl, Naphthylmethyl, substituiertes Naphthylmethyl, Thienylmethyl, substituiertes Thienylmethyl, Benzothienylmethyl, substituiertes Benzothienylmethyl, Imidazolylmethyl, substituiertes Imidazolylmethyl, Indolylmethyl und substituiertes Indolylmethyl umfasst.

8. Verbindung nach einem der Ansprüche 5 bis 7, **dadurch gekennzeichnet, dass** für X5 und für X7 einzeln und unabhängig voneinander R3 aus der Gruppe ausgewählt ist, die C3-C5-Alkyl, substituiertes C3-C5-Alkyl, C5-C7-Cycloalkyl, substituiertes C5-C7-Cycloalkyl, C5-C7-Cycloalkylmethyl, substituiertes C5-C7-Cycloalkylmethyl, Cycloalkylethyl, substituiertes Cycloalkylethyl, Benzyl, substituiertes Benzyl, Phenylethyl, Naphthylmethyl, Thienylmethyl, Propenyl, Propinyl, Methylthioethyl, Imidazolylmethyl, substituiertes Imidazolylmethyl, Indolylmethyl und substituiertes Indolylmethyl umfasst.

9. Eine Verbindung nach einem der Ansprüche 1 bis 8, wobei
X2 ein Aminosäurederivat einer Aminosäure ist, das ausgewählt ist aus der Gruppe, die Phenylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine, 1-Naphtylalanin, 2-Thienylalanin, 3-Thienylalanin, 3,3-Diphenylalanin, Tyrosin, Tryptophan, Histidin und jeweilige Derivate davon umfasst;
oder X2 und X1 sind zusammengenommen PhCH₂CH₂CO- oder PhCH₂-;
X6 ein Aminosäurederivat einer Aminosäure ist, die ausgewählt ist aus der Gruppe, die Tryptophan, Phenylalanin, Tyrosin, Histidin, 1-Naphtylalanin, Benzothienylalanin, 2-Aminoindan-2-carbonsäure, 2-Thienylalanin, 3-Thienylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine und jeweilige Derivate davon umfasst;
X5 ein Aminosäurederivat einer Aminosäure ist, die ausgewählt ist aus der Gruppe, die D-Cyclohexylalanin, D-Cyclohexylglycin, D-Homo-Cyclohexylalanin, Octahydroindol-2-carbonsäure, 2-Methyl-D-Phenylalanin und jeweilige Derivate davon umfasst; und
X7 ein Aminosäurederivat einer Aminosäure ist, die ausgewählt ist aus der Gruppe, die Norvalin, Norleucin, Homo-Leucin, Leucin, Isoleucin, Valin, Cystein, Cystein(Me), Cystein(Et), Cystein(Pr), Methionin, Allylglycin, Propargylglycin, Cyclohexylglycin, Cyclohexylalanin, Phenylalanin, Tyrosin, Tryptophan, Histidin, 1-Naphtylalanin, 2-Thienylalanin, 3-Thienylalanin und jeweilige Derivate davon umfasst.

10. Verbindung nach einem der Ansprüche 1 bis 9, wobei X1 und/oder X4 eine oder mehrere die Wasserlöslichkeit verbessernde Gruppen aufweisen, wobei die die Wasserlöslichkeit verbessernde Gruppe ausgewählt ist aus der Gruppe, die Hydroxy, Keto, Carboxamido, Ether, Harnstoff, Carbamat, Amino, substituiertes Amino, Guanidino, Pyridyl und Carboxyl umfasst.

11. Verbindung, bevorzugterweise ein C5a-Rezeptor-Antagonist, mit der Struktur 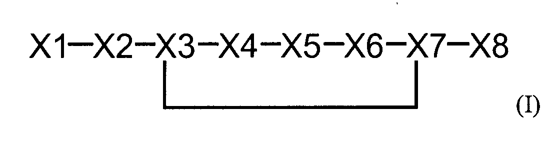 , wobei X1-X3 und X5-X8 definiert sind, wie in einem der Ansprüche 1 bis 10 und wobei
X4 eine zyklische oder eine nichtzyklische Aminosäure ist, wobei die zyklische Aminosäure ausgewählt ist aus der Gruppe, die Prolin, Pipecolinsäure, Azetidin-2-carbonsäure, Tetrahydroisochinolin-3-carbonsäure, Tetrahydroisochinolin-1-carbonsäure, Octahydroindol-2-carbonsäure, 1-Aza-bicyclo-[3.3.0]-octan-2-carbonsäure, 4-Phenyl-pyrrolidin-2-carbonsäure, cis-Hyp und trans-Hyp umfasst, und die nichtzyklische Aminosäure ausgewählt aus der Gruppe, die Ser, Gln, Asn, Cys(O₂CH₂CH₂CONH₂), Arg, Hyp(COCH₂OCH₂CH₂OCH₂CH₂OCH₃), Hyp(CONH-CH₂CH(OH)-CH₂OH) und jeweilige Derivate davon und jeweilige Analoga davon umfasst; und
die Verbindungslinien - in Formel (I) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

12. Verbindung nach Anspruch 11, **dadurch gekennzeichnet, dass** die Aminosäuren bevorzugt ausgewählt sind aus der Gruppe, die Prolin, Pipecolinsäure, Azetidin-2-carbonsäure, Tetrahydroisochinolin-3-carbonsäure, Tetrahydroisochinolin-1-carbonsäure, Octahydroindol-2-carbonsäure, 1-Aza-bicyclo-[3.3.0]-octan-2-carbonsäure, 4-Phenylpyrrolidin-2-carbonsäure, Hyp, Ser, Gln, Asn, Cys(O₂CH₂CH₂CONH₂) und Arg umfasst.

13. Verbindung, bevorzugterweise ein C5a-Rezeptor-Antagonist, mit der Struktur 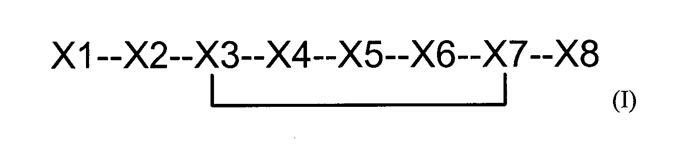 , wobei X1-X2 und X4-X8 definiert sind, wie in einem der Ansprüche 1 bis 12 und wobei
X3 folgende Struktur aufweist 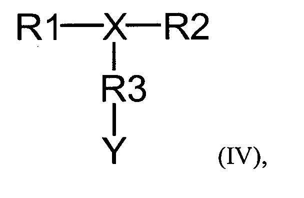 worin
X C(R4) oder N ist,
R1 optional vorhanden ist und wenn R1 vorhanden ist, Rlein Radikal ist, das ausgewählt ist aus der Gruppe, die >N-R1B, >C(R1B)(R1C) und >O umfasst, wobei R1B und R1C einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, PO(OH), B(OH), CH₂, CH₂CO, CHF und CF₂ umfasst;
R4 ein Radikal ist, wobei das Radikal ausgewählt ist aus der Gruppe, die H, F, CF₃, Alkyl und substituiertes Alkyl umfasst;
die Bindung von Struktur (IV) an die Molekülbestandteile X2 und X4 bevorzugt über R1 und R2 erfolgt;
R3 ein Radikal ist, das ausgewählt ist aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Cycloalkylalkyl, substituiertes Cycloalkylalkyl, Heterocyclylalkyl, substituiertes Heterocyclylalkyl, Arylalkyl, substituiertes Arylalkyl, Heteroarylalkyl und substituiertes Heteroarylalkyl umfasst.
Y optional vorhanden ist und wenn Y vorhanden ist, Y ein Radikal ist, das ausgewählt ist aus der Gruppe, die -N(YB)-, -O-, -S-, -S-S-, -CO-, -C=N-O-, -CO-N(YB)- und 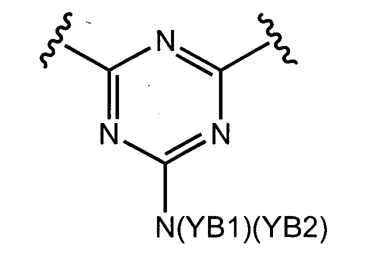 umfasst, wobei YB, YB1 und YB2 einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst.

14. Verbindung nach Anspruch 13, **dadurch gekennzeichnet, dass**
R3 ein Radikal ist, das ausgewählt ist aus der Gruppe, die Methyl, Ethyl, Propyl, Butyl, Benzyl und 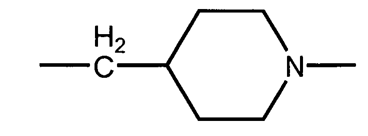 umfasst;
Y optional vorhanden ist und wenn Y vorhanden ist, Y ein Radikal ist, das ausgewählt ist aus der Gruppe, die -N(YB)-, -O-, -S- und -S-S- umfasst.

15. Verbindung, bevorzugterweise ein C5a-Rezeptor-Antagonist, wobei die Verbindung die folgende Struktur aufweist: 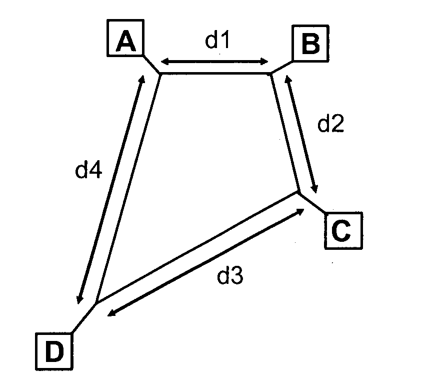 wobei d1, d2, d3 und d4 die Abstände von A, B, C und D in wenigstens einem energetisch zugänglichen Konformer der Verbindung bezeichnen und die folgenden Werte aufweisen:
d1 = 5.1 ± 1.0 Å
d2 = 11.5 ± 1.0 Å
d3 = 10.0 ± 1.5 Å
d4 = 6.9 ± 1.5 Å
A und C einzeln und unabhängig voneinander ein hydrophobes Radikal sind, wobei das hydrophobe Radikal ausgewählt ist aus der Gruppe, die Alkyl, Cycloalkyl, Heterocyclyl, Aryl und Heteroaryl umfasst;
B und D einzeln und unabhängig voneinander ein aromatisches oder heteroaromatisches Radikal sind, wobei bevorzugterweise das aromatische Radikal Aryl ist, und bevorzugterweise das heteroaromatische Radikal Heteroaryl ist.

16. Verbindung nach Anspruch 15, wobei A und C einzeln und unabhängig voneinander ausgewählt sind aus der Gruppe, die C3-C6-Alkyl, C5-C7-Cycloalkyl, Methylthioethyl, Indolyl, Phenyl, Naphtyl, Thienyl, Propenyl, Propinyl, Hydroxyphenyl, Indolyl und Imidazolyl umfasst;
B ausgewählt ist aus der Gruppe, die Phenyl, Naphthyl, Thienyl, Benzothienyl, Hydroxyphenyl, Indolyl, und Imidazolyl umfasst; und
D ausgewählt ist aus der Gruppe, die Phenyl, Naphthyl, Thienyl, Thiazolyl, Furanyl, Hydroxyphenyl, Indolyl und Imidazolyl umfasst.

17. Verbindung, bevorzugterweise ein C5a-Rezeptor-Antagonist,
mit folgender Struktur: 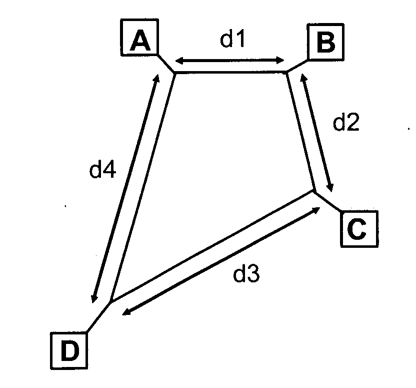 , wobei
A, B, C und D die C-alpha-Atome in Aminosäuren, Aminosäure-Analoga oder AminosäureDerivaten bezeichnen,
d1, d2, d3 und d4 die Abstände zwischen A, B, C und D in wenigstens einem energetisch zugänglichen Konformer der Verbindung bezeichnen und die folgenden Werte aufweisen:
d1 = 3,9 ± 0,5 Å
d2 = 3,9 ± 0,5 Å
d3 = 9,0 t 1,5 Å
d4 = 9,0 ± 1,5 Å;
A und C einzeln und unabhängig voneinander eine hydrophobe Aminosäure-Seitenkette sind, die eine Alkyl-, Cycloalkyl, Heterocyclyl, Aryl- oder Heteroaryl-Gruppe umfasst.
B und D einzeln und unabhängig voneinander eine aromatische oder heteroaromatische Aminosäure-Seitenkette sind, die eine Aryl- oder Heteroaryl-Gruppe umfasst.

18. Verbindung nach Anspruch 17, wobei
A ausgewählt ist aus der Gruppe, die C3-C6-Alkyl, Methylthioethyl, Propenyl, Propinyl, R5, Methyl-R5 und Ethyl-R5 umfasst, wobei R5 ein Radikal ist, das ausgewählt ist aus der Gruppe, die C5-C7-Cycloalkyl, Phenyl, substituiertes Phenyl, Hydroxyphenyl, Indolyl, Imidazolyl, Naphtyl und Thienyl umfasst;
B ausgewählt ist aus der Gruppe, die R5, Methyl-R5 und Ethyl-R5 umfasst, wobei R5 ausgewählt ist aus der Gruppe, die Phenyl, substituiertes Phenyl, Naphtyl, Thienyl, Benzothienyl, Hydroxyphenyl, Indolyl und Imidazolyl umfasst;
C ausgewählt ist aus der Gruppe, die C3-C6-Alkyl, R5, Methyl-R5 und Ethyl-R5 umfasst, wobei R5 ein Radikal ist, das aus der Gruppe ausgewählt ist, die C5-C7-Cycloalkyl, Phenyl, 1-Methyl-Phenyl, 2-Methyl-Phenyl und 3-Methyl-Phenyl umfasst; und
D ausgewählt ist aus der Gruppe, die R5, Methyl-R5 und Ethyl-R5 umfasst, wobei R5 ausgewählt ist aus der Gruppe, die Phenyl, Naphthyl, Thienyl, Thiazolyl, Furanyl, Hydroxyphenyl, Indolyl und Imidazolyl umfasst.

19. Verbindung, bevorzugterweise ein C5a-Rezeptor-Antagonist, mit der folgenden Struktur:
X1-X2-X3-X4-X5-X6-X7-X8 (II)
, wobei
X1 ein Radikal mit einer Masse von etwa 1-300 ist und wobei X1 bevorzugterweise ausgewählt ist aus der Gruppe, die R5-, R5-CO-, R5-N(R6)-CO-, R5-O-CO-, R5-SO₂-, R5-N(R6)-SO₂-, R5-N(R6)-, R5-N(R6)-CS-, R5-N(R6)-C(NH)-, R5-CS-, R5-P(O)OH-, R5-B(OH)-, R5-CH=N-O-CH₂-CO- umfasst, wobei R5 und R6 einzeln und unabhängig voneinander ausgewählt sind aus der Gruppe, die H, F, Hydroxy, Alkyl, substituiertes Alkyl, Cycloalkyl, substituiertes Cycloalkyl, Heterocyclyl, substituiertes Heterocyclyl, Arylalkyl, substituiertes Arylalkyl, Aryl, substituiertes Aryl, Heteroaryl, substituiertes Heteroaryl, Acyl, substituiertes Acyl, Alkoxy, Alkoxyalkyl, substituiertes Alkoxyalkyl, Aryloxyalkyl und substituiertes Aryloxyalkyl umfasst,
X2 ein Radikal ist, das die biologischen Bindungseigenschaften einer Phenylalanin-Einheit mimikt,
X3 und X4 einzeln und unabhängig voneinander ein Spacer ist, wobei der Spacer bevorzugterweise aus der Gruppe ausgewählt ist, die Aminosäuren, Aminosäure-Analoga und Aminosäure-Derivate umfasst,
X5 ein Radikal ist, das die biologischen Bindungseigenschaften einer Cyclohexylalanin-Einheit mimikt,
X6 ein Radikal ist, das die biologischen Bindungseigenschaften einer Tryptophan-Einheit mimikt,
X7 ein Radikal ist, das die biologischen Bindungseigenschaften einer Norleucin- oder Phenylalanin-Einheit mimikt,
X8 ein Radikal ist, wobei das Radikal optional in Struktur I enthalten ist und wenn es enthalten ist, ausgewählt ist aus der Gruppe, die H, NH₂, OH, NH-OH, Amino, substituiertes Amino, Alkoxy, substituiertes Alkoxy, Hydrazino, substituiertes Hydrazino, Aminooxy, substituiertes Aminooxy, Alkyl, substituiertes Alkyl, Cycloalkyl, substituiertes Cycloalkyl, Heterocyclyl, substituiertes Heterocyclyl, Heteroaryl, substituiertes Heteroaryl, Arylalkyl, substituiertes Arylalkyl, Aryl, substituiertes Aryl, Aminosäure, Aminosäurederivat und Aminosäureanalogon umfasst;
die Verbindungslinien - in Formel (II) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

20. Verbindung nach Anspruch 19, wobei
X1 ein Radikal mit einer Masse von etwa 1-300 ist, wobei das Radikal bevorzugterweise ausgewählt ist aus der Gruppe, die R5, R5-CO-, R5-N(R6)-CO-, R5-O-CO-, R5-SO₂-, R5-N(R6)-C(NH)-, umfasst, wobei bevorzugtererweise R5 und R6 einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl und substituiertes Aryl enthält;
X2 und X6 jeweils und unabhängig voneinander eine aromatische Aminosäure, ein Derivat oder ein Analogon davon ist;
X5 und X7 einzeln und unabhängig voneinander eine hydrophobe Aminosäure, ein Derivat oder ein Analogon davon sind.

21. Verbindung nach Anspruch 20, **dadurch gekennzeichnet, dass** X1 ausgewählt ist aus der Gruppe, die H, Acetyl, Propanoyl, Butanoyl, Benzoyl, Fluormethylcarbonyl, Difluormethylcarbonyl, Phenyl, Oxycarbonyl, Methyl-oxycarbonyl, Phenyl-aminocarbonyl, Methyl-aminocarbonyl, Phenyl-sulfonyl und Methyl-sulfonyl umfasst.

22. Verbindung nach einem der Ansprüche 19 bis 21, wobei X2, X5, X6 und X7 einzeln und unabhängig voneinander die folgende Struktur aufweisen: 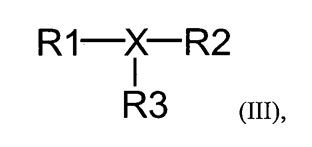 worin
X C(R4) oder N ist,
R1 ein Radikal ist, das ausgewählt ist aus der Gruppe, die >N-R1B, >C(R1B)(R1C) und >O umfasst, wobei R1B und R1C einzeln und unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, S=O, C=NH, C=N-CN, PO(OH), B(OH), CH₂, CH₂CO, CHF und CF₂ umfasst;
R4 ein Radikal ist, wobei das Radikal ausgewählt ist aus der Gruppe, die H, F, CH₃, CF₃, Alkyl und substituiertes Alkyl umfasst;
und die Bindung von Struktur (III) an die Molekülbestandteile X1 und X3, X4 und X6, X5 und X7, und X6 und X8 bevorzugt über R1 und R2 erfolgt;
für X2 und für X6 einzeln und unabhängig voneinander R3 ein Radikal ist, wobei das Radikal eine aromatische Gruppe enthält und ausgewählt ist aus der Gruppe, die Aryl, substituiertes Aryl, Heteroaryl, substituiertes Heteroaryl, Arylalkyl, substituiertes Arylalkyl, Heteroarylalkyl, substituiertes Heteroarylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst; und
für X5 und für X7 einzeln und unabhängig voneinander R3 ein Radikal ist, wobei das Radikal eine aliphatische oder aromatische Gruppe enthält und bevorzugterweise ausgewählt ist aus der Gruppe, die Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylalkyl, substituiertes Arylalkyl, Heteroarylalkyl, substituiertes Heteroarylalkyl, Cycloalkylalkyl, substituiertes Cycloalkylalkyl, Heterocyclylalkyl, substituiertes Heterocyclylalkyl, Alkyloxy-Alkyl, substituiertes Alkyloxy-Alkyl, Alkyloxy-Cycloalkyl, substituiertes Alkyloxy-Cycloalkyl, Alkyloxy-Heterocyclyl, substituiertes Alkyloxy-Heterocyclyl, Alkyloxy-Aryl, substituiertes Alkyloxy-Aryl, Alkyloxy-Heteroaryl, substituiertes Alkyloxy-Heteroaryl, Alkylthio-Alkyl, substituiertes Alkylthio-Alkyl, Alkylthio-Cycloalkyl und substituiertes Alkylthio-Cycloalkyl umfasst.

23. Verbindung nach Anspruch 22, **dadurch gekennzeichnet, dass** unter Beteiligung von R3 und R4 ein Ring ausgebildet wird.

24. Verbindung nach Anspruch 22 oder 23, **dadurch gekennzeichnet, dass** für X2 und für X6 einzeln und unabhängig voneinander R3 aus der Gruppe ausgewählt ist, die Phenyl, substituiertes Phenyl, Benzyl, substituiertes Benzyl, 1,1-Diphenylmethyl, substituiertes 1,1-Diphenylmethyl, Naphthylmethyl, substituiertes Naphthylmethyl, Thienylmethyl, substituiertes Thienylmethyl, Benzothienylmethyl, substituiertes Benzothienylmethyl, Imidazolylmethyl, substituiertes Imidazolylmethyl, Indolylmethyl und substituiertes Indolylmethyl umfasst.

25. Verbindung nach einem der Ansprüche 19 bis 24, **dadurch gekennzeichnet, dass** für X5 und für X7 einzeln und unabhängig voneinander R3 aus der Gruppe ausgewählt ist, die C3-C5-Alkyl, substituiertes C3-C5-Alkyl, C5-C7-Cycloalkyl, substituiertes C5-C7-Cycloalkyl, C5-C7-Cycloalkylmethyl, substituiertes C5-C7-Cycloalkylmethyl, Cycloalkylethyl, substituiertes Cycloalkylethyl, Benzyl, substituiertes Benzyl, Phenylethyl, Naphthylmethyl, Thienylmethyl, Propenyl, Propinyl, Methylthioethyl, Imidazolylmethyl, substituiertes Imidazolylmethyl, Indolylmethyl und substituiertes Indolylmethyl umfasst.

26. Eine Verbindung nach einem der Ansprüche 19 bis 25, wobei
X2 ein Aminosäurederivat einer Aminosäure ist, das ausgewählt ist aus der Gruppe, die Phenylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine, 1-Naphtylalanin, 2-Thienylalanin, 3-Thienylalanin, 3,3-Diphenylalanin, Tyrosin, Tryptophan, Histidin und jeweilige Derivate davon umfasst;
oder X2 und X1 sind zusammengenommen PhCH₂CH₂CO- oder PhCH₂-;
X6 ein Aminosäurederivat einer Aminosäure ist, die ausgewählt ist aus der Gruppe, die Tryptophan, Phenylalanin, Tyrosin, Histidin, 1-Naphtylalanin, Benzothienylalanin, 2-Aminoindan-2-carbonsäure, 2-Thienylalanin, 3-Thienylalanin, 2-Fluor-Phenylalanin, 3-Fluor-Phenylalanin, 4-Fluor-Phenylalanin, 2-Chlorphenylalanine, 3-Chlorphenylalanine, 4-Chlorphenylalanine und jeweilige Derivate davon umfasst;
X5 ein Aminosäurederivat einer Aminosäure ist, die ausgewählt ist aus der Gruppe, die D-Cyclohexylalanin, D-Cyclohexylglycin, D-Homo-Cyclohexylalanin, Octahydroindol-2-carbonsäure, 2-Methyl-D-Phenylalanin und jeweilige Derivate davon umfasst; und
X7 ein Aminosäurederivat einer Aminosäure ist, die ausgewählt ist aus der Gruppe, die Norvalin, Norleucin, Homo-Leucin, Leucin, Isoleucin, Valin, Cystein, Cystein(Me), Cystein(Et), Cystein(Pr), Methionin, Allylglycin, Propargylglycin, Cyclohexylglycin, Cyclohexylalanin, Phenylalanin, Tyrosin, Tryptophan, Histidin, 1-Naphtylalanin, 2-Thienylalanin, 3-Thienylalanin und jeweilige Derivate davon umfasst.

27. Verbindung nach einem der Ansprüche 19 bis 26, wobei X1 und/oder X4 eine oder mehrere die Wasserlöslichkeit verbessernde Gruppen aufweisen, wobei die die Wasserlöslichkeit verbessernde Gruppe ausgewählt ist aus der Gruppe, die Hydroxy, Keto, Carboxamido, Ether, Harnstoff, Carbamat, Amino, substituiertes Amino, Guanidino, Pyridyl und Carboxyl umfasst.

28. Verbindung, bevorzugterweise ein C5a-Rezeptor-Antagonist, mit der Struktur
**X1-X2-X3-X4-X5-X6-X7-X8** (II)
, wobei X1-X3 und X5-X8 definiert sind, wie in einem der Ansprüche 17 bis 26 und wobei
X4 eine zyklische oder eine nichtzyklische Aminosäure ist, wobei die zyklische Aminosäure ausgewählt ist aus der Gruppe, die Prolin, Pipecolinsäure, Azetidin-2-carbonsäure, Tetrahydroisochinolin-3-carbonsäure, Tetrahydroisochinolin-1-carbonsäure, Octahydroindol-2-carbonsäure, 1-Aza-bicyclo-[3.3.0]-octan-2-carbonsäure, 4-Phenyl-pyrrolidin-2-carbonsäure, cis-Hyp und trans-Hyp umfasst und die nichtzyklische Aminosäure ausgewählt aus der Gruppe, die Ser, Gln, Asn, Cys(O₂CH₂CH₂CONH₂), Arg, Hyp(COCH₂OCH₂CH₂OCH₂CH₂OCH₃), Hyp(CONH-CH₂CH(OH)-CH₂OH) und jeweilige Derivate davon und jeweilige Analoga davon umfasst; und
die Verbindungslinien - in Formel (I) chemische Bindungen bezeichnen, wobei die chemische Bindung bevorzugt aus der Gruppe ausgewählt ist, die kovalente Bindungen, ionische Bindungen und koordinative Bindungen umfasst, wobei bevorzugterweise die Bindung eine chemische Bindung ist und noch bevorzugtererweise die chemische Bindung eine solche Bindung ist, die ausgewählt ist aus der Gruppe, die Amid-Bindungen, Disulfid-Bindungen, Ether-Bindungen, Thioether-Bindungen, Oxim-Bindungen und Aminotriazin-Bindungen umfasst.

29. Verbindung nach Anspruch 28, **dadurch gekennzeichnet, dass** die Aminosäuren bevorzugt ausgewählt sind aus der Gruppe, die Prolin, Pipecolinsäure, Azetidin-2-carbonsäure, Tetrahydroisochinolin-3-carbonsäure, Tetrahydroisochinolin- 1 -carbonsäure, Octahydroindol-2-carbonsäure, 1-Aza-bicyclo-[3.3.0]-octan-2-carbonsäure, 4-Phenylpyrrolidin-2-carbonsäure, Hyp, Ser, Gln, Asn, Cys(O₂CH₂CH₂CONH₂) und Arg umfasst.

30. Verbindung, bevorzugterweise ein C5a-Rezeptor-Antagonist, mit der Struktur
**X1--X2--X3--X4--X5--X6--X7--X8** (II)
, wobei X1-X2 und X4-X8 definiert sind, wie in einem der Ansprüche 19 bis 29 und wobei
X3 folgende Struktur aufweist: 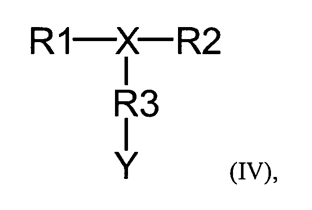 worin
X C(R4) oder N ist,
R1 optional vorhanden ist und wenn R1 vorhanden ist, R1 ein Radikal ist, das ausgewählt ist aus der Gruppe, die >N-R1B, >C(R1B)(R1C) und >O umfasst, wobei R1B und R1C unabhängig voneinander ausgewählt sind aus der Gruppe, die H, Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst;
R2 optional vorhanden ist und wenn R2 vorhanden ist, R2 ein Radikal ist, das ausgewählt ist aus der Gruppe, die C=O, C=S, SO₂, PO(OH), B(OH), CH₂, CH₂CO, CHF und CF₂ umfasst;
R4 ein Radikal ist, wobei das Radikal ausgewählt ist aus der Gruppe, die H, F, CH₃, CF₃, Alkyl und substituiertes Alkyl umfasst;
die Bindung von Struktur (IV) an die Molekülbestandteile X2 und X4 bevorzugt über R1 und R2 erfolgt;
R3 ein Radikal ist, das aus der Gruppe ausgewählt ist, die H, Alkyl, substituiertes Alkyl, Cycloalkyl, substituiertes Cycloalkyl, Cycloalkylalkyl, substituiertes Cycloalkylalkyl, Heterocyclyl, substituiertes Heterocyclyl, Heterocyclylalkyl, substituiertes Heterocyclylalkyl, Aryl, substituiertes Aryl, Arylalkyl, substituiertes Arylalkyl, Heteroaryl, substituiertes Heteroaryl, Heteroarylalkyl, substituiertes Heteroarylalkyl, Acyl, substituiertes Acyl, Alkoxyalkyl, substituiertes Alkoxyalkyl, Aryloxyalkyl, substituiertes Aryloxyalkyl, Sulfhydrylalkyl, substituiertes Sulfhydrylalkyl, Hydroxyalkyl, substituiertes Hydroxyalkyl, Carboxyalkyl, substituiertes Carboxyalkyl, Carboxamidoalkyl, substituiertes Carboxamidoalkyl, Carboxyhydrazinoalkyl, Ureidoalkyl Aminoalkyl, substituiertes Aminoalkyl, Guanidinoalkyl und substituiertes Guanidinoalkyl umfasst.
Y optional vorhanden ist und wenn Y vorhanden ist, Y ein Radikal ist, das ausgewählt ist aus der Gruppe, die H, -N(YB1)-CO-YB2, -N(YB1)-CO-N(YB2)(YB3), -N(YB1)-C(N-YB2)-N(YB3)(YB4), -N(YB1)(YB2), -N(YB1)-SO₂-YB2, O-YB1, S-YB1, -CO-YB1, -CO-N(YB1)(YB2) und -C=N-O-YB1 umfasst, wobei YB1, YB2, YB3 und YB4 einzeln und unabhängig voneinander ausgewählt sind aus der Gruppe, die H, CN, NO₂ Alkyl, substituiertes Alkyl, Cycloalkyl, substituiertes Cycloalkyl, Heterocyclyl, substituiertes Heterocyclyl, Aryl, substituiertes Aryl, Heteroaryl, substituiertes Heteroaryl, Arylakyl, substituiertes Arylalkyl, Cycloalkylalkyl und substituiertes Cycloalkylalkyl umfasst.

31. Verbindung nach Anspruch 30, **dadurch gekennzeichnet, dass**
R3 ein Radikal ist mit der Struktur
-(CH₂)ₘ-Y (V)
oder
-(CH₂)ₘ-C₆H₄-Y (VI)
, wobei
m 1, 2, 3 oder 4 ist;
Y N(R3b)(R3c) oder -N(YB1)-C(N-YB2)-N(YB3)(YB4) ist, wobei R3b, R3c, YB1, YB2, YB3 und YB4 einzeln und unabhängig voneinander ausgewählt sind aus der Gruppe, die H, CN und Alkyl umfasst.

32. Verbindung nach Anspruch 30 oder 31, **dadurch gekennzeichnet, dass** ein Ring zwischen jeweils zwei Gruppierungen ausgebildet ist, wobei die Gruppierungen einzeln und unabhängig voneinander aus der Gruppe ausgewählt sind, die YB1, YB2, YB3 und YB 4 umfasst.

33. Verbindung nach Anspruch 32, **dadurch gekennzeichnet, dass** der Ring durch YB2 und YB3 ausgebildet ist.

34. Verbindung nach einem der Ansprüche 30 bis 33, **dadurch gekennzeichnet, dass** Y -NH₂
oder 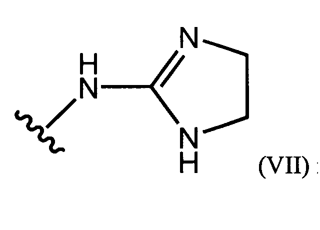 ist.

35. Verbindung nach einem der vorhergehenden Ansprüche, wobei die Verbindung eine der folgenden Verbindungen ist:
| **Nr.** | **Verbindung** |
|---|---|
| **1** | Ac-Phe-[Orn-Pro-cha-Trp-Phe] |
| **2** | Ac-Phe-[Orn-Hyp-cha-Trp-Phe] |
| **3** | HOCH₂(CHOH)₄-C=N-O-CH₂-CO-Phe-[Orn-Pro-cha-Trp-Nle] |
| **4** | X-Phe-[Orn-Pro-cha -Trp-Nle]; X = 2-Acetamido-1-Methyl-Glucuronyl |
| **5** | Ac-Phe-[Orn-Hyp(COCH₂OCH₂CH₂OCH₂CH₂OCH₃)-cha-Trp-Nle] |
| **6** | Ac-Phe-[Orn-Hyp(CONH-CH₂CH(OH)-CH₂OH)-cha-Trp-Nle] |
| **20** | Ac-Phe-[Orn-Pro-cha-Trp-Ecr] |
| **28** | Ac-Phe-[Orn-Pro-cha-Trp-Nle] |
| **29** | Ac-Phe-[Orn-Pro-cha-Trp-Met] |
| **31** | Ac-Phe-[Orn-Pro-cha-Trp-Nva] |
| **32** | Ac-Phe-[Orn-Pro-cha-Trp-Hle] |
| **33** | Ac-Phe-[Orn-Pro-cha-Trp-Eaf] |
| **34** | Ac-Phe-[Orn-Pro-cha-Trp-Ebd] |
| **35** | Ac-Phe-[Orn-Pro-cha-Trp-Eag] |
| **36** | Ac-Phe-[Orn-Pro-cha- Trp-Pmf] |
| **37** | Ac-Phe-[Orn-Pro-cha-Trp-2Ni] |
| **38** | Ac-Phe-[Orn-Pro-cha-Trp-Thi] |
| **41** | Ph-CH₂-CH₂-CO-[Orn-Pro-cha-Trp-Nle] |
| **42** | H-Phe-[Orn-Pro-cha-Trp-Nle] |
| **43** | Ac-Lys-Phe-[Orn-Pro-cha-Trp-Nle] |
| **44** | H-Phe-[Orn-Ser-cha-Trp-Nle] |
| **51** | Ac-Phe-Orn-Pro-cha-Trp-Phe-NH₂ |
| **52** | Ac-Phe-Orn-Aze-cha-Bta-Phe-NH₂ |
| **53** | Ac-Phe-Orn-Pro-cha-Bta-2Ni-NH₂ |
| **54** | Ac-Phe-Orn-Pro-cha-Bta-Cha-NH₂ |
| **55** | Ac-Phe-Orn-Pip-cha-Trp-Phe-NH₂ |
| **56** | Ph-CH₂-[Orn-Pro-cha-Trp-Nle] |
| **57** | Ph-CH₂-[Orn-Pro-cha-Trp-Phe] |
| **58** | Ac-Phe-[Orn-Pro-cha-Trp-1Ni] |
| **59** | Ph-CH(OH)-CH₂-CO-[Om-Pro-cha-Trp-Nle] |
| **61** | Ac-Phe-Orn-Pro-cha-Trp-Phe-NH₂ |
| **62** | Ac-Phe-Orn-Pro-cha-Bta-Phe-NH₂ |
| **64** | Ac-Phe-Orn-Pro-cha-Trp-2Ni-NH₂ |
| **65** | Ac-Phe-Orn-Pro-cha-Trp-Cha-NH₂ |
| **66** | Ac-Thi-Orn-Aze-cha-Bta-Phe-NH₂ |
| **67** | Ac-Thi-Orn-Pip-cha-Bta-Phe-NH₂ |
| **68** | Ac-Phe-Orn-Pro-cha-Trp-Eap-NH₂ |
| **69** | Me₂-Phe-Orn-Pro-cha-Trp-Phe-NH₂ |
| **70** | Ph₂-CH-CH₂-CO-Orn-Pro-cha-Trp-Phe-NH₂ |
| **71** | Ac-Ebw-Orn-Pro-cha-Trp-Phe-NH₂ |
| **72** | Ac-Phe-Orn-Pro-cha-Trp-NH-CH₂-CH₂-Ph |
| **73** | Ac-Phe-Orn-Aze-cha-Bta-NH-CH₂-CH₂-Ph |
| **74** | H-Phe-Orn-Pro-cha-Trp-Phe-NH₂ |
| **75** | H-Me-Phe-Orn-Pro-cha-Trp-Phe-NH₂ |
| **76** | Bu-NH-CO-Phe-Orn-Pro-cha-Trp-Phe-NH₂ |
| **77** | Ac-Thi-Orn-Pro-cha-Trp-Phe-NH₂ |
| **78** | Ac-Ebw-Orn-Pro-cha-Trp-Phe-NH₂ |
| **79** | Ac-Phe-Orn-Ala-cha-Trp-Phe-NH₂ |
| **80** | Ac-Phe-Orn-Pro-cha-Trp-Thi-NH₂ |
| **81** | Ac-Phe-Orn-Aze-cha-Pcf Phe-NH₂ |
| **82** | Ac-Phe-Orn(Ac)-Pro-cha-Trp-Phe-NH₂ |
| **83** | Ac-Phe-Orn-Aze-cha-Trp-Phe-NH₂ |
| **84** | Ac-Phe-Trp-Pro-cha-Trp-Phe-NH₂ |
| **85** | Ph-NH-CO-Phe-Orn-Pro-cha-Trp-Phe-NH₂ |
| **86** | Bu-O-CO-Phe-Orn-Pro-cha-Trp-Phe-NH₂ |
| **87** | Ac-Phe-Lys-Pro-cha-Trp-Phe-NH₂ |
| **88** | Ac-Phe-Arg-Pro-cha-Trp-Phe-NH₂ |
| **89** | Ac-Phe-Gln-Pro-cha-Trp-Phe-NH₂ |
| **92** | Ac-Phe-Orn-Pip-cha-Trp-Phe-NH₂ |
| **93** | Ac-Phe-Orn-Hyp-cha-Trp-Phe-NH₂ |
| **94** | Ac-Phe-Orn-Pro-cha-Trp-1Ni-NH₂ |
| **95** | Ac-Phe-Orn-Aze=cha-Bta-Phe-NH-Me |
| **96** | CH₃-SO₂-Phe-Orn-Aze-cha-Bta-Phe-NH₂ |
| **99** | Ac-Phe-Orn-Aze-cha-Pff Phe-NH₂ |
| **100** | Ac-Phe-Orn-Aze-cha-Mcf Phe-NH₂ |
| **101** | Ac-Phe-Orn(Ac)-Aze-cha-Bta-Phe-NH₂ |
| **102** | Ac-Ebw-Orn-Pro-cha-Trp-Phe-NH₂ |
| **103** | Ac-Phe-Trp-Pro-cha-Trp-Phe-NH₂ |
| **104** | Ac-Phe-Arg-Pro-cha-Trp-Phe-NH₂ |
| **105** | Ac-Phe-Om-Pip-cha-Trp-Phe-NH₂ |
| **106** | 3PP-Orn-Aze-cha-Bta-Phe-NH₂ |
| **107** | Ac-Phe-Orn-Tic-cha-Trp-Phe-NH₂ |
| **108** | Ac-Phe-Orn-Ser-cha-Trp-Phe-NH₂ |
| **109** | Ac-Phe-Orn-Pro-chg-Trp-Phe-NH₂ |
| **110** | Ac-Phe-Orn-Pro-hch-Trp-Phe-NH₂ |
| **111** | Ac-Phe-Orn-Pro-cha-Trp-Phg-NH₂ |
| **112** | Ac-Phe-Bta-Aze-cha-Bta-Phe-NH₂ |
| **113** | Ac-Phe-Trp-Pro-cha-Bta-Phe-NH₂ |
| **115** | Ac-Phe-Orn-Pip-cha-Trp-Phe-OH |
| **116** | Ac-Phe-Orn-Tic-cha-Trp-Phe-OH |
| **117** | Ac-Phe-Orn-Ser-cha-Trp-Phe-OH |
| **118** | Ac-Phe-Orn-Pro-chg-Trp-Phe-OH |
| **119** | Ac-Phe-Eec-Pro-cha-Bta-Phe-NH₂ |
| **120** | Ac-Phe-Nle-Pro-cha-Bta-Phe-NH₂ |
| **121** | Ac-Phe-Har-Pro-cha-Bta-Phe-NH₂ |
| **122** | Ac-Phe-Arg-Pro-cha-Bta-Phe-NH₂ |
| **123** | Ac-Phe-Cys(Acm)-Pro-cha-Bta-Phe-NH₂ |
| **124** | Ac-Phe-Mpa-Pro-cha-Bta-Phe-NH₂ |
| **125** | Ac-Eby-Om-Pro-cha-Bta-Phe-NH₂ |
| **126** | Ac-Phg-Orn-Pro-cha-Bta-Phe-NH₂ |
| **127** | Ac-Phe-Paf-Pro-cha-Bta-Phe-NH₂ |
| **128** | H₂N-CO-Phe-Orn-Pro-cha-Bta-Phe-NH₂ |
| **129** | Me-O-CO-Phe-Orn-Pro-cha-Bta-Phe-NH₂ |
| **130** | (-CO-CH₂-NH-CO-)-Phe-Orn-Pro-cha-Bta-Phe-NH₂ |
| **132** | Ac-Phe-Orn-Pro-hch-Trp-Phe-OH |
| **133** | (-CO-CH₂-CH₂-CO-)-Phe-Orn-Pro-cha-Bta-Phe-NH₂ |
| **134** | ^{t}Bu-CO-Phe-Orn-Pro-cha-Bta-Phe-NH₂ |
| **135** | Ac-Lys-Phe-Orn-Aze-cha-Bta-Phe-NH₂ |
| **136** | Ac-Gly-Phe-Orn-Aze-cha-Bta-Phe-NH₂ |
| **137** | Ac-Arg-Phe-Orn-Aze-cha-Bta-Phe-NH₂ |
| **138** | Ac-His-Phe-Orn-Aze-cha-Bta-Phe-NH₂ |
| **139** | Ac-Ser-Phe-Orn-Aze-cha-Bta-Phe-NH₂ |
| **140** | Ac-Guf-Phe-Orn-Aze-cha-Bta-Phe-NH₂ |
| **141** | Ac-Dab-Phe-Orn-Aze-cha-Bta-Phe-NH₂ |
| **142** | FH₂C-CO-Phe-Orn-Pro-cha-Bta-Phe-NH₂ |
| **143** | Ac-Phe-Orn(Et₂)-Pro-cha-Trp-Phe-NH₂ |
| **144** | Ac-Phe-[Orn-Hyp-cha-Trp-Nle] |
| **145** | 3PP-[Orn-Hyp-cha-Trp-Nle] |
| 146 | Ac-Phe-[Orn-Pro-cha-Trp-Tyr] |
| **147** | Ac-Phe-[Orn-Pro-omf Trp-Nle] |
| **149** | Ac-Phe-Orn-Pro-hle-Bta-Phe-NH₂ |
| **150** | Ac-Phe-Arg(CH₂-CH₂)-Pro-cha-Bta-Phe-NH₂ |

36. Pharmazeutische Formulierung umfassend mindestens eine Verbindung gemäß einem der vorangehenden Ansprüchen und zusätzlich ein pharmazeutisch akzeptables Trägermittel.

37. Verwendung mindestens einer Verbindung nach einem der vorangehenden Ansprüche zur Herstellung eines Medikamentes.

38. Verwendung nach Anspruch 37, **dadurch gekennzeichnet, dass** das Medikament für die Prävention und/oder Behandlung einer Erkrankung verwendet wird, bei der das Komplementsystem aktiviert ist und/oder bei der die Inhibierung des Komplementsystems eine Linderung der Symptome hervorruft.

39. Verwendung nach Anspruch 37 oder 38, **dadurch gekennzeichnet, dass** die Krankheit und/oder die zu behandelnden Symptome ausgewählt sind aus der Gruppe, die Rheumatoide Arthritis, systemischen Lupus erythematodes, Multiple Sklerose, Psoriasis, septischer Schock, Asthma, Vaskulitis, Dermatomyositis, entzündliche Darmerkrankungen (IBD: inflammatory bowel disease) Pemphigus, Myasthenia gravis, akute respiratorische Insuffizienz, Gehirnschlag, Herzinfarkt, Reperfusionsschaden, Verbrennungen und akute Verletzungen des zentralen Nervensystems umfasst.

40. Verwendung mindestens einer Verbindung nach einem der vorangehenden Ansprüche zur Prävention und/oder Unterstützung chirurgischer Eingriffe.

41. Verwendung nach Anspruch 37 oder 38, **dadurch gekennzeichnet, dass** das Medikament zur Prävention und/oder Unterstützung chirurgischer Eingriffe verwendet werden.

42. Verwendung nach Anspruch 37, **dadurch gekennzeichnet, dass** das Medikament zur Unterstützung und/oder zur Prävention und/oder Nachsorge eines chirurgischen Eingriffs verwendet werden, wobei der chirurgische Eingriff ausgewählt ist aus der Gruppe, die CABG, PACT; PTA; MidCAB, OPCAB, , Thrombolyse und Gefäßverschluss (clamping) umfasst.

43. Verwendung nach Anspruch 37, **dadurch gekennzeichnet, dass** das Medikament für die thrombolytische Behandlung verwendet wird.

44. Verwendung nach Anspruch 37, **dadurch gekennzeichnet, dass** das Medikament im Rahmen einer Dialyse-Behandlung, gegebenenfalls vor, während oder danach, verwendet wird.
